(19) Europäisches Patentamt
European Patent Office
Office européen des brevets

(11) EP 4 559 894 A1

# (12) EUROPEAN PATENT APPLICATION
published in accordance with Art. 153(4) EPC

(43) Date of publication:
**28.05.2025 Bulletin 2025/22**

(21) Application number: **23842317.2**

(22) Date of filing: **18.07.2023**

(51) International Patent Classification (IPC):
***C07C 219/06*** *(2006.01)* ***A61K 9/127*** *(2025.01)*
***A61K 47/18*** *(2017.01)*

(52) Cooperative Patent Classification (CPC):
**A61K 9/127; A61K 9/51; A61K 47/18; C07C 213/08; C07C 219/06; C07C 219/08; C07C 219/24; C07C 227/04; C07C 227/08; C07C 227/18; C07C 229/14; C07C 229/48; C07C 233/06; C07C 237/24; C07C 255/46**

(86) International application number:
**PCT/CN2023/107940**

(87) International publication number:
**WO 2024/017254 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.07.2022 CN 202210847325**

(71) Applicant: **Shenzhen Shenxin Biotechnology Co., Ltd.**
**Shenzhen, Guangdong 518000 (CN)**

(72) Inventors:
- **LI, Linxian**
  **Shenzhen, Guangdong 518000 (CN)**
- **CHEN, Yonghao**
  **Shenzhen, Guangdong 518000 (CN)**
- **HUANG, Xing**
  **Shenzhen, Guangdong 518000 (CN)**

(74) Representative: **Epping - Hermann - Fischer Patentanwaltsgesellschaft mbH**
**Schloßschmidstraße 5**
**80639 München (DE)**

Remarks:
The complete document including Reference Table(s) and the Sequence Listing(s) can be downloaded from the EPO website

# (54) AMINO LIPID COMPOUND, PREPARATION METHOD THEREFOR, AND USE THEREOF

(57) The present application relates to an amino lipid compound having the following structural formula (I) or a pharmaceutically acceptable salt or stereoisomer thereof, and use thereof as a component of a lipid nanoparticle preparation for delivering a therapeutic agent. The present application further relates to a composition comprising the amino lipid compound, and particularly, to a lipid nanoparticle, and use thereof.

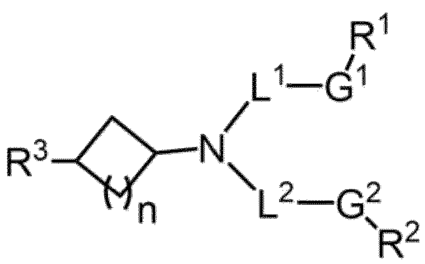

(I)

EP 4 559 894 A1

## Description

### TECHNICAL FIELD

The present disclosure belongs to the pharmaceutical field, and specifically relates to an amino lipid compound, a preparation method therefor, and use thereof.

### BACKGROUND ART

Gene medicine is to deliver a gene with specific genetic information to target cells by artificial means. The expressed target protein has regulatory, therapeutic, and even curative effects on diseases caused by congenital or acquired gene defects, or the gene sequence can interfere with or regulate the expression of related genes, achieving clinical therapeutic effects. Both nucleic acid and cell membrane carry negative charges, and naked nucleic acids are difficult to be directly introduced into cells and are easily degraded by nucleic acid-degrading enzymes in cytoplasm, failing to achieve the effect of gene introduction and gene therapy. Therefore, external forces or vectors are required to realize gene delivery.

Gene vectors are generally divided into viral vectors and non-viral vectors. Although the viral vectors show high transfection efficiency in vivo and in vitro, they have many defects, such as high toxicity, strong immune response, small gene capacity, poor targeting, and complicated preparation process. The non-viral vectors have attracted more and more companies to invest in research and development due to their advantages, such as easy preparation, transportation and storage, safety, efficacy, and non-immunogenicity.

Lipid nanoparticles (LNP) are currently the mainstream non-viral vectors, and are often used in vaccines because they are easily absorbed by antigen-presenting cells. In the prior art, a variety of compounds useful in lipid nanoparticles have been reported, but it was found in actual use that when used for vaccine delivery, these compounds showed low delivery efficiency and poor drug stability, together with problems such as long elimination phase half-life, high toxicity, and low safety, which are unfavorable to clinical application and cannot meet the needs of modern vaccine preparations.

For example, the commercially available cationic liposome compound MC3 showed insufficient protein expression level and low levels of antibody production in the body, when used in vaccine delivery in reality. In addition, it is reported in Xiaoping Zhang et al., Pharmacokinetics of Patisiran, the First Approved RNA Interference Therapy in Patients With Hereditary Transthyretin-Mediated Amyloidosis, The Journal of Clinical Pharmacology 2020, 60(5) 573-585 that the cationic liposome MC3 has an elimination phase half-life of 14.6 to 28.7 days in a Phase II clinical study of Patisiran.

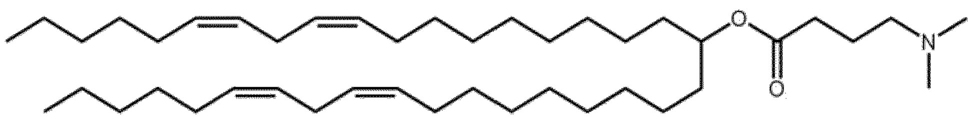

MC3

Therefore, there is an urgent need to develop substances that can be used for intracellular delivery of therapeutic agents, which have strong delivery capabilities, good stability, moderate elimination phase half-life, and/or high safety (i.e., low incidence of adverse reactions).

### SUMMARY OF THE INVENTION

Through extensive research, the inventors of the present disclosure unexpectedly discovered that an amino lipid compound, which has a cycloalkyl structural unit and an additional substituent at the meta position of the amine substituent of the cycloalkyl, achieves good delivery ability, good stability and high safety, and can be used to deliver bioactivators (e.g. nucleic acids) into cells to improve their protein expression levels.

The present disclosure therefore provides an amino lipid compound, which has a cycloalkyl structural unit and an additional substituent at the meta position of the amine substituent of the cycloalkyl. The amino lipid compound of the present disclosure is stable during in vivo circulation, can be rapidly degraded in endosomes/lysosomes, and has significantly enhanced delivery efficiency.

The amino lipid compound of the present disclosure has a cycloalkyl structural unit and an additional substituent at the meta position of the amine substituent of the cycloalkyl, such that the compound or the lipid nanoparticles comprising the compound show good biological activity, high protein expression level, significant immune activity, and good stability when used as a vaccine vector, and can be stored, transported and used at room temperature and has a low incidence of adverse reactions.

The present disclosure also provides a method for preparing the amino lipid compound or a pharmaceutically

acceptable salt or stereoisomer thereof, which has readily available raw materials, mild reaction conditions, good reaction selectivity, high yield, low instrument and equipment requirements, and simple operation.

The present disclosure also provides a composition such as a lipid nanoparticle, comprising the amino lipid compound or a pharmaceutically acceptable salt or stereoisomer thereof.

In one aspect, the present disclosure provides a compound represented by the following structural formula I or a pharmaceutically acceptable salt or stereoisomer thereof:

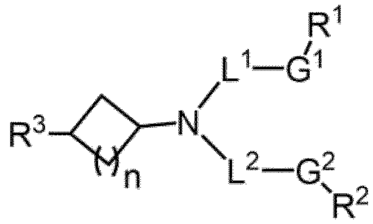

(I)

wherein:

$L^1$ and $L^2$ are identical or different, and each independently selected from the group consisting of $C_1$-$C_{12}$ alkylene, $C_2$-$C_{12}$ alkenylene and $C_2$-$C_{12}$ alkynylene; preferably, $L^1$ and $L^2$ are identical or different, and each independently selected from the group consisting of $C_3$-$C_{10}$ alkylene, $C_3$-$C_{10}$ alkenylene and $C_3$-$C_{10}$ alkynylene; further preferably, $L^1$ and $L^2$ are identical or different, and each independently selected from the group consisting of $C_3$-$C_{10}$ alkylene; most preferably, $L^1$ and $L^2$ are identical or different, and each independently selected from the group consisting of $C_5$-$C_8$ alkylene;

$G^1$ and $G^2$ are identical or different, and each independently selected from the group consisting of -O-(C=O)-, -(C=O)-O-, -C(=O)-, -O-, -C(=O)-S- and -S-C(=O)-; preferably, $G^1$ and $G^2$ are identical or different, and each independently selected from the group consisting of -O-(C=O)-, -(C=O)-O-, -C(=O)- and -O-; most preferably, $G^1$ and $G^2$ are identical or different, and each independently selected from the group consisting of -O-(C=O)- and -(C=O)-O-;

$R^1$ and $R^2$ are identical or different, and each independently selected from the group consisting of $C_5$-$C_{27}$ alkyl, and $C_5$-$C_{27}$ alkenyl comprising one or more double bonds, which are attached via any one carbon thereof; preferably, $R^1$ and $R^2$ are identical or different, and each independently selected from the group consisting of $C_8$-$C_{20}$ alkyl, and $C_8$-$C_{20}$ alkenyl comprising one or more double bonds, which are attached via any one carbon thereof; further preferably, $R^1$ and $R^2$ are identical or different, and each independently selected from the group consisting of $C_9$-$C_{17}$ alkyl, and $C_9$-$C_{18}$ alkenyl comprising one or two double bonds, which are attached via any one carbon thereof; most preferably, $R^1$ and $R^2$ are identical or different, and each independently selected from the group consisting of

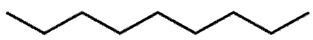 , 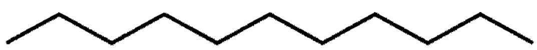 ,

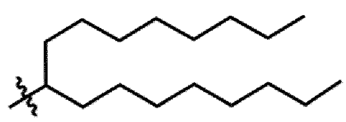 , 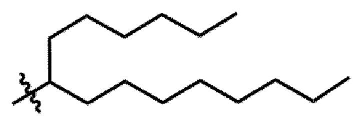 ,

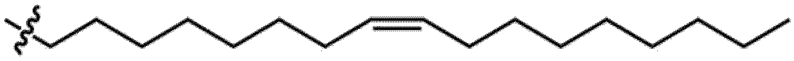 ,

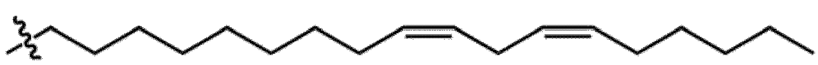 ,

and

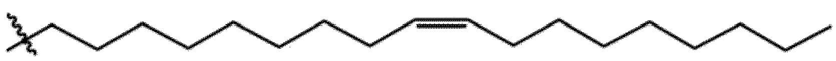 ;

$R^3$ is selected from the group consisting of halogen, hydroxy, cyano, $C_1$-$C_6$ alkyl, nitro, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino; preferably, $R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino; further preferably, $R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl, and $C_1$-$C_4$ alkylcarbonylamino; most preferably, $R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

Furthermore, the present disclosure provides a compound represented by the above structural formula (I) or a pharmaceutically acceptable salt or stereoisomer thereof, wherein:

$L^1$ and $L^2$ are identical or different, and each independently selected from the group consisting of $C_3$-$C_{10}$ alkylene, $C_3$-$C_{10}$ alkenylene and $C_3$-$C_{10}$ alkynylene;
$G^1$ and $G^2$ are identical or different, and each independently selected from the group consisting of -O-(C=O)-, -(C=O)-O-, -C(=O)- and -O-;
$R^1$ and $R^2$ are identical or different, and each independently selected from the group consisting of $C_8$-$C_{20}$ alkyl, and $C_8$-$C_{20}$ alkenyl comprising one or more double bonds, which are attached via any one carbon thereof;
$R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino;
n is selected from the group consisting of 1, 2, and 3.

Furthermore, the present disclosure provides a compound represented by the above structural formula (I) or a pharmaceutically acceptable salt or stereoisomer thereof, wherein:

$L^1$ and $L^2$ are identical or different, and each independently selected from the group consisting of $C_3$-$C_{10}$ alkylene;
$G^1$ and $G^2$ are identical or different, and each independently selected from the group consisting of -O-(C=O)- and -(C=O)-O-;
$R^1$ and $R^2$ are identical or different, and each independently selected from the group consisting of $C_9$-$C_{17}$ alkyl, and $C_9$-$C_{18}$ alkenyl comprising one or two double bonds, which are attached via any one carbon thereof;
$R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl and $C_1$-$C_4$ alkylcarbonylamino;
n is selected from the group consisting of 1, 2, and 3.

Still furthermore, the present disclosure provides a compound represented by the above structural formula (I) or a pharmaceutically acceptable salt or stereoisomer thereof, wherein:

$L^1$ and $L^2$ are identical or different, and each independently selected from the group consisting of $C_5$-$C_8$ alkylene;
$G^1$ and $G^2$ are identical or different, and each independently selected from the group consisting of -O-(C=O)- and -(C=O)-O-;
$R^1$ and $R^2$ are identical or different, and each independently selected from the group consisting of

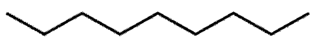,
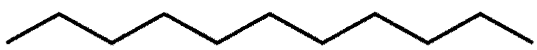,

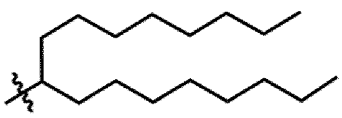,
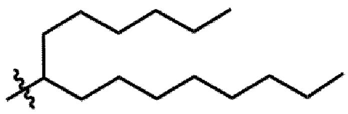,

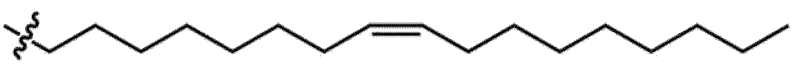,

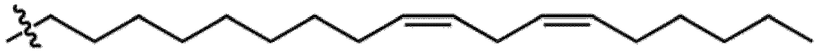,

and

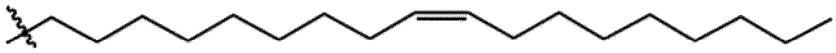

;

$R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

Yet furthermore, the present disclosure provides a compound represented by the above structural formula (I) or a pharmaceutically acceptable salt or stereoisomer thereof, wherein said compound is selected from the group consisting of:

| No. | Structure |
|---|---|
| 1 | 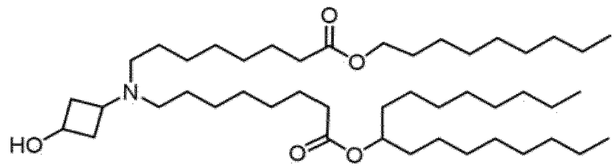 |
| 2 | 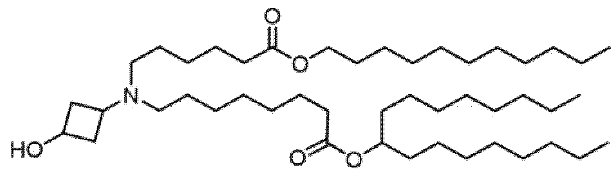 |
| 4 | 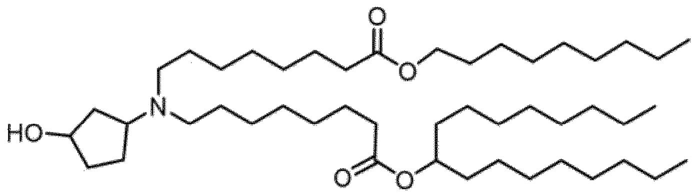 |
| 5 | 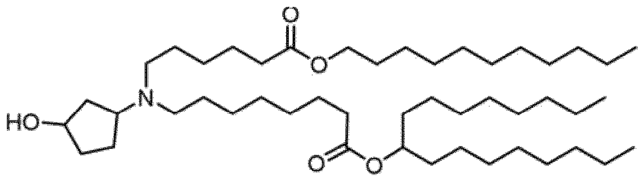 |
| 6 | 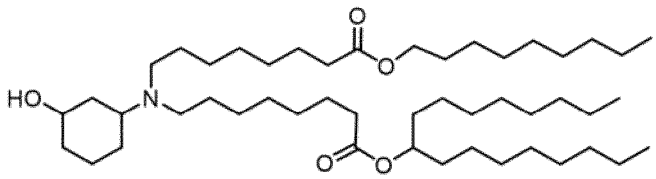 |
| 7 | 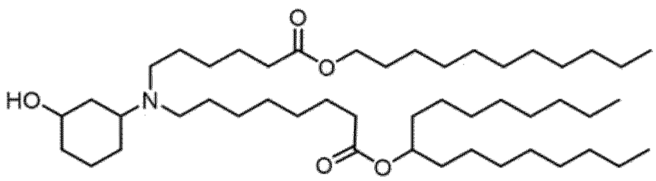 |

(continued)

| No. | Structure |
|---|---|
| 8 | 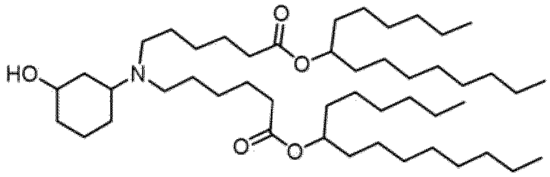 |
| 9 | 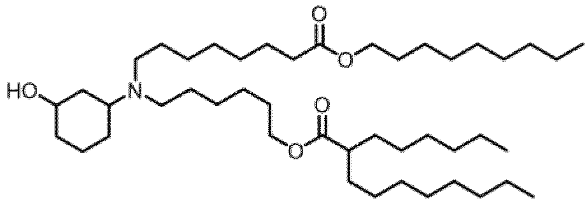 |
| 10 | 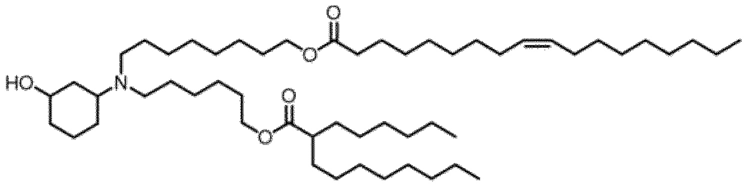 |
| 11 | 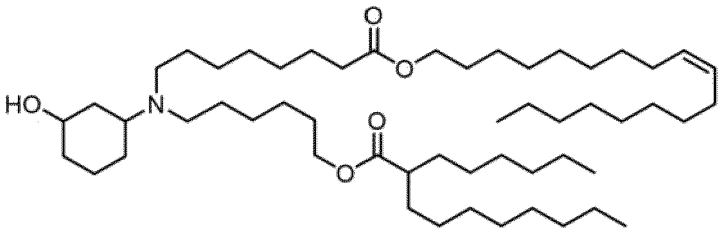 |
| 12 | 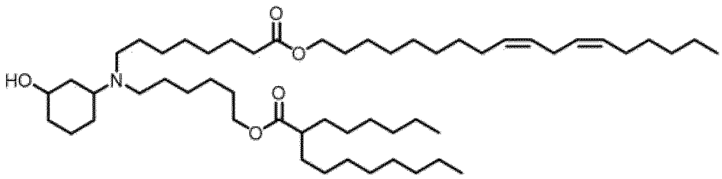 |
| 14 | 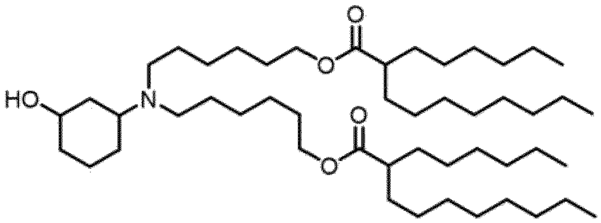 |
| 15 | 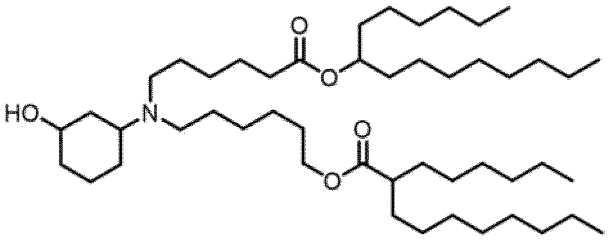 |

(continued)

| No. | Structure |
|---|---|
| 16 | 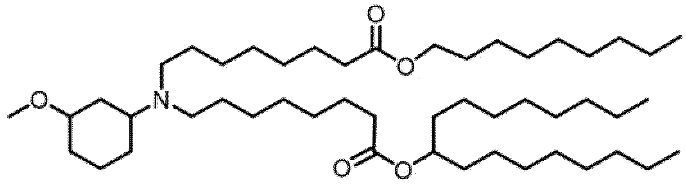 |
| 17 | 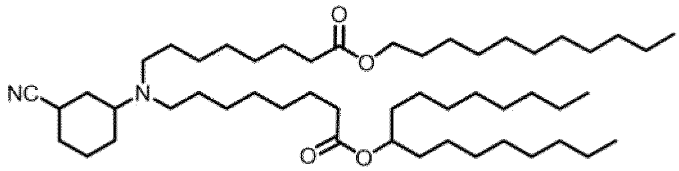 |
| 18 | 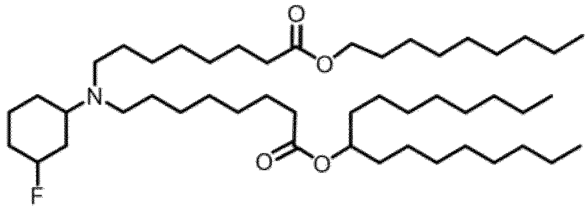 |
| 19 | 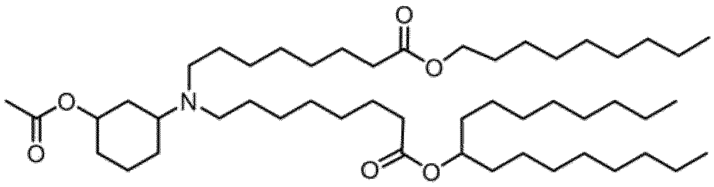 |
| 20 | 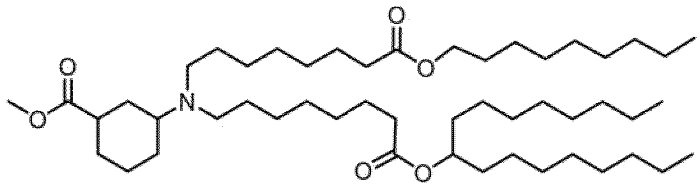 |
| 21 | 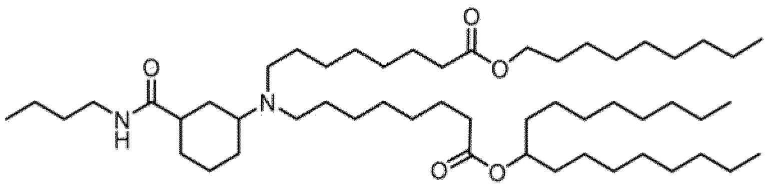 |
| 22 | 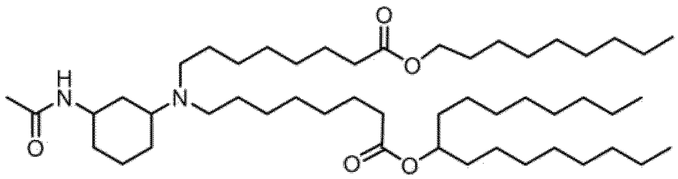 |
| 25 | 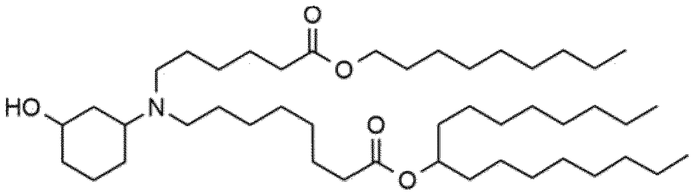 |

(continued)

| No. | Structure |
|---|---|
| 26 | 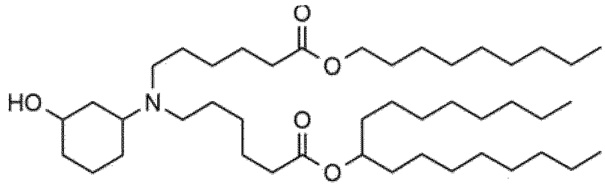 |
| 27 | 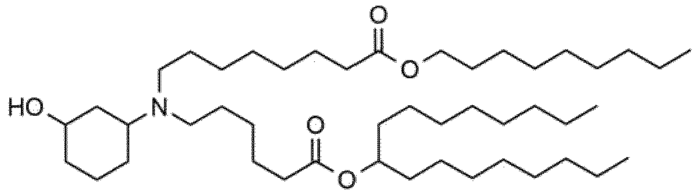 |

.

In another aspect, the present disclosure provides a compound having a structure selected from the following structures, an optical isomer thereof or a pharmaceutically acceptable salt thereof:

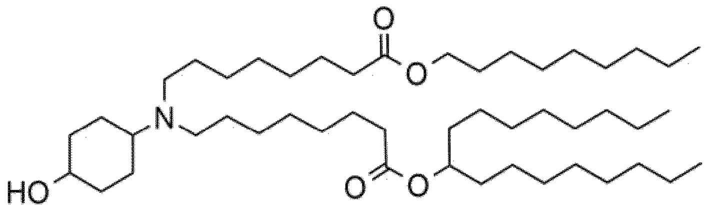

,

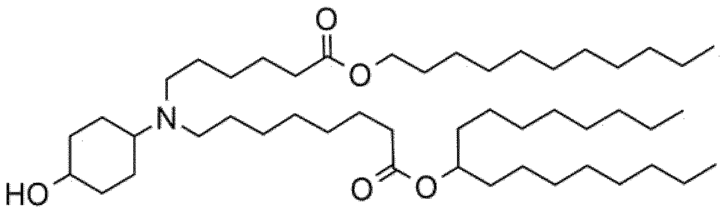

,

and

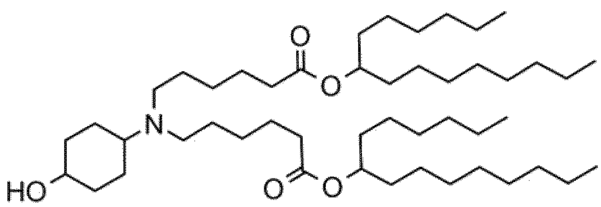

.

In another aspect, the present disclosure provides a compound having the following structure, an optical isomer thereof or a pharmaceutically acceptable salt thereof:

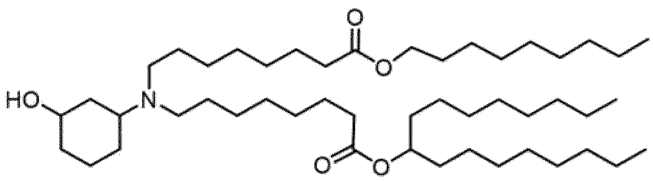

.

Furthermore, the present disclosure provides a compound represented by a structure selected from the following

structural formulae, or a pharmaceutically acceptable salt thereof:

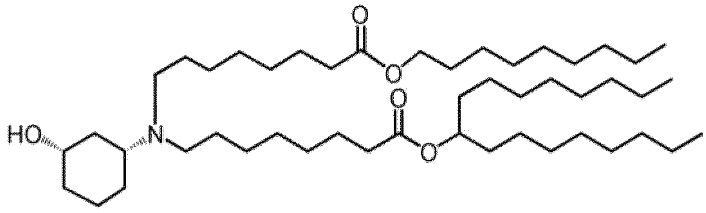

(I-6-II)

,

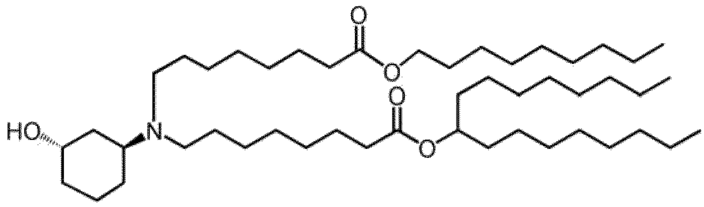

(I-6-III)

,

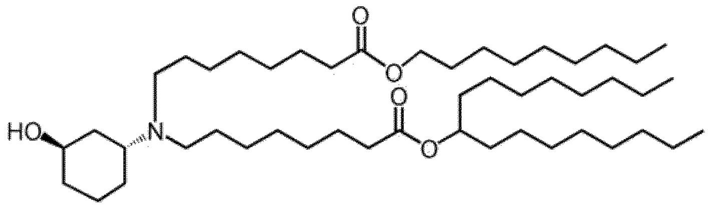

(I-6-IV)

,

and

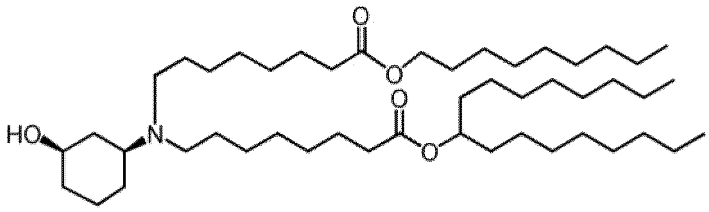

(I-6-V)

.

In another aspect, the present disclosure provides a compound having the following structure, an optical isomer thereof or a pharmaceutically acceptable salt thereof:

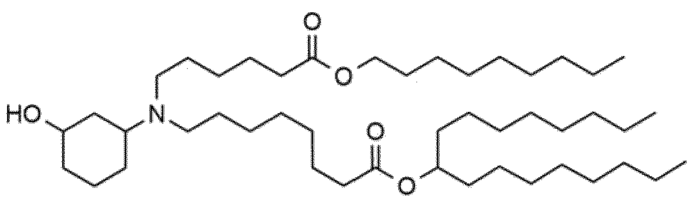

.

Furthermore, the present disclosure provides a compound represented by a structure selected from the following structural formulae, or a pharmaceutically acceptable salt thereof:

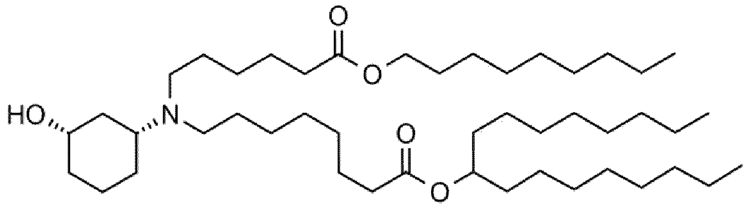

(I-25-II) ,

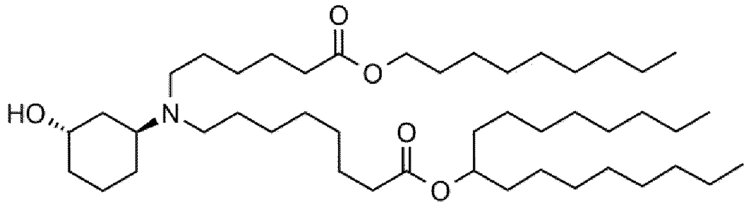

(I-25-III) ,

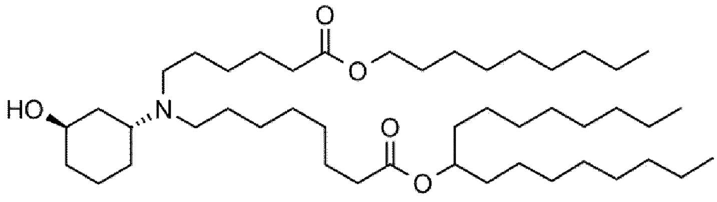

(I-25-IV) ,

and

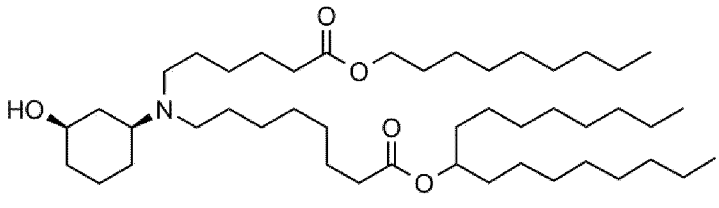

(I-25-V) .

In another aspect, the present disclosure provides a compound having the following structure, an optical isomer thereof or a pharmaceutically acceptable salt thereof:

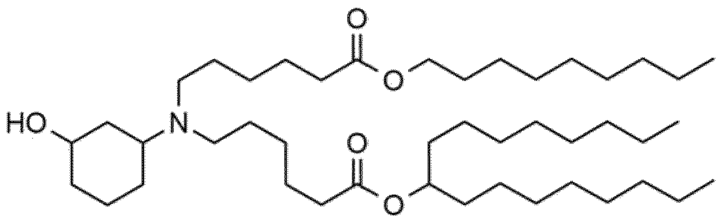

.

Furthermore, the present disclosure provides a compound represented by a structure selected from the following structural formulae, or a pharmaceutically acceptable salt thereof:

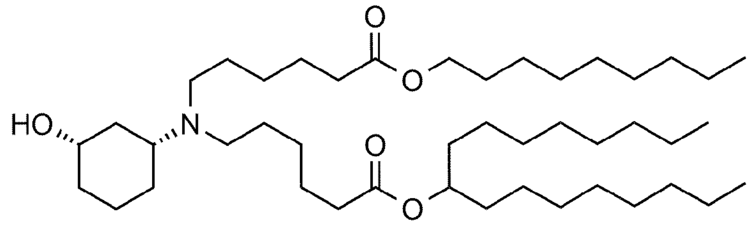

(I-26-II) ,

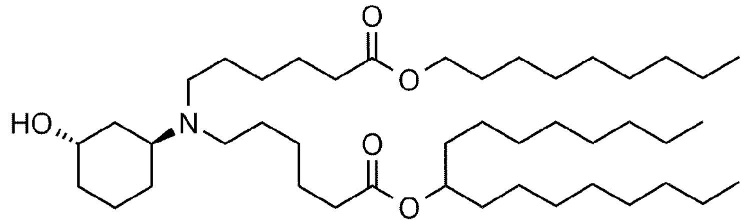

(I-26-III) ,

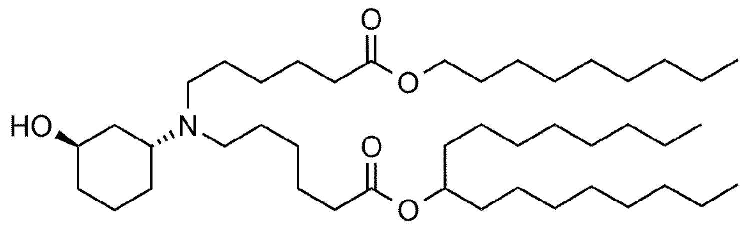

(I-26-IV) ,

and

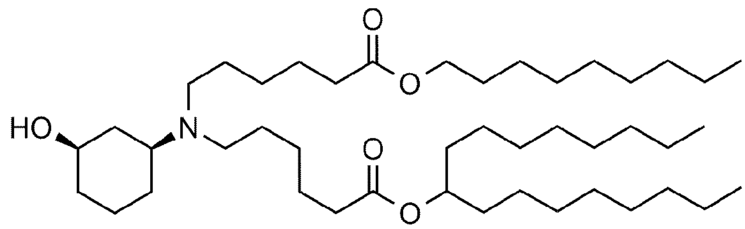

(I-26-V) .

In another aspect, the present disclosure provides a compound having the following structure, an optical isomer thereof or a pharmaceutically acceptable salt thereof:

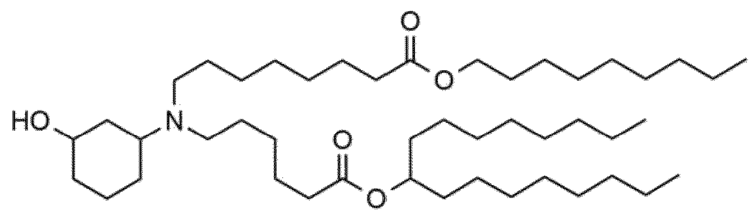

.

Furthermore, the present disclosure provides a compound represented by a structure selected from the following structural formulae, or a pharmaceutically acceptable salt thereof:

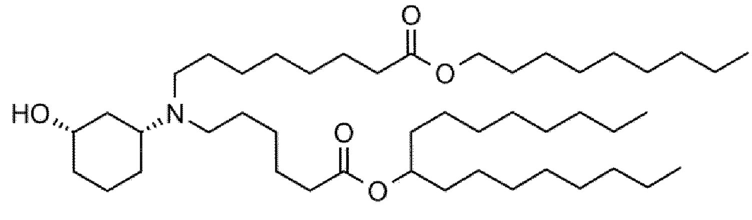

(I-27-II) ,

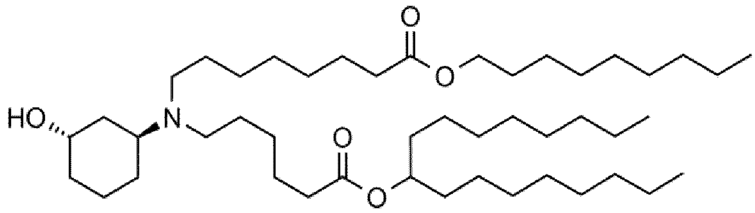

(I-27-III) ,

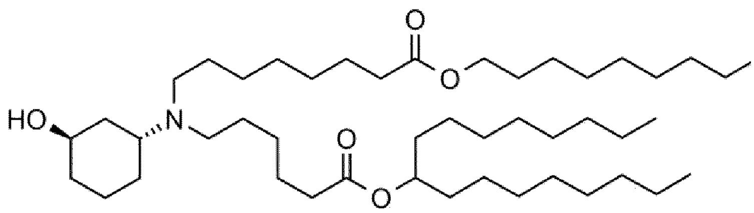

(I-27-IV)

and

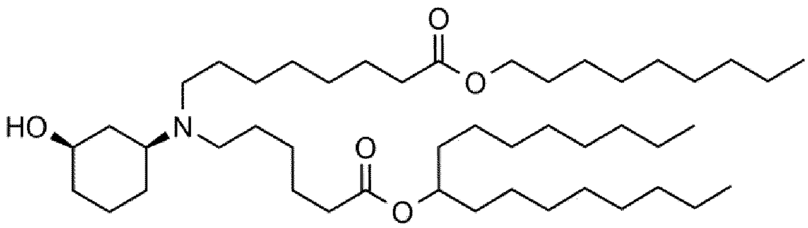

(I-27-V) .

In one aspect, the present disclosure provides lipid nanoparticles (LNPs) comprising the compound represented by the structural formula (I) of the present disclosure according to any of the above embodiments, or a pharmaceutically acceptable salt or stereoisomer thereof.

In some embodiments, the lipid nanoparticles refer to particles in nanometer scale (e.g., 1 nm to 1000 nm), which comprises one or more lipids.

In some embodiments, the lipid nanoparticles have an average diameter of 20nm-800nm, 20nm-500nm, 20nm-400nm, 20nm-300nm, 20nm-200nm, 20nm-100nm, 30nm-700nm, 30nm-500nm, 30nm-300nm, 30nm-200nm, 30nm-100nm, 40nm-800nm, 40nm-600nm, 40nm-500nm, 40nm-300nm, 40nm-200nm, 40nm-100nm, 50nm-800nm, 50nm-600nm, 50nm-500nm, 50nm-500nm, 50nm-400nm, 50nm-500nm, 50nm-400nm, 50nm-300nm, 50nm-200nm, 50nm-100nm, 60nm-800nm, 60nm-600nm, 60nm-500nm, 60nm-400nm, 60nm-300nm, 60nm-200nm or 60nm-100nm. In some alternative specific examples, the lipid nanoparticles have an average diameter of 26nm, 31nm, 36nm, 41nm, 46nm, 51nm, 56nm, 61nm, 66nm, 71nm, 76nm, 81nm, 86nm, 91nm, 96nm, 101nm, 106nm, 111nm, 116nm, 121nm, 126nm, 131nm, 136nm, 141nm, 146nm, 151nm, 156nm, 161nm, 166nm, 171nm, 176nm, 181nm, 186nm, 191nm, 196nm, 201nm, 206nm, 211nm, 216nm, 221nm, 226nm, 231nm, 236nm, 241nm, 246nm or 249nm. Herein, the average diameter of the lipid nanoparticles can be expressed as the z-average value determined by dynamic light scattering.

In some embodiments, the lipid nanoparticles further comprise one or more of the following: a helper lipid, a structural lipid, and a polymer-lipid (e.g., polyethylene glycol-lipid).

In some embodiments, the lipid nanoparticles comprise (1) a helper lipid, (2) a structural lipid, (3) a polymer-lipid and (4) a compound represented by the structural formula (I) of the present disclosure, or a pharmaceutically acceptable salt or stereoisomer thereof.

In some embodiments, the helper lipid of the lipid nanoparticles includes a phospholipid. The phospholipid is usually semi-synthetic, or it may be naturally derived or chemically modified. In an alternative specific example, the helper lipid of the lipid nanoparticles is a phospholipid. In some embodiments, the phospholipid of the lipid nanoparticles includes one or more of the following: DSPC (distearoyl phosphatidylcholine), DOPE (dioleoyl phosphatidylethanolamine), DOPC (dioleoyl phosphatidylcholine), DOPS (dioleoyl phosphatidylserine), DSPG (1,2-distearoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl)-homoserine) and lysophospholipid. In some embodiments, the helper lipid of the lipid nanoparticles is one or more of the following: DSPC, DOPE, DOPC and DOPS. In some embodiments, the helper lipid of the lipid nanoparticles is DSPC and/or DOPE.

In some embodiments, the structural lipid of the lipid nanoparticles includes a sterol. In an alternative specific example, the structural lipid of the lipid nanoparticles is a sterol. In some embodiments, the sterol of the lipid nanoparticles includes one or more of the following: 20α-hydroxycholesterol, cholesterol, cholesteryl esters, steroid hormones, steroid vitamins, bile acid, cholesterin, ergosterol, β-sitosterol and oxidized cholesterol derivatives. In some embodiments, the structural lipid of the lipid nanoparticles includes at least one of cholesterol, cholesteryl esters, steroid hormones, steroid vitamins and bile acid. In some embodiments, the structural lipid of the lipid nanoparticles is cholesterol. In an alternative specific example, the structural lipid of the lipid nanoparticles is a high-purity cholesterol, particularly an injection-grade high-purity cholesterol, such as CHO-HP (produced by AVT). In other embodiments, the structural lipid is 20α-hydroxycholesterol.

In some embodiments, the polymer-lipid of the lipid nanoparticle refers to a conjugate comprising a polymer and a lipid coupled to the polymer. The polymer-lipid (e.g., polyethylene glycol-lipid) in the lipid nanoparticles can improve the stability of the lipid nanoparticles in vivo.

In some embodiments, the lipid for forming the polymer-lipid of the lipid nanoparticles includes one or more of the following: 1,2-dimyristoyl-sn-glycerol (DMG), distearoyl-phosphatidyl-ethanolamine (DSPE), diacylglycerol (DAG), dialkyloxypropyl (DAA), phospholipids, ceramide (Cer), 1,2-distearoyl-rac-glycerol (DSG) and dipalmitoyl-rac-glycerol (DPG).

In some embodiments, the polymer for forming the polymer-lipid of the lipid nanoparticles includes one or two of the following: a hydrophilic polymer and an amphiphilic polymer.

In some embodiments, the polymer for forming the polymer-lipid of the lipid nanoparticles is a hydrophilic polymer. In other embodiments, the polymer for forming the polymer-lipid of the lipid nanoparticles is an amphiphilic polymer.

In some embodiments, the hydrophilic polymer includes one or more of the following: polyethylene glycol (PEG), poly(oxazoline) (POX), poly(glycerols) (PGs), poly(hydroxypropyl methacrylate) (PHPMA), poly(2-hydroxyethyl methacrylate) (PHEMA), poly(N-(2-hydroxypropyl)methacrylamide) (HPMA), poly(vinylpyrrolidone) (PVP), poly(N,N-dimethyl acrylamide) (PDMA), poly(N-acryloyl morpholine) (PAcM), polyaminoacids, glycosaminoglycans (GAGs), heparin, hyaluronic acid (HA), polysialic acid (PSA), elastin-like polypeptides (ELPs), serum albumin and CD47. In an alternative specific example, the hydrophilic polymer includes polyethylene glycol.

Correspondingly, the polymer-lipid include one or more of the following: polyethylene glycol-lipid (PEG-lipid), poly(oxazoline)-lipid, poly(glycerol)-lipid, poly(hydroxypropyl methacrylate)-lipid, poly(2-hydroxyethyl methacrylate)-lipid, poly(N-(2-hydroxypropyl)methacrylamide)-lipid, poly(vinylpyrrolidone)-lipid, poly(N,N-dimethyl acrylamide)-lipid, poly(N-acryloyl morpholine)-lipid, glycosaminoglycan-lipid, heparin-lipid, hyaluronic acid-lipid, polysialic acid-lipid, elastin-like polypeptide-lipid, serum albumin-lipid and CD47-lipid. It should be noted that the "PEG-lipid" is a conjugate of the polyethylene glycol and the lipid, the "poly(oxazoline)-lipid" refers to a conjugate formed by coupling the poly(oxazoline) and the lipid, and the "poly(glycerol)-lipid" refers to a conjugate formed by coupling the poly(glycerol) and the lipid; the same applies to other polymer-lipids.

In some embodiments, the polymer-lipid comprises a PEG-lipid. In an alternative specific example, the polymer-lipid is a PEG-lipid. In some embodiments, the PEG-lipid includes one or more of the following: PEG-1,2-dimyristoyl-sn-glycerol (PEG-DMG), PEG-distearoyl-phosphatidyl-ethanolamine (PEG-DSPE), PEG-diacylglycerol (PEG-DAG), PEG-dialkyloxypropyl (PEG-DAA), PEG-phospholipid, PEG-ceramide (PEG-Cer), PEG-1,2-distearoyl-rac-glycerol (PEG-DSG) and PEG-1,2-dipalmitoyl-rac-glycerol (PEG-DPG). The PEG-lipid is preferably one or more of the following: PEG-DMG, PEG-DSG and PEG-DPG. PEG-DMG is a polyethylene glycol derivative of 1,2-dimyristoyl glycerol. In some embodiments, the PEG in the PEG-lipid has an average molecular weight of about 2000-5000. In an alternative specific example, the PEG in the PEG-lipid has an average molecular weight of about 2000. In some embodiments, the PEG-lipid is PEG2000-DMG.

In some embodiments, the amphiphilic polymer includes one or more of the following: poly(carboxybetaine) (pCB), poly(sulfobetaine) (pSB), phosphobetaine-base polymer and phosphorylcholine polymer. In some embodiments,

the amphiphilic polymer includes one or more of the following: poly(carboxybetaine acrylamide) (pCBAA), poly(carboxybetaine methacrylate), poly(sulfobetaine methacrylate), poly(methacryloyloxyethyl phosphorylcholine), poly(vinyl-pyridinio propanesulfonate), poly(carboxybetaine) based on vinylimidazole, poly(sulfobetaine) based on vinylimidazole, poly(sulfobetaine) based on vinylpyridine.

Correspondingly, the polymer-lipid includes one or more of the following: polyhydroxybetaine-lipid, polysulfobetaine-lipid, phosphobetaine-base polymer-lipid and phosphorylcholinepolymer-lipid. In some embodiments, the polymer-lipid includes one or more of the following: poly(carboxybetaine acrylamide)-lipid, poly(carboxybetaine methacrylate)-lipid, poly(sulfobetaine methacrylate)-lipid, poly(methacryloyloxyethyl phosphorylcholine)-lipid, poly(vinyl-pyridinio propanesulfonate)-lipid, poly(carboxybetaine) based on vinylimidazole-lipid, poly(sulfobetaine) based on vinylimidazole-lipid, and poly(sulfobetaine) based on vinylpyridine-lipid.

In addition, in some embodiments, the polymers used in nanoparticles in Hoang Thi, Thai Thanh et al. "The Importance of Poly(ethylene glycol) Alternatives for Overcoming PEG Immunogenicity in Drug Delivery and Bioconjugation." Polymers vol. 12, 2 298 are also incorporated herein.

In some embodiments, the lipid nanoparticle comprises (1) a helper lipid, (2) a structural lipid, (3) a PEG-lipid, and (4) a compound represented by the structural formula (I) of the present disclosure, or a pharmaceutically acceptable salt or stereoisomer thereof, and based on the total amount of the compound represented by the structural formula (I) of the present disclosure or a pharmaceutically acceptable salt or stereoisomer thereof, the helper lipid, the structural lipid and the PEG-lipid, the lipid nanoparticle comprises the structural formula (I) of the present disclosure or a pharmaceutically acceptable salt or stereoisomer thereof in an amount (percentage by mole) of following: about 25%-75%, e.g., about 25%-28%, 28%-32%, 32%-35%, 35%-40%, 40%-42%, 42%-45%, 45%-48%, 48%-55%, 55%-65%, 65%-75%, 45%-46.3%, 46.3%-48%, 48%-49.5%, 49.5%-50%, 50%-55% or 60%-65%.

In some embodiments, the lipid nanoparticle comprises (1) a helper lipid, (2) a structural lipid, (3) a PEG-lipid, and (4) a compound represented by the structural formula (I) of the present disclosure, or a pharmaceutically acceptable salt or stereoisomer thereof, and based on the total amount of the compound represented by the structural formula (I) of the present disclosure or a pharmaceutically acceptable salt or stereoisomer thereof, the helper lipid, the structural lipid and the PEG-lipid, the lipid nanoparticle comprises the helper lipid in an amount (percentage by mole) of following: about 5%-45%, e.g., about 5%-10%, 10%-16%, 16%-25%, 25%-33.5%, 33.5%-37%, 37%-40%, 40%-42%, 42%-45%, 5%-9%, 9%-9.4%, 9.4%-10%, 10%-10.5%, 10.5%-11%, 11%-15%, 15%-16%, 16%-18%, 18%-20% or 20%-25%.

In some embodiments, the lipid nanoparticle comprises (1) a helper lipid, (2) a structural lipid, (3) a PEG-lipid, and (4) a compound represented by the structural formula (I) of the present disclosure, or a pharmaceutically acceptable salt or stereoisomer thereof, and based on the total amount of the compound represented by the structural formula (I) of the present disclosure or a pharmaceutically acceptable salt or stereoisomer thereof, the helper lipid, the structural lipid and the PEG-lipid, the lipid nanoparticle comprises the structural lipid in an amount (percentage by mole) of following: about 0%-55%, e.g. about 0%-10%, 10%-15.5%, 15.5%-22.5%, 22.5%-35%, 35%-36.5%, 36.5%-39.5%, 39.5%-40.5%, 40.5%-41.5%, 41.5%-45%, 45%-46.5%, 46.5%-50%, 15.5%-18.5%, 18.5%-22.5%, 22.5%-23.5%, 23.5%-28.5%, 28.5%-33.5%, 33.5%-35%, 36.5%-38%, 38%-38.5%, 38.5%-39%, 39%-39.5%, 41.5%-42.5%, 42.5%-42.7%, 42.7%-43%, 43%-43.5%, 43.5%-45% or 46.5%-48.5%.

In some embodiments, the lipid nanoparticle comprises (1) a helper lipid, (2) a structural lipid, (3) a PEG-lipid, and (4) a compound represented by the structural formula (I) of the present disclosure, or a pharmaceutically acceptable salt or stereoisomer thereof, and based on the total amount of the compound represented by the structural formula (I) of the present disclosure or a pharmaceutically acceptable salt or stereoisomer thereof, the helper lipid, the structural lipid and the PEG-lipid, the lipid nanoparticle comprises the PEG-lipid in an amount (percentage by mole) of following: about 0.5%-5%, e.g. about 0.5%-1%, 1%-1.5%, 1.5%-2%, 2%-2.5%, 2.5%-3%, 3%-3.5%, 3.5%-4%, 4%-4.5%, 4.5%-5%, 1.5%-1.6% or 1.6%-2%.

In some embodiments as described above, the lipid nanoparticle comprises (1) a helper lipid, (2) a structural lipid, (3) a PEG-lipid, and (4) a compound represented by the structural formula (I) of the present disclosure or a pharmaceutically acceptable salt or stereoisomer thereof, and the molar ratio of the compound represented by the structural formula (I) of the present disclosure or a pharmaceutically acceptable salt or stereoisomer thereof : the helper lipid : the structural lipid : the PEG-lipid is about (25-75):(5-45):(0-55):(25-65):(5-42):(10-55):(0.5-4), (28-60):(5-42):(15.5-53.5):(0.5-3.5) or (35-60):(5-40):(18.5-53.5):(1.5-3), e.g. 45:11:41.5:2.5, 42:10.5:45:2.5, 42:16:39.5:2.5, 40:16:41.5:2.5, 40:18:39.5:2.5, 35:16:46.5:2.5, 35:25:36.5:3.5, 28:33.5:35:3.5, 32:37:40.5:0.5, 35:40:22.5:2.5, 40:42:15.5:2.5, 45:10:42.5:2.5, 40:20:38.5:1.5, 45:15:38.5:1.5, 55:5:38.5:1.5, 60:5:33.5:1.5, 45:20:33.5:1.5, 50:20:28.5:1.5, 55:20:23.5:1.5, 60:20:18.5:1.5, 40:15:43.5:1.5, 50:15:33.5:1.5, 55:15:28.5:1.5, 60:15:23.5:1.5, 40:10:48.5:1.5, 45:10:43.5:1.5, 55:10:33.5:1.5, 40:5:53.5:1.5, 45:5:48.5:1.5, 50:5:43.5:1.5, 48:10:40.5:1.5, 50:10:38.5:1.5, 50:9:38:3, 49.5:10:39:1.5, 46.3:9.4:42.7:1.6, or 45:9:43:3.

In some embodiments, the lipid nanoparticle comprises DOPE, cholesterol (e.g. CHO-HP), a PEG-lipid, and a compound represented by the structural formula (I) of the present disclosure or a pharmaceutically acceptable salt or stereoisomer thereof, and the molar ratio of the compound represented by the structural formula (I) of the present

disclosure or a pharmaceutically acceptable salt or stereoisomer thereof : DOPE : cholesterol : the PEG-lipid is about (28-60):(5-42):(15.5-53.5):(0.5-3.5).

In some embodiments, the lipid nanoparticle comprises DOPE, cholesterol (e.g. CHO-HP), a PEG-lipid and a compound represented by the structural formula (I) of the present disclosure or a pharmaceutically acceptable salt or stereoisomer thereof, and the molar ratio of the compound represented by the structural formula (I) of the present disclosure or a pharmaceutically acceptable salt or stereoisomer thereof : the helper lipid : the structural lipid : the PEG-lipid is about 45:11:41.5:2.5, 42:10.5:45:2.5, 42:16:39.5:2.5, 40:16:41.5:2.5, 40:18:39.5:2.5, 35:16:46.5:2.5, 35:25:36.5:3.5, 28:33.5:35:3.5, 32:37:40.5:0.5, 35:40:22.5:2.5, 40:42:15.5:2.5, 45:10:42.5:2.5, 40:20:38.5:1.5, 45:15:38.5:1.5, 55:5:38.5:1.5, 60:5:33.5:1.5, 45:20:33.5:1.5, 50:20:28.5:1.5, 55:20:23.5:1.5, 60:20:18.5:1.5, 40:15:43.5:1.5, 50:15:33.5:1.5, 55:15:28.5:1.5, 60:15:23.5:1.5, 40:10:48.5:1.5, 45:10:43.5:1.5, 55:10:33.5:1.5, 40:5:53.5:1.5, 45:5:48.5:1.5, or 50:5:43.5:1.5.

In some embodiments, the lipid nanoparticle comprises DSPC, cholesterol (e.g. CHO-HP), a PEG-lipid and a compound represented by the structural formula (I) of the present disclosure or a pharmaceutically acceptable salt or stereoisomer thereof, and the molar ratio of the compound represented by the compound represented by the structural formula (I) of the present disclosure or a pharmaceutically acceptable salt or stereoisomer thereof : DSPC : cholesterol : PEG-lipid is about (35-60):(5-40):(18.5-53.5):(1.5-3).

In some embodiments, the lipid nanoparticle comprises DSPC, cholesterol (e.g. CHO-HP), a PEG-lipid and a compound represented by the structural formula (I) of the present disclosure or a pharmaceutically acceptable salt or stereoisomer thereof, and the molar ratio of the compound represented by the structural formula (I) of the present disclosure or a pharmaceutically acceptable salt or stereoisomer thereof : the helper lipid : the structural lipid : the PEG-lipid is about 48:10:40.5:1.5, 50:10:38.5:1.5, 50:9:38:3, 49.5:10:39:1.5, 46.3:9.4:42.7:1.6, 45:9:43:3, 45:11:41.5:2.5, 42:10.5:45:2.5, 42:16:39.5:2.5, 40:16:41.5:2.5, 40:18:39.5:2.5, 35:40:22.5:2.5, 40:20:38.5:1.5, 45:15:38.5:1.5, 55:5:38.5:1.5, 60:5:33.5:1.5, 45:20:33.5:1.5, 50:20:28.5:1.5, 55:20:23.5:1.5, 60:20:18.5:1.5, 40:15:43.5:1.5, 50:15:33.5:1.5, 55:15:28.5:1.5, 60:15:23.5:1.5, 40:10:48.5:1.5, 45:10:43.5:1.5, 55:10:33.5:1.5, 40:5:53.5:1.5, 45:5:48.5:1.5, or 50:5:43.5:1.5.

In some embodiments, the lipid nanoparticle comprises (1) a helper lipid, (2) a structural lipid, (3) a PEG-lipid, (4) a compound represented by the structural formula (I) of the present disclosure or a pharmaceutically acceptable salt or stereoisomer thereof, and (5) an active ingredient (e.g. RNA or DNA) or a pharmaceutically acceptable carrier, diluent or excipient (e.g. a buffer).

In some embodiments, the lipid nanoparticle comprises (1) a helper lipid, (2) a structural lipid, (3) a PEG-lipid, (4) a compound represented by the structural formula (I) of the present disclosure, or a pharmaceutically acceptable salt or stereoisomer thereof, (5) an active ingredient, and (6) a pharmaceutically acceptable carrier, diluent or excipient.

In another aspect, the present disclosure provides a pharmaceutical composition comprising the a lipid nanoparticle of the present disclosure, or a compound represented by the structural formula (I) of the present disclosure or a pharmaceutically acceptable salt or stereoisomer thereof. Since the compound represented by the structural formula (I) of the present disclosure or a pharmaceutically acceptable salt or stereoisomer thereof comprises a long non-polar residue, all of the obtained compounds have hydrophobic characteristics, and due to the amino group, they also have hydrophilic characteristics. This amphiphilic characteristic can be used to form lipid nanoparticles, such as lipid bilayers, micelles, liposomes, etc.

In some embodiments, the pharmaceutical composition comprises a pharmaceutically acceptable carrier, diluent or excipient, and one of the following: the compound represented by the structural formula (I) of the present disclosure or a pharmaceutically acceptable salt or stereoisomer thereof, and the lipid nanoparticle of the present disclosure.

In some embodiments, the pharmaceutical composition is a lipid nanoparticle.

The compound represented by the structural formula (I) of the present disclosure or a pharmaceutically acceptable salt or stereoisomer thereof enables the lipid nanoparticle comprising the same has excellent performance of encapsulating a biologically active ingredient, and the lipid nanoparticle of the present disclosure comprising a biologically active ingredient can be used to deliver any one or more of therapeutic agents to a cell, tissue or organ. The present disclosure provides a use of the lipid nanoparticle of the present disclosure in delivering a biologically active ingredient (e.g. DNA, RNA or the like) to a cell, tissue or organ, and also provides a use of the lipid nanoparticle or pharmaceutical composition, which comprise a biologically active ingredient (e.g. DNA, RNA or the like), in producing a polypeptide and/or protein of interest, in manufacture of a medicament, in manufacture of a medicament for nucleic acid transfer, and in preventing and/or treating diseases.

The present disclosure provides a use of a compound represented by the structural formula (I) or a pharmaceutically acceptable salt or stereoisomer thereof, or the lipid nanoparticle of the present disclosure in delivering a biologically active ingredient (e.g. DNA, RNA or the like) to a cell, tissue or organ.

In some embodiments, the use comprises a step of contacting a cell, tissue or organ with the lipid nanoparticle comprising a biologically active ingredient (e.g. a nucleic acid (e.g. mRNA) encoding a polypeptide and/or protein of

interest).

In some embodiments, the tissue or organ is selected from the group consisting of spleen, liver, kidney, lung, femur, eye tissue, vascular endothelium in blood vessels, lymph, and tumor tissue.

In some embodiments, the cell is a mammalian cell. As used herein, the mammalian cell can be a cell of any mammal. In some embodiments, the mammal includes one or more of the following: human, mouse, rat, pig, cat, dog, horse, goat, cattle, and monkey. In some embodiments, the mammal is human.

In some embodiments, the mammalian cell is in vivo in a mammal.

The present disclosure provides a method for producing a polypeptide and/or protein of interest, comprising a step of contacting a cell, tissue or organ with the lipid nanoparticle or pharmaceutical composition of the present disclosure comprising a biologically active ingredient (e.g. a nucleic acid (e.g. mRNA) encoding a polypeptide and/or protein of interest).

In some embodiments, after the cell is contacted with the lipid nanoparticle or pharmaceutical composition, the nucleic acid can be taken up into the cell, and the nucleic acid taken up into the cell is utilized by the cell to produce the polypeptide and/or protein of interest.

In some embodiments, the cell, tissue or organ is the same as that described above in the use of the compound represented by the structural formula (I) or a pharmaceutically acceptable salt or stereoisomer thereof, or the lipid nanoparticle of the present disclosure in delivering a biologically active ingredient (e.g. DNA, RNA or the like) to a cell, tissue or organ, and will not be repeated here.

In some embodiments, the method of producing a polypeptide and/or protein of interest is performed in vivo or partially in vivo.

In another aspect, the present disclosure further provides a use of the compound represented by the structural formula (I) or a pharmaceutically acceptable salt or stereoisomer thereof, the lipid nanoparticle, or the pharmaceutical composition of the present disclosure in manufacture of a medicament.

In some embodiments, the medicament is used in treatment and/or prevention of a disease.

In some embodiments, the disease is selected from the group consisting of rare diseases, infectious diseases, cancers, genetic diseases, autoimmune diseases, diabetes, neurodegenerative diseases, cardiovascular diseases, renal vascular diseases, and metabolic diseases.

In some embodiments, the cancer is selected from one or more of lung cancer, stomach cancer, liver cancer, esophagus cancer, colon cancer, pancreatic cancer, brain cancer, lymphatic cancer, blood cancer, and prostatic cancer. In some embodiments, the genetic disease is selected from one or more of hemophilia, thalassemia, and Gaucher's disease.

In some embodiments, the rare diseases include one or more of the following: Brittle Bone Disease, Wilson Disease, Spinal Muscular Atrophy (SMA), Huntingdon's Disease, Rett Syndrome, Amyotrophic Lateral Sclerosis (ALS), Duchenne Type Muscular dystrophy, Friedrichs Ataxia, methylmalonic acidemia (MMA), Cystic Fibrosis (CF), glycogen storage disease 1a (GSD1a), glycogen storage disease III (GSD III), Crigler-Najjar syndrome, ornithine transcarbamylase deficiency (OTCD), propionic acidemia (PA), phenylketonuria (PKU), hemophilia A, hemophilia B, β-thalassemia, Lafora disease, Dravet syndrome (DS), Alexander disease, Leber's congenital amaurosis (LCA), myelodysplastic syndrome (MDS), Homocystinuria due to CBS deficiency. In addition, included herein are also the rare diseases listed at www.orpha.net/consor/cgi-bin/Disease_Search_List.php and rarediseases.info.nih.gov/diseases.

In some embodiments, the medicament is used in, e.g., gene therapy, protein replacement therapy, antisense therapy, or interfering RNA therapy, or genetic vaccination.

In some embodiments, the medicament is a nucleic acid medicament. In some embodiments, the nucleic acid in the nucleic acid medicament includes at least one of the following: RNA, messenger RNA (mRNA), antisense oligonucleotide, DNA, plasmid, ribosomal RNA (rRNA), microRNA (miRNA), transfer RNA (tRNA), small interfering RNA (siRNA), small nuclear RNA (snRNA), small hairpin RNA (shRNA), single-stranded guide RNA (sgRNA) and cas9 mRNA.

In some embodiments, the medicament is a vaccine. In some embodiments, the medicament is used in antigen producing the pathogen or a part thereof.

In some embodiments, the medicament is a gene vaccine. In some embodiments, the medicament is a nucleic acid vaccine. In some embodiments, the medicament is an mRNA vaccine.

In some embodiments, the gene vaccine is inoculated for treatment and/or prevention of one or more of cancer, allergy, toxicity, and pathogen infection.

In some embodiments, the pathogen is selected from one or more of viruses, bacteria and fungi.

In some embodiments, the medicament is a gene therapy agent. In some embodiments, the medicament is used in production of a protein associated with a genetic disease.

In some embodiments, the medicament is used in production of an antibody, such as scFV or nanoantibody.

In yet another aspect, the present disclosure further provides a use of the compound represented by structural formula (I) or a pharmaceutically acceptable salt or stereoisomer thereof, the lipid nanoparticle or the pharmaceutical composition of the present disclosure in the manufacture of a medicament for nucleic acid transfer.

In some embodiments, the nucleic acid in the medicament for nucleic acid transfer is selected from the group

consisting of RNA, antisense oligonucleotide and DNA.

In some embodiments, the RNA is selected from messenger RNA (mRNA), ribosomal RNA (rRNA), microRNA (miRNA), transfer RNA (tRNA), small interfering RNA (siRNA), small nuclear RNA (snRNA), small hairpin RNA (shRNA), single-stranded guide RNA (sgRNA), cas9 mRNA, or a mixture thereof.

In some embodiments, the DNA is a plasmid.

In addition, the present disclosure also provides a medicament comprising the compound represented by the structural formula (I) or a pharmaceutically acceptable salt or stereoisomer thereof, or the lipid nanoparticle of the present disclosure.

In addition, the present disclosure also provides a method for preventing and/or treating a disease, comprising administering to a subject in need thereof the lipid nanoparticle, the pharmaceutical composition or the medicament comprising the compound represented by structural formula (I) of the present disclosure or a pharmaceutically acceptable salt or stereoisomer thereof and a biologically active ingredient (e.g., a nucleic acid (e.g., mRNA) encoding a polypeptide and/or protein of interest).

In some embodiments, the disease or condition can be referred to those as described in use of the compound represented by structural formula (I) or a pharmaceutically acceptable salt or stereoisomer thereof, the lipid nanoparticle or pharmaceutical composition of the present disclosure in the manufacture of a medicament as recited above.

In another respect, the present disclosure provides a use of the pharmaceutical composition, especially the lipid nanoparticle, in the manufacture of a medicament ,which is used for, e.g., gene therapy, genetic vaccination, antisense therapy or treatment through RNA interference. Preferably, the gene therapy can be used for treatment of a cancer or a genetic disease. The cancer is preferably selected from one or more of lung cancer, gastric cancer, liver cancer, esophageal cancer, colon cancer, pancreatic cancer, brain cancer, lymphoma, blood cancer and prostate cancer; and the genetic disease is preferably selected from one or more of hemophilia, thalassemia, and Gaucher's disease. The gene vaccine is preferably inoculated for treatment of cancer, allergies, toxicity and pathogen infection. The pathogen is preferably selected from one or more of viruses, bacteria and fungi.

In another aspect, the present disclosure relates to a use of the compound or a pharmaceutically acceptable salt or stereoisomer thereof, the lipid nanoparticle comprising the compound or a pharmaceutically acceptable salt or stereoisomer thereof, in the manufacture of a medicament for nucleic acid transfer. Preferably, the nucleic acid is selected from the group consisting of RNA, DNA and antisense oligonucleotides. Preferably, the RNA is selected from the group consisting of messenger RNA (mRNA), ribosomal RNA (rRNA), microRNA (miRNA), transfer RNA (tRNA), small interfering RNA (siRNA) and small nuclear RNA (snRNA); and preferably, the DNA is a plasmid.

In another aspect, the present disclosure also provides a method for preparing the compound of formula (I) as described above, and the general procedures of the method are as follows.

General procedure 1:

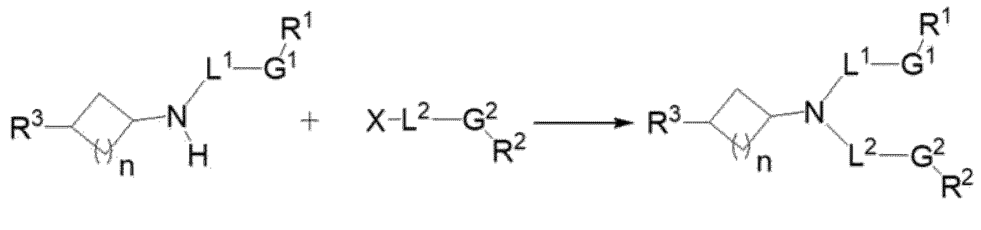

II III I

wherein,

n, $L^1$, $L^2$, $G^1$, $G^2$, $R^1$, $R^2$, $R^3$ have the same meanings as those defined above for the compound of formula (I) of the present disclosure;

X is a halogen; preferably, X is bromine.

Specifically, the compound of formula (I) of the present disclosure is obtained by subjecting the intermediate (II) and the intermediate (III) to a substitution reaction in an organic solvent at room temperature in the presence or absence of an acid-binding agent and an iodide.

Wherein the organic solvent is selected from the group consisting of nitriles, alcohols, halogenated hydrocarbons, amides and aromatic hydrocarbons, such as acetonitrile, methanol, ethanol, dichloromethane and dichloroethane (DCE); the acid-binding agent is selected from the group consisting of organic and inorganic bases, such as sodium hydroxide, potassium hydroxide, sodium carbonate, potassium carbonate, triethylamine and DIPEA; and the

iodide is, e.g., potassium iodide.

General Procedure 2

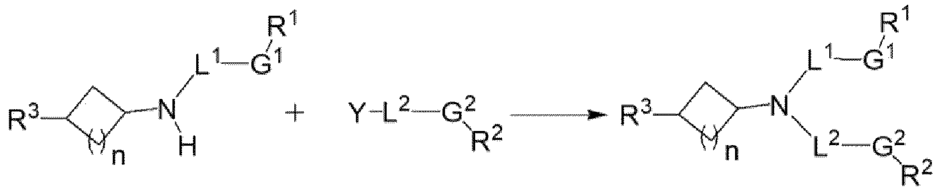

II V I

wherein,
n, $L^1$, $L^2$, $G^1$, $G^2$, $R^1$, $R^2$, $R^3$ have the same meanings as those defined above for the compound of formula (I) of the present disclosure;

Y is (=O).

Specifically, the compound of formula (I) of the present disclosure is obtained by subjecting the intermediate (II) and the intermediate (V) to a reduction reaction in an organic solvent at room temperature in the presence of a reducing agent.
Wherein, the organic solvent is selected from the group consisting of nitriles, alcohols, halogenated hydrocarbons, amides and aromatic hydrocarbons, such as acetonitrile, methanol, ethanol, dichloromethane and dichloroethane (DCE); and the reducing agent is, e.g., sodium triacetoxyborohydride.
Further, when $L^1$ and $L^2$ are the same, $R^1$ and $R^2$ are the same and $G^1$ and $G^2$ are the same in the compound of formula (I) of the present disclosure, the compound of formula (I) of the present disclosure is prepared by the following general procedure 3.

General Procedure 3

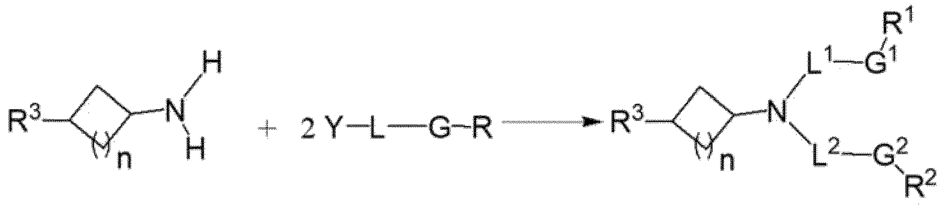

IV VI I

wherein,
n, $L^1$, $L^2$, $G^1$, $G^2$, $R^1$, $R^2$, $R^3$ have the same meanings as defined above for the compound of formula (I) of the present disclosure; and $L = L^1 = L^2$, $G = G^1 = G^2$, $R = R^1 = R^2$.
Specifically, the compound of formula (I) of the present disclosure is obtained by subjecting the intermediate (IV) and the intermediate (VI) to a reduction reaction in an organic solvent at room temperature in the presence of a reducing agent.
Wherein the organic solvent is selected from the group consisting of nitriles, alcohols, halogenated hydrocarbons, amides and aromatic hydrocarbons, such as acetonitrile, methanol, ethanol, dichloromethane and dichloroethane (DCE); and the reducing agent is, e.g., sodium triacetoxyborohydride.
In another aspect, the present disclosure also provides an intermediate (II) for preparing the compound of formula (I) of the present disclosure, having a structural formula shown below:

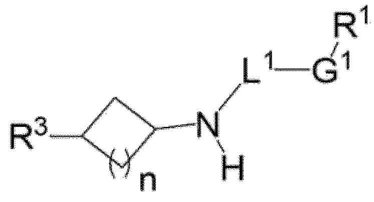

II

wherein,

$L^1$ is selected from the group consisting of $C_1$-$C_{12}$ alkylene, $C_2$-$C_{12}$ alkenylene and $C_2$-$C_{12}$ alkynylene; preferably, $L^1$ is selected from the group consisting of $C_3$-$C_{10}$ alkylene, $C_3$-$C_{10}$ alkenylene and $C_3$-$C_{10}$ alkynylene; further preferably, $L^1$ is selected from the group consisting of $C_3$-$C_{10}$ alkylene; most preferably, $L^1$ is selected from the group consisting of $C_5$-$C_8$ alkylene;

$G^1$ is selected from the group consisting of -O-(C=O)-, -(C=O)-O-, - C(=O)-, -O-, -C(=O)-S- and -S-C(=O)-; preferably, $G^1$ is selected from the group consisting of -O-(C=O)-, -(C=O)-O-, -C(=O)-, and -O-; most preferably, $G^1$ is selected from the group consisting of -O-(C=O)- and - (C=O)-O-;

$R^1$ is selected from the group consisting of $C_5$-$C_{27}$ alkyl and $C_5$-$C_{27}$ alkenyl comprising one or more double bonds, which are attached via any one carbon thereof; preferably, $R^1$ is selected from the group consisting of $C_8$-$C_{20}$ alkyl and $C_8$-$C_{20}$ alkenyl comprising one or more double bonds, which are attached via any one carbon thereof; further preferably, $R^1$ is selected from the group consisting of $C_9$-$C_{17}$ alkyl and $C_9$-$C_{18}$ alkenyl comprising one or two double bonds, which are attached via any one carbon thereof; most preferably, $R^1$ is selected from the group consisting of

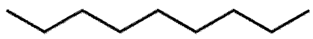,
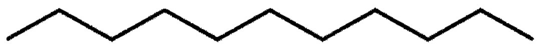,

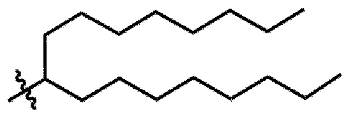,
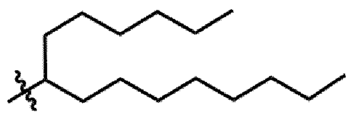,

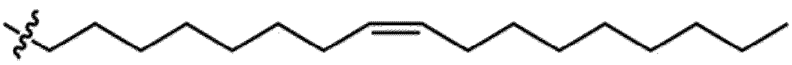,

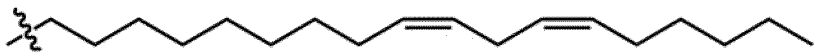,

and

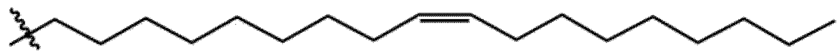;

$R^3$ is selected from the group consisting of halogen, hydroxy, cyano, $C_1$-$C_6$ alkyl, nitro, $C_1$-$C_6$ alkyloxy, $C_1$-$C_6$ alkylcarbonyloxy, $C_1$-$C_6$ alkyloxycarbonyl, $C_1$ -$C_6$ alkylaminocarbonyl and $C_1$-$C_6$ alkylcarbonylamino; preferably, $R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl and $C_1$-$C_6$ alkylcarbonylamino; further preferably, $R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl and $C_1$-$C_4$ alkylcarbonylamino; most preferably, $R^3$ is selected from the group consisting of fluoro, hydroxy, cyano, methoxy, acetoxy, methoxycarbonyl, butylaminocarbonyl and acetamido;

n is selected from the group consisting of 1, 2, and 3.

Preferably, the present disclosure provides the above intermediate (II) or a pharmaceutically acceptable salt or stereoisomer thereof, wherein:

$L^1$ is selected from the group consisting of $C_3$-$C_{10}$ alkylene, $C_3$-$C_{10}$ alkenylene and $C_3$-$C_{10}$ alkynylene;
$G^1$ is selected from the group consisting of -O-(C=O)-, -(C=O)-O-, - C(=O)- and -O-;
$R^1$ is selected from the group consisting of $C_8$-$C_{20}$ alkyl and $C_8$-$C_{20}$ alkenyl comprising one or more double bonds, which are attached via any one carbon thereof;
$R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl and $C_1$-$C_6$ alkylcarbonylamino;
n is selected from the group consisting of 1, 2, and 3.

Further preferably, the present disclosure provides the above intermediate (II) or a pharmaceutically acceptable salt or stereoisomer thereof, wherein:

$L^1$ is selected from the group consisting of $C_3$-$C_{10}$ alkylene;
$G^1$ is selected from the group consisting of -O-(C=O)- and -(C=O)-O-;
$R^1$ is selected from the group consisting of $C_9$-$C_{17}$ alkyl and $C_9$-$C_{18}$ alkenyl comprising one or two double bonds, which are attached via any one carbon thereof;
$R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl and $C_1$-$C_4$ alkylcarbonylamino;
n is selected from the group consisting of 1, 2, and 3.

Still preferably, the present disclosure provides the above intermediate (II) or a pharmaceutically acceptable salt or stereoisomer thereof, wherein:

$L^1$ is selected from the group consisting of $C_5$-$C_8$ alkylene;
$G^1$ is selected from the group consisting of -O-(C=O)- and -(C=O)-O-;
$R^1$ is selected from the group consisting of

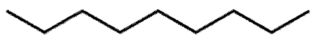,
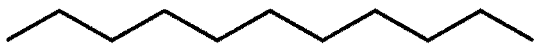,
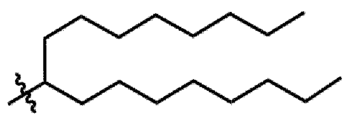,
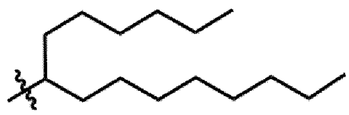,
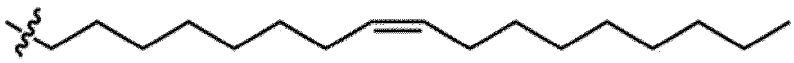,
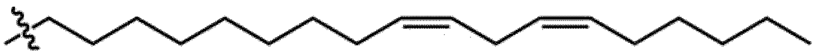,

and

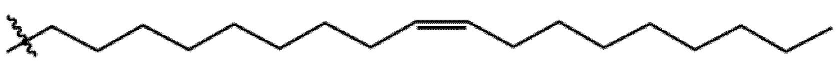;

$R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

Most preferably, the intermediate (II) is selected from the group consisting of:

| No. | Structure |
|---|---|
| II-1 | 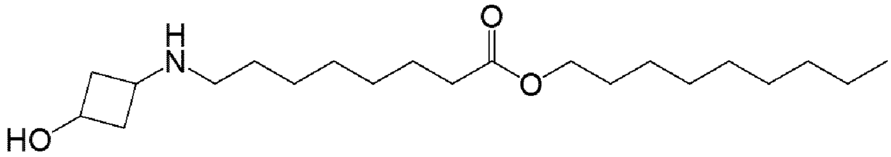 |
| II-2 | 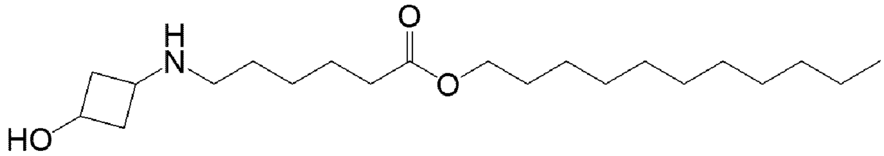 |
| II-4 | 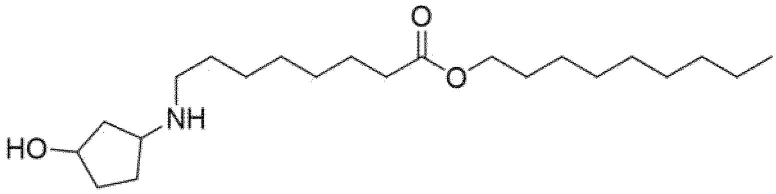 |
| II-5 | 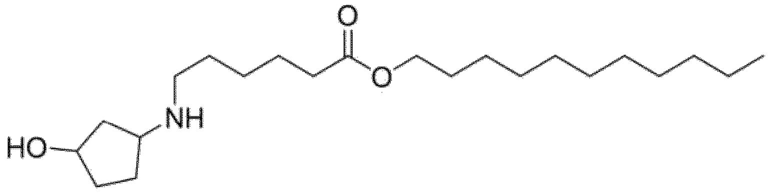 |
| II-6 | 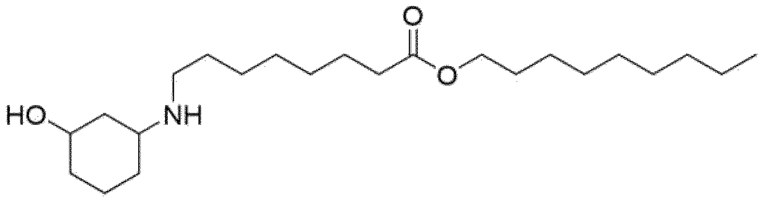 |
| II-7 | 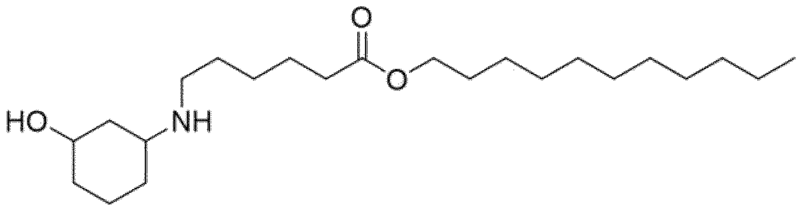 |
| II-8 | 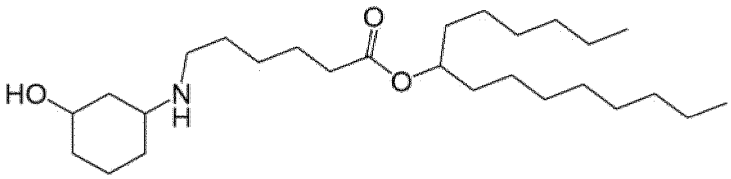 |
| II-9 | 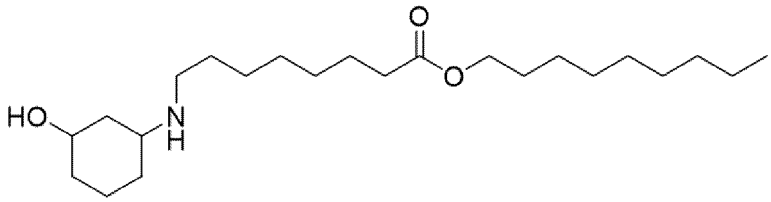 |

(continued)

| No. | Structure |
|---|---|
| II-10 | 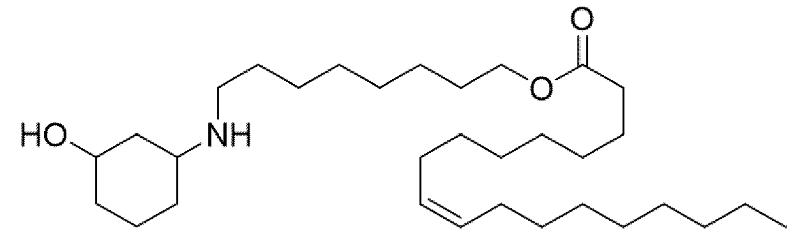 |
| II-11 | 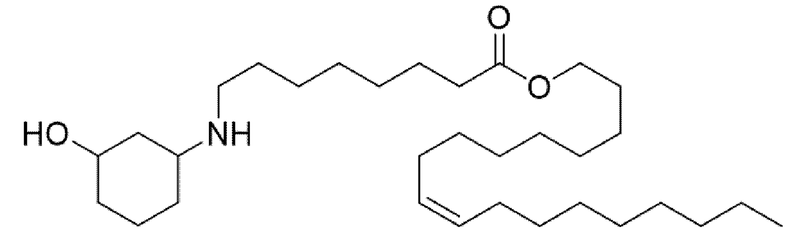 |
| II-12 | 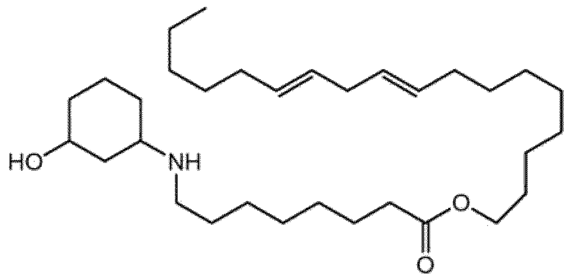 |
| II-14 | 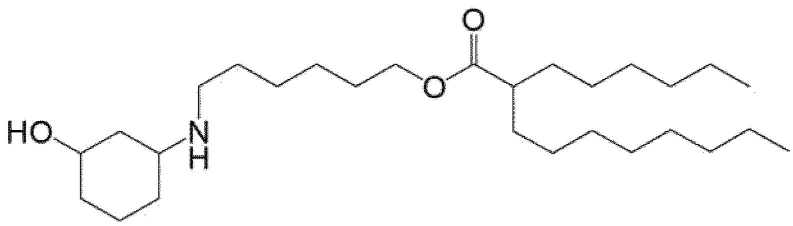 |
| II-15 | 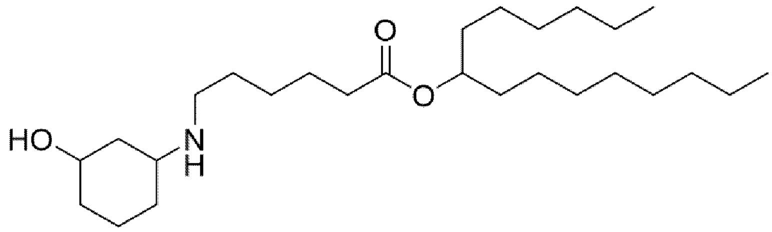 |
| II-16 | 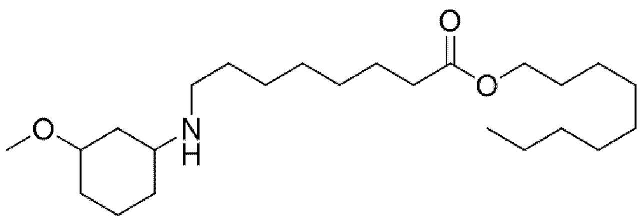 |

(continued)

| No. | Structure |
|---|---|
| II-17 | 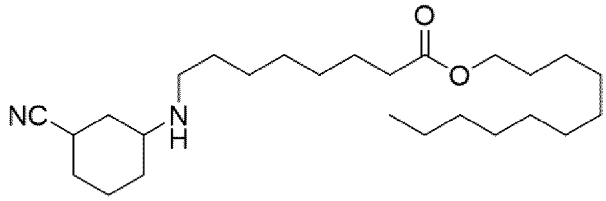 |
| II-18 | 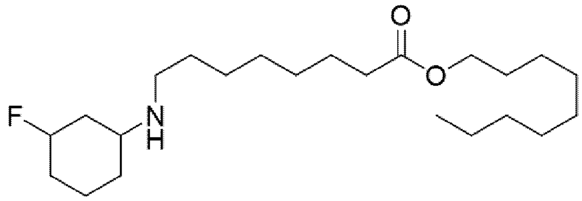 |
| II-19 | 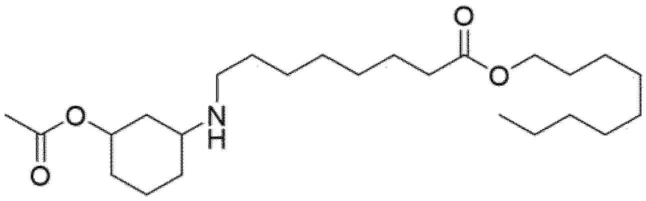 |
| II-20 | 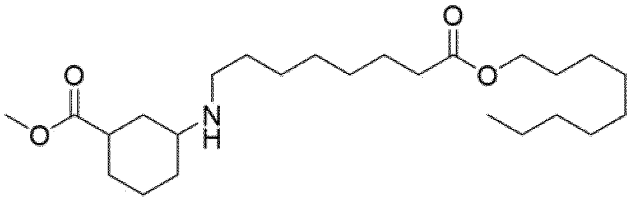 |
| II-21 | 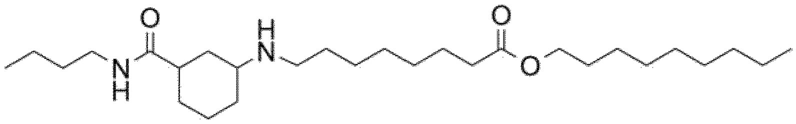 |
| II-22 | 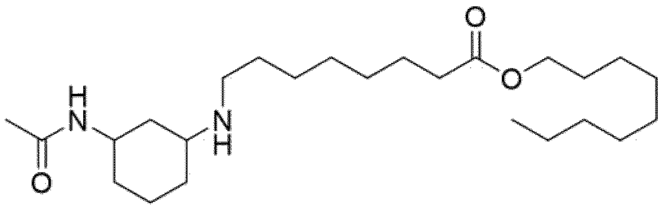 |

Furthermore, the present disclosure also provides a method for preparing the intermediate (II), and the general procedure of the method is as follows:

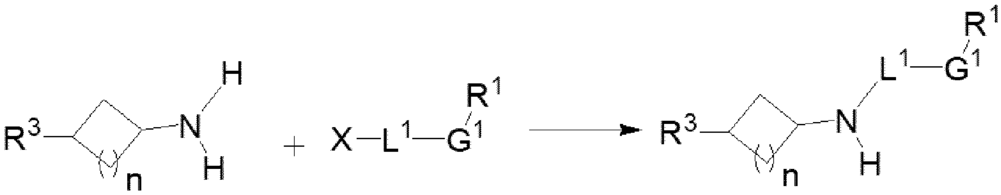

IV VII II

wherein, $R^1$, $R^3$, $G^1$, $L^1$, and n have the same meanings as those defined above for the intermediate (II); and X is a halogen, preferably bromine.

Specifically, the intermediate (II) is prepared by subjecting the intermediate (IV) and the intermediate (VII) to a substitution reaction in an organic solvent in the presence of an acid-binding agent at room temperature. The organic solvent is selected from the group consisting of nitriles, alcohols, halogenated hydrocarbons, amides and aromatic hydrocarbons, such as acetonitrile, methanol, ethanol, dichloromethane and dichloroethane (DCE); and the acid-binding agent is selected from the group consisting of organic and inorganic bases, such as sodium hydroxide, potassium hydroxide, sodium carbonate, potassium carbonate, triethylamine and DIPEA.

In another aspect, the present disclosure provides a method for preparing an optical isomer (X) of the compound of formula (I) of the present disclosure, and the general procedure of the method is as follows:

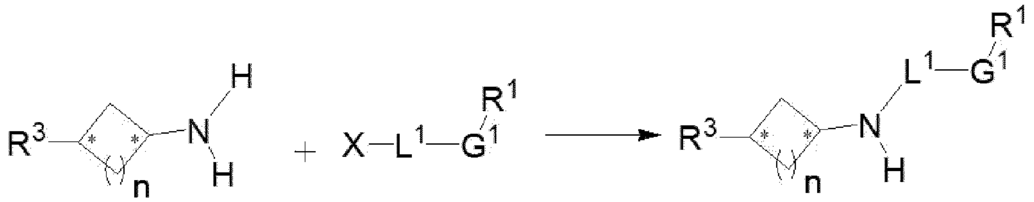

VIII VII VIIII

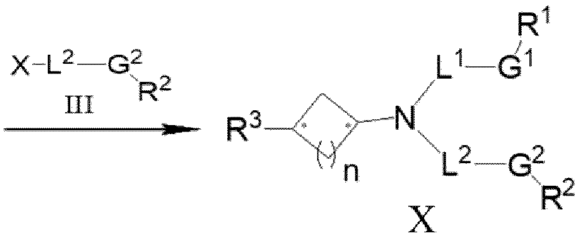


X

wherein, n, $L^1$, $L^2$, $G^1$, $G^2$, $R^1$, $R^2$, and $R^3$ have the same meanings as those defined above for the compound of formula (I); and X is a halogen, preferably bromine.

Specifically, the intermediate (VIIII) is first prepared by subjecting the intermediate (VIII) and the intermediate (VII) to a substitution reaction in an organic solvent in the presence of an acid-binding agent, wherein the organic solvent is selected from the group consisting of nitriles, alcohols, halogenated hydrocarbons, amides and aromatic hydrocarbons, such as acetonitrile, methanol, ethanol, dichloromethane and dichloroethane (DCE); and the acid-binding agent is selected from the group consisting of organic and inorganic bases, such as sodium hydroxide, potassium hydroxide, sodium carbonate, potassium carbonate, triethylamine and DIPEA. Then, the optical isomer (X) of the compound (I) is prepared by subjecting the intermediate (VIIII) and the intermediate (III) to a reaction in the presence or absence of an acid-binding agent and an iodide, wherein the organic solvent is selected from the group consisting of nitriles, alcohols, halogenated hydrocarbons, amides and aromatic hydrocarbons, such as acetonitrile, methanol, ethanol, dichloromethane and dichloroethane (DCE); the acid-binding agent is selected from the group consisting of organic and inorganic bases, such as sodium hydroxide, potassium hydroxide, sodium carbonate, potassium carbonate, triethylamine and DIPEA; and the iodide is, e.g., KI.

Furthermore, the present disclosure provides a method for preparing the optical isomer (I-6) of the compound 6 of the present disclosure, and the general procedure of the method is as follows:

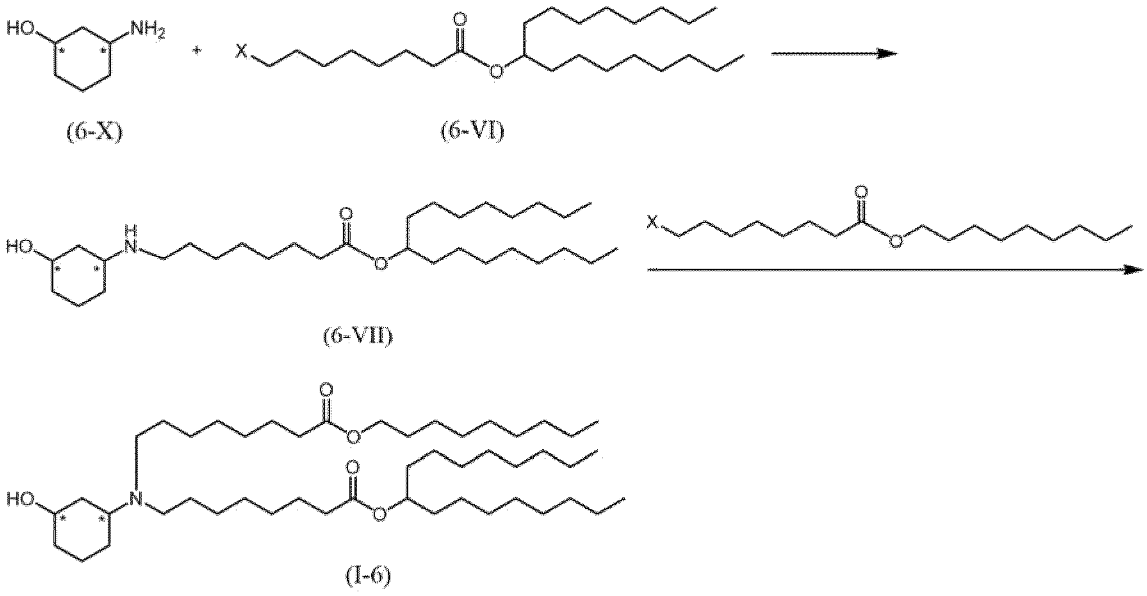

(6-X) (6-VI)

(6-VII)

(I-6)

wherein, X is a halogen, preferably bromine.

Specifically, the optical isomer (I-6) of the compound 6 of the present disclosure is prepared by the following steps:

the compound (6-VII) is prepared by subjecting the compound (6-X) and the compound (6-VI) to a N-alkylation reaction at 30-50 °C in a solvent such as nitriles, alcohols, halogenated hydrocarbons, amides or aromatic hydrocarbons, specifically such as acetonitrile, methanol, ethanol, dichloromethane, dichloroethane (DCE), etc.;
the compound (I-6) is prepared by subjecting the compound (6-VII) and nonyl 8-halogenated octanoate to a N-alkylation reaction at 60-110 °C in a solvent, such as nitriles, alcohols, halogenated hydrocarbons, amides, aromatic hydrocarbons or ether solvents, specifically such as acetonitrile, methanol, ethanol, dichloromethane, dichloroethane (DCE), cyclopentane methyl ether, methyl tert-butyl ether, etc., in the presence or absence of an acid-binding agent and a catalyst, wherein the acid-binding agent is selected from the group consisting of organic and inorganic bases, such as sodium hydroxide, potassium hydroxide, sodium carbonate, potassium carbonate, triethylamine and DIPEA; and the catalyst is an iodide, preferably KI.

Further, according to the above method, the compound (I-6-II) as an optical isomer of the compound 6 is obtained when the compound (6-X) is (1S,3R)-3-aminocyclohexanol, and the compound (6-VII) is as shown in the structural formula (6-VII-I).

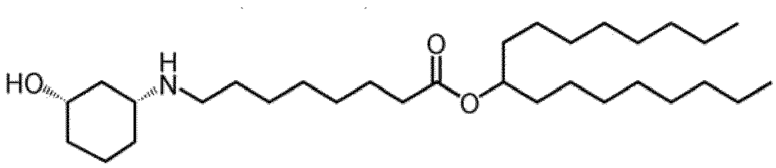

(6-VII-I)

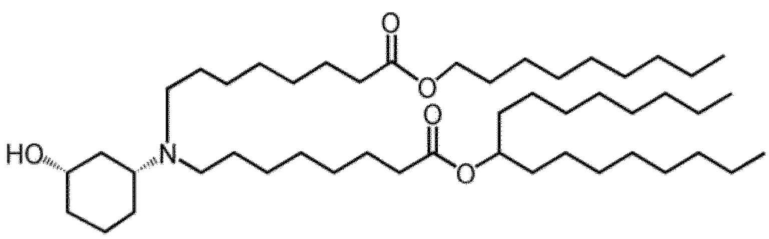

(I-6-II)

The compound (I-6-III) as an optical isomer of the compound 6 is obtained when the compound (6-X) is (1S,3S)-3-aminocyclohexanol, and the compound (6-VII) is as shown in the structural formula (6-VII-II).

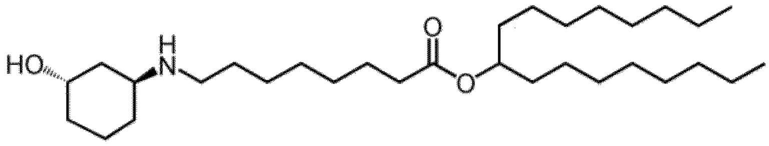

(6-VII-II)

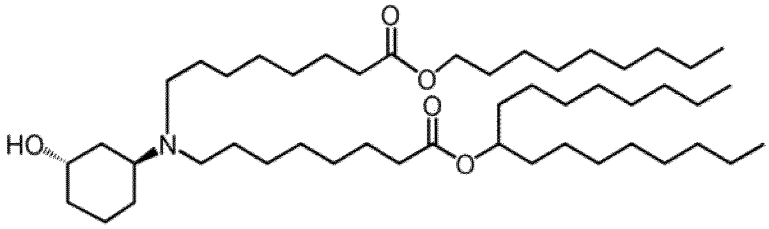

(I-6-III)

The compound (I-6-IV) as an optical isomer of the compound 6 is obtained when the compound (6-X) is (1R,3R)-3-aminocyclohexanol, and the compound (6-VII) is as shown in the structural formula (6-VII-III).

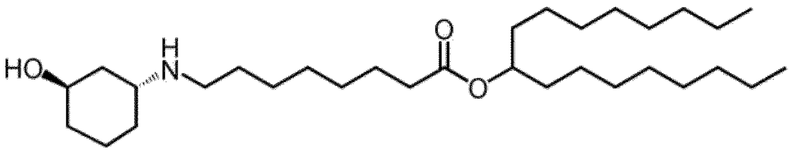

(6-VII-III)

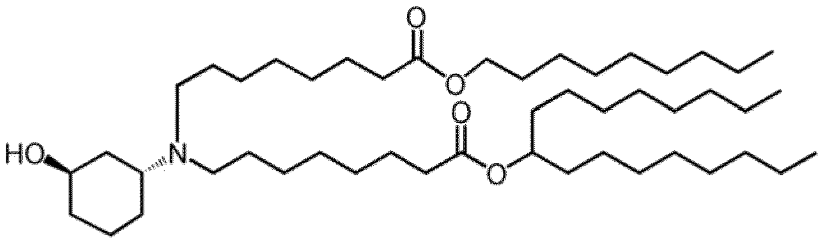

(I-6-IV)

The compound (I-6-V) as an optical isomer of the compound 6 is obtained when the compound (6-X) is (1R,3S)-3-aminocyclohexanol, and the compound (6-VII) is as shown in the structural formula (6-VII-IV).

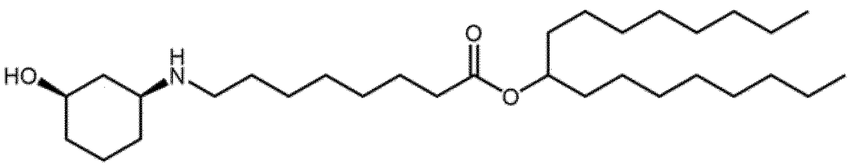

(6-VII-IV)

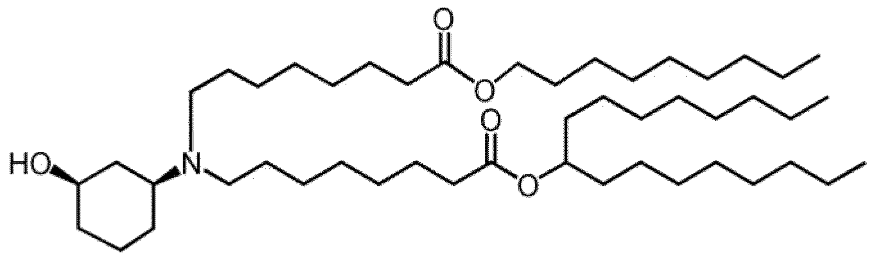

(I-6-V)

In another aspect, the present disclosure provides the intermediate (6-VII) having the following structure for preparing the optical isomer (I-6) of the above compound 6:

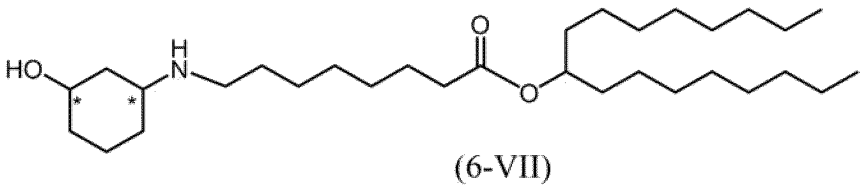

(6-VII)

.

Specifically, the intermediate (6-VII) is selected from the group consisting of

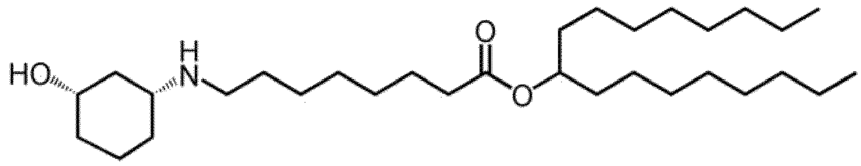

(6-VII-I)

,

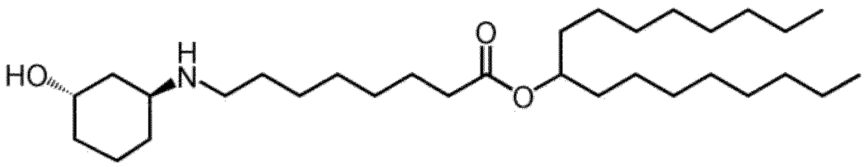

(6-VII-II)

,

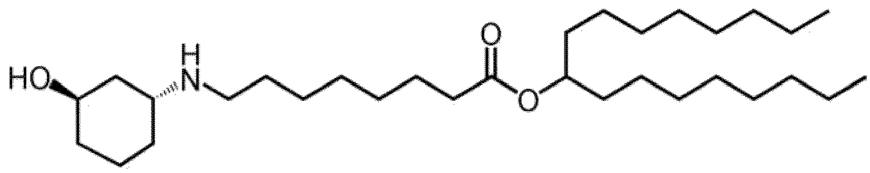

(6-VII-III)

and

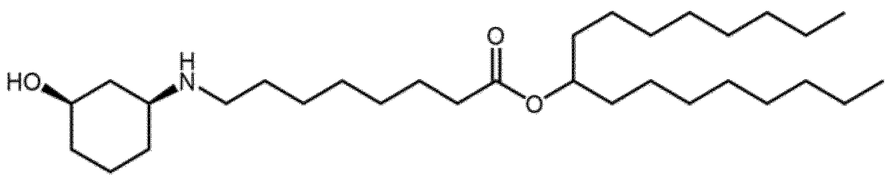

(6-VII-IV)

.

## BRIEF DESCRIPTION OF THE DRAWINGS

FIG. 1 shows humoral antibody titers generated by delivering OVA mRNA through subcutaneous administration of representative amino lipid compounds, e.g., in the test example 2 of the biological experiments of the present disclosure.
FIG. 2 shows humoral antibody titers generated by delivering influenza mRNA vaccine through intramuscular administration of representative amino lipid compounds, e.g., in the test example3 of the biological experiments of the present disclosure.
FIG. 3 is a HPLC spectrum of a mixture of the compounds (I-6-II), (I-6-III), (I-6-IV) and (I-6-V).
FIG. 4 is a HPLC spectrum of the compound (I-6-II).
FIG. 5 is a HPLC spectrum of the compound (I-6-III).
FIG. 6 is a HPLC spectrum of the compound (I-6-IV).
FIG. 7 is a HPLC spectrum of the compound (I-6-V).
FIG. 8 shows fluorescence intensities in liver after intramuscular injection in the targeting assay.
FIG. 9 shows statistics of severe swelling at the injection site and moderate lameness in the adverse reaction assay by intramuscular injection.

Various exemplary embodiments of the amino lipid compound, the composition and the lipid nanoparticle comprising the same, and their use in delivering a bioactive agent such as a nucleic acid into cells are described in further detail below.

## DETAILED DESCRIPTION OF THE INVENTION

### DEFINITIONS

Unless the context requires otherwise, throughout the present specification and claims, the word "comprise" and variations thereof, such as, "comprises" and "comprising" are to be construed in an open and inclusive sense, that is, as "including, but not limited to".

Reference throughout this specification to "one embodiment" or "an embodiment" means that a particular feature, structure or characteristic described in connection with the embodiment is included in at least one embodiment of the present disclosure. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Unless defined otherwise, all technical and scientific terms used herein have the same meaning as is commonly understood by one of skilled in the art to which this disclosure belongs.

The expression "$C_x$-$C_y$" as used in the present disclosure represents the range of carbon atoms, wherein x and y are both integers, e.g., $C_3$-$C_8$ cycloalkyl refers to a cycloalkyl having 3-8 carbon atoms, $C_0$-$C_2$ alkyl refers to an alkyl having 0-2 carbon atoms, wherein $C_0$ alkyl refers to a chemical single bond.

In this disclosure, the term "alkyl" refers to a saturated aliphatic hydrocarbon group, including a straight or branched group of 1 to 30 carbon atoms, e.g., it may be a straight or branched group of 1 to 6 carbon atoms, 5 to 27 carbon atoms, 8 to 20 carbon atoms, or 9 to 17 carbon atoms. Non-limiting examples include n-nonyl, undecyl, 7-pentadecyl, 9-heptadecyl and various isomers thereof, etc.

In this disclosure, the term "cycloalkyl" refers to a saturated monocyclic or polycyclic cyclic hydrocarbon group, including 3 to 12 ring atoms, e.g., it may have 3 to 12, 3 to 10, 3 to 8, or 3 to 6 ring atoms, or it may be a 3-, 4-, 5-, or 6-membered ring. Non-limiting examples of the monocyclic cycloalkyl include cyclopropyl, cyclobutyl, cyclopentyl, cyclohexyl, cycloheptyl, cyclooctyl, etc.

In this disclosure, the term "alkenyl" refers to an unsaturated aliphatic hydrocarbon group having at least one double bond, including straight and branched alkenyls of 1 to 30 carbon atoms, e.g., it may be a straight and branched alkenyl of 5 to 27, 8 to 20, or 9 to 18 carbon atoms with one or two double bonds. Non-limiting examples include 8-heptadecenyl, 12-octadecadienyl and various isomers thereof, etc.

In this disclosure, the term "alkynyl" refers to an unsaturated aliphatic hydrocarbon group having at least one triple bond, including straight and branched alkynyls of 1 to 30 carbon atoms, e.g., it may be a straight and branched alkynyl of 5 to 27, 5 to 15, or 8 to 10 carbon atoms with one or two triple bonds. Non-limiting examples include 2-nonynyl, 3-decynyl and various isomers thereof, etc.

In this disclosure, the term "alkylene" refers to a substituted or unsubstituted alkyl having two terminal monovalent radical cores, which is produced by removing one hydrogen atom from each of the two terminal atoms;

the alkyl has the meaning as described above. Non-limiting examples of "alkylene" include $C_3$-$C_{10}$ alkylene, $C_5$-$C_8$ alkylene, etc.

In this disclosure, the term "alkenylene" refers to a substituted or unsubstituted alkenyl having two terminal monovalent radical cores, which is produced by removing a hydrogen atom from each of the two terminal atoms; the alkenyl has the meaning as described above. Non-limiting examples of "alkenylene" include $C_3$-$C_{10}$ alkenylene, etc.

In this disclosure, the term "alkynylene" refers to a substituted or unsubstituted alkynyl having two terminal monovalent radical cores, which is produced by removing a hydrogen atom from each of the two terminal atoms; the alkynyl has the meaning as described above. Non-limiting examples of "alkynylene" include $C_3$-$C_{10}$ alkynylene, etc.

In this disclosure, the term "halogen" refers to fluorine, chlorine, bromine or iodine.

In this disclosure, the term "alkoxy" refers to alkyl-oxy, wherein the alkyl has the meaning as described above. Preferably, the alkoxy is $C_1$-$C_{10}$ alkoxy; more preferably, the alkoxy is $C_1$-$C_6$ alkoxy; further preferably, the alkoxy is $C_1$-$C_4$ alkoxy; most preferably, the alkoxy is methoxy.

In this disclosure, the term "alkylcarbonyloxy" refers to alkyl-C(O)O-group, wherein the alkyl has the meaning as described above. Preferably, the alkylcarbonyloxy is $C_1$-$C_{10}$ alkylcarbonyloxy; more preferably, the alkylcarbonyloxy is $C_1$-$C_6$ alkylcarbonyloxy; further preferably, the alkylcarbonyloxy is $C_1$-$C_4$ alkylcarbonyloxy; most preferably, the alkylcarbonyloxy group is acetoxy.

In this disclosure, the term "alkoxycarbonyl" refers to alkyl-OC(O)-group, wherein the alkyl has the meaning as described above. Preferably, the alkoxycarbonyl is $C_1$-$C_{10}$ alkoxycarbonyl; more preferably, the alkoxycarbonyl is $C_1$-$C_6$ alkoxycarbonyl; further preferably, the alkoxycarbonyl is $C_1$-$C_4$ alkoxycarbonyl; most preferably, the alkoxycarbonyl is methoxycarbonyl.

In this disclosure, the term "alkylcarbonylamino" refers to alkyl-C(O)NH- group, wherein the alkyl has the meaning as described above. Preferably, the alkylcarbonylamino is $C_1$-$C_{10}$ alkylcarbonylamino; more preferably, the alkylcarbonylamino is $C_1$-$C_6$ alkylcarbonylamino; further preferably, the alkylcarbonylamino is $C_1$-$C_4$ alkylcarbonylamino; most preferably, the alkylcarbonylamino is acetamido.

In this disclosure, the term "alkylaminocarbonyl" refers to alkyl-NHC(O)- group, wherein the alkyl has the meaning as described above. Preferably, the alkylaminocarbonyl is $C_1$-$C_{10}$ alkylaminocarbonyl; more preferably, the alkylaminocarbonyl is a $C_1$-$C_6$ alkylaminocarbonyl; further preferably, the alkylaminocarbonyl is $C_1$-$C_4$ alkylaminocarbonyl; most preferably, the alkylaminocarbonyl is butylaminocarbonyl.

In this disclosure, the term "optionally substituted" refers to one or more hydrogen atoms attached to an atom or group are independently unsubstituted or substituted with one or more (e.g., 1, 2, 3 or 4) substituents. The substituents may be independently selected from the group consisting of, but are not limited to, a halogen (e.g., chlorine, bromine, fluorine or iodine), a deuterium (D), a tritium (T), a carboxylic acid (e.g., -C(=O)OH), an oxygen (e.g., =O), a sulfur (e.g., =S), a hydroxyl (e.g., -OH), an ester group(e.g., -C(=O)ORi or -OC(=O)Ri), an aldehyde group (e.g., -C(=O)H), a carbonyl group (e.g., -C(=O)Ri, or represented by C=O), an acyl halide group (e.g., -C(=O)X, wherein X is selected from the group consisting of bromine, fluorine, chlorine and iodine), a carbonate group (e.g., - OC(=O)ORi), an alkoxy (e.g., -ORi), an acetal (e.g., -C(ORi)2Ri, wherein each ORi is the same or different alkoxy), a phosphate (e.g., $P(=O)_4^{3-}$), a thiol (e.g., -SH), a sulfoxide (e.g., -S(=O)Ri-), a sulfinic acid (e.g., - S(=O)OH), a sulfonic acid (e.g., $-S(=O)_2OH$), a thioaldehyde (e.g., -C(=S)H), a sulfate (e.g., $S(=O)_4^{2-}$), a sulfonyl (e.g., $-S(=O)_2Ri$), a sulfinyl (e.g., -S(=O)Ri), an amide (e.g., -C(=O)N(Ri)2 or -N(Ri)C(=O)Ri), an azido (e.g., $-N_3$), a nitro (e.g., -NO2), a cyano (e.g., -CN), an isocyano (e.g., -NC), an acyloxy (e.g., -OC(=O)Ri), an amino (e.g., $-N(Ri)_2$, -N(Ri)H or $-NH_2$), a carbamoyl (e.g., $-OC(=O)N(Ri)_2$, -OC(=O)N(Ri)H or $-OC(=O)NH_2$), a sulfonamide (e.g., $-S(=O)_2N(Ri)_2$, $-S(=O)_2N(Ri)H$, $-S(=O)_2NH_2$, $- N(Ri)S(=O)_2Ri$, $-N(H)S(=O)_2Ri$, $-N(Ri)S(=O)_2H$ or $-N(H)S(=O)_2H$), an alkyl, an alkenyl, an alkynyl, a cyclohydrocarbyl (e.g., cycloalkyl, cycloalkenyl or cycloalkynyl), a heterocyclohydrocarbyl (e.g., heterocycloalkyl containing one or more heteroatoms selected from the group consisting of S, N and O, or heterocycloalkenyl containing one or more heteroatoms selected from the group consisting of S, N and O), an aryl (e.g., phenyl, or a fused ring group), a heteroaryl (e.g., 8- to 10-membered bicyclic heteroaryl containing 1 to 4 heteroatoms independently selected from the group consisting of nitrogen, oxygen and sulfur), -C(=O)SRi, $- C(=N-CN)N(Ri)_2$, $-C(=NO-CH_3)N(Ri)_2$, $-C(=N-SO_2-NH_2)N(Ri)_2$, $-C(=CH-NO_2)N(Ri)_2$, $-OC(=O)N(Ri)_2$, $-CHN(Ri)N(Ri)_2$, -C(=O)N(Ri)ORi, $- N(Ri)_2C(=O)ORi$, $-OP(=O)(ORi)_2$, $-P(=O)(ORi)_2$, -N(ORi)C(=O)Ri, $- N(ORi)S(=O)_2Ri$, -N(ORi)C(=O)ORi, $-N(ORi)C(=O)N(Ri)_2$, $- N(ORi)C(=S)N(Ri)_2$, $-N(ORi)C(NRi)N(Ri)_2$ and $-N(ORi)C(CHRi)N(Ri)_2$. In any of the foregoing, Ri is a hydrogen, or alkyl, alkenyl, alkynyl, heteroalkyl, heteroalkenyl, or heteroalkynyl as defined herein. In some embodiments, Ri is a hydrogen, or $C_1$-$C_{12}$ alkyl, $C_1$-$C_{12}$ alkenyl, $C_1$-$C_{12}$ alkynyl, $C_1$-$C_{12}$ heteroalkyl, $C_1$-$C_{12}$ heteroalkenyl, or $C_1$-$C_{12}$ heteroalkynyl as defined herein. When an atom or group is substituted with multiple substituents, the multiple substituents may be the same or different. In some embodiments, the substituent itself may be further substituted with, e.g., one or more substituents as defined herein. For example, the $C_1$-$C_6$ alkyl as a substituent may be further substituted with one or more substituents as described herein.

Unless specifically stated otherwise, the alkyl, cycloalkyl, alkenyl, alkynyl, alkylene, alkenylene, alkynylene, alkoxy, alkylcarbonyloxy, alkoxycarbonyl, alkylcarbonylamino, and alkylaminocarbonyl groups as described herein may be optionally substituted.

The "pharmaceutically acceptable salt" of the present disclosure has been discussed in Berge, et al., "Pharmaceutically acceptable salts", J. Pharm. Sci., 66, 1-19 (1977), and is obvious to pharmaceutical chemists. The salt is substantially non-toxic and can provide the desired pharmacokinetic properties, palatability, absorption, distribution, metabolism or excretion, etc.

Examples of the "pharmaceutically acceptable salt" of the present disclosure include acid addition salts and base addition salts of the compounds of the present disclosure, which retain the biological effectiveness and properties of the compounds of the present disclosure and are generally not biologically or otherwise undesirable. In many cases, the compounds of the present disclosure are capable of forming acid salts and/or basic salts due to the presence of amino and/or carboxyl groups or groups similar thereto.

Pharmaceutically acceptable acid addition salts may be formed by use of inorganic and/or organic acids and the compounds of the present disclosure, the inorganic acids may be, for example, but not limited to, hydrochloric acid, hydrobromic acid, sulfuric acid, nitric acid, phosphoric acid and the like; the organic acids may be, for example, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid and undecylenic acid, etc.

Pharmaceutically acceptable basic addition salts can be formed by use of inorganic and/or organic bases and the compounds of the present disclosure, and salts derived from inorganic bases include, but are not limited to, sodium salts, potassium salts, lithium salts, ammonium salts, calcium salts, magnesium salts, iron salts, zinc salts, copper salts, manganese salts and aluminum salts, etc. Preferred inorganic salts are ammonium salts, sodium salts, potassium salts, calcium salts and magnesium salts. Salts derived from organic bases include, but are not limited to, salts of the following primary amines, secondary amines, tertiary amines, substituted amines (including naturally occurring substituted amines), cyclic amines and basic ion exchange resins, such as ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purine, piperazine, piperidine, N-ethylpiperidine, and polyamine resins, etc. Particularly preferred organic bases are isopropylamine, diethylamine, ethanolamine, trimethylamine, dicyclohexylamine, choline, and caffeine.

The pharmaceutically acceptable salts of the present disclosure may be synthesized by common chemical methods.

Generally, a salt may be prepared by reacting a free base or acid with an equal chemical equivalent or excess of an acid (inorganic or organic) or base in a suitable solvent or solvent combination.

In this disclosure, the term "meta" refers to a position on a cycloalkyl structural unit that is one carbon atom away from the position of the amine substituent of cycloalkyl structural unit.

In this disclosure, the term "compound" encompasses all compounds that are isotopically labeled by replacing one or more atoms with atoms having a different atomic mass or mass number. "Isotopes" refers to atoms having the same atomic number but with different mass numbers due to the different number of neutrons in the nucleus. For example, isotopes of hydrogen include tritium and deuterium.

The compound of the present disclosure or pharmaceutically acceptable salts thereof may contain one or more asymmetric centers, and thus may generate enantiomers, diastereomers and other stereoisomeric forms, for example, an amino acid may be defined as (R)- or (S)-, or as (D)- or (L)-, according to absolute stereochemistry. The present disclosure is **intended** to include all of these possible isomers, as well as racemic and optically pure forms thereof. Optically active (+) and (-), (R)- and (S)-, or (D)- and (L)-isomers may be prepared by using chiral synthons or chiral reagents, or may be resolved by conventional techniques, such as chromatography and fractional crystallization. Conventional techniques for preparing/isolating a single enantiomer include chiral synthesis from a suitable optically pure precursor, or resolution of racemates (or racemates of salts or derivatives) by using, for example, chiral high pressure liquid chromatography (HPLC). When the compound described herein contains an olefinic double bond or other geometric asymmetric center, unless otherwise specified, it is intended that the compound includes E- and Z-type geometric isomers. Likewise, all tautomeric forms are intended to be included.

As used herein, the term "stereoisomer" refers to a compound composed of the same atoms bonded by the same bonds, but with different three-dimensional structures, which are not interconvertible. "Optical isomer" refers to two or more stereoisomers that exhibit different optical properties due to differences in configuration. "Enantiomers" are a pair of

stereoisomers that are non-superimposable mirror images of each other. A mixture of a pair of enantiomers in any ratio can be called a "racemic" mixture. "Diastereomers" are stereoisomers that have at least two asymmetric atoms but are not mirror images of each other.

"Stereoisomer" may also include E- and Z-isomers or mixtures thereof, as well as cis- and trans-isomers or mixtures thereof. In certain embodiments, the compound described herein is isolated as an E- or Z- isomer. In other embodiments, the compound described herein is a mixture of E- and Z-isomers.

"Tautomers" refers to isomeric forms of a compound that are in equilibrium with each other. The concentration of the isomeric forms will depend on the environment in which the compound is found, and may differ depending on, for example, whether the compound is a solid or in an organic or aqueous solution.

In this disclosure, the term "lipid nanoparticle" refers to a nano-sized substance (lipid nanoparticle) prepared by introducing the amino lipid compound to an aqueous solution, and the particle is particularly a lipid bilayer vesicle (liposome), a multilayer vesicle or a micelle. In a preferred embodiment, the lipid nanoparticle is a liposome containing the amino lipid compound of the present disclosure.

In this disclosure, the term "liposome" refers to a microvesicle composed of a bilayer of lipid amphiphilic molecules enclosing an aqueous compartment. The formation of liposome is not a spontaneous process. Lipid vesicles are first formed when lipids are placed in water, thus forming a bilayer or a series of bilayers, each separated by water molecules. Liposomes can be formed by ultrasonic treatment of lipid vesicles in water.

In this disclosure, the term "lipid bilayer" refers to a film formed by two layers of lipid molecules.

In this disclosure, the term "micelle" refers to an aggregate of surfactant molecules dispersed in a liquid colloid. Typical micelles in an aqueous solution form aggregates with the hydrophilic head regions upon contact with water, and chelate with the hydrophobic single tail region at the center of the micelles.

In this disclosure, the term "cell" has the meaning known in the art, and includes cultured individual cells, tissues, organs, insect cells, avian cells, fish cells, amphibian cells, mammalian cells, primary cells, continuous cell lines, stem cells and/or genetically engineered cells (such as recombinant cells expressing heterologous polypeptides or proteins). Recombinant cells include, for example, cells expressing heterologous polypeptides or proteins (such as growth factors or blood factors).

In a preferred embodiment, the lipid nanoparticle or liposome of the present disclosure further contains a helper lipid. In a preferred embodiment, the helper lipid is a non-cationic lipid. In a more preferred embodiment, the helper lipid is a non-cationic phospholipid. Within the scope of the present disclosure, the non-cationic lipid can contain a cationic functional group (e.g., an ammonium group), but should contain an anionic functional group to at least neutralize the molecule. The total of all functional groups in the lipid molecule should be non-cationic. Liposomes composed of a mixture of cationic amino lipids and non-cationic (neutral) phospholipids are most effective for delivering nucleic acids to cells. In an even more preferred embodiment, the non-cationic lipid is DOPE or DSPC.

In a further preferred embodiment, the lipid nanoparticle or liposome of the present disclosure further comprises a sterol. Sterols, such as cholesterol, are natural components of cell membranes, and can be used to stabilize the particles and help integration with the cell membrane.

In another embodiment, the lipid nanoparticle or liposome of the present disclosure further contains a bioactive agent. Within the scope of the present disclosure, bioactive agents are substances that have biological effects when introduced into cells or hosts, for example, by stimulating immune responses or inflammatory responses, by exerting enzymatic activity or by complementary mutations. The bioactive agent is particularly a nucleic acid, a peptide, a protein, an antibody, or a small molecule. The term "lipid nanoparticle drug" is applicable when liposomes are used to encapsulate a drug into lipid bilayers or into the inner aqueous space of the liposomes.

In a preferred embodiment, the bioactive agent is a nucleic acid. In another preferred embodiment, the bioactive agent is a member selected from the group consisting of an anti-tumor agent, an antibiotic, an immunomodulator, an anti-inflammatory agent, an agent acting on the central nervous system, a polypeptide and a polypeptoid.

In another embodiment, the lipid nanoparticle or liposome further contains at least one polyethylene glycol (PEG)-lipid. The PEG lipid contributes to protecting the particles and their contents from degradation in vitro or in vivo. In addition, PEG forms a protective layer on the liposome surface and increases the circulation time in vivo. It can be used in liposome drug delivery (PEG-liposome). Preferably, the polyethylene glycol lipid is PEG2000-DMG.

Lipid nanoparticle or liposome containing a bioactive agent can be used to deliver any of a variety of therapeutic agents into cells. The present disclosure includes the use of the lipid nanoparticle (particularly the liposome) as described above for delivering a bioactive agent into a cell.

Preferably, the bioactive agent is a nucleic acid, including but not limited to, RNA, DNA and antisense oligonucleotides; wherein the RNA includes but is not limited to messenger RNA (mRNA), ribosomal RNA (rRNA), microRNA (miRNA), transfer RNA (tRNA), small interfering RNA (siRNA) and small nuclear RNA (snRNA); the DNA includes but is not limited to plasmids. The bioactive agent may also be an anti-tumor agent, an antibiotic, an immunomodulator, an anti-inflammatory agent, an agent acting on the central nervous system, an antigen or a fragment thereof, a protein, a peptide, a polypeptoid, a vaccine, a small molecule, or a mixture thereof. As shown above, the lipid

nanoparticle or liposome containing the amino lipid compound defined in the present disclosure or a pharmaceutically acceptable salt or stereoisomer thereof are suitable for delivering the bioactive agent to cells.

Specific characteristics of liposomes imparted by various amino lipid compounds synthesized by the general synthesis method can be screened for specific applications. The characteristics may be, e.g., transfection efficiency, cytotoxicity, adhesion of the agent to be delivered into cells, stability of liposomes, size of liposomes, etc.

The lipid nanoparticle or liposome of the present disclosure can be used to transfect multicellular tissues or organs, which provides patients with the possibility for new therapeutic treatment.

According to the present disclosure, the patient can be any mammal, preferably selected from the group consisting of human, mice, rats, pigs, cats, dogs, horses, goats, cows and monkeys and/or others. Most preferably, the patient is human.

A preferred embodiment of the present disclosure relates to a use of the lipid nanoparticle or liposome containing the amino lipid compound of the present disclosure or a pharmaceutically acceptable salt or stereoisomer thereof as a medicament.

Particularly, the lipid particle or liposome can be administered to a patient for gene therapy, genetic vaccination, antisense therapy or RNA interfering therapy. Specific applications include but are not limited to:

(1) The lipid nanoparticle of the present disclosure can deliver a nucleic acid for gene therapy. Exogenous genes may be introduced into target cells through the amino lipid of the present disclosure to correct or remedy a disease caused by defective and abnormal genes, so as to achieve the therapeutic purpose. Among them, also included is technical application in transgenics etc., that is, inserting an exogenous gene into appropriate recipient cells of a patient by gene transfer technology, so that the products produced by the exogenous gene can treat a certain disease, such as common lung cancer, gastric cancer, liver cancer, esophageal cancer, colon cancer, pancreatic cancer, brain cancer, lymphatic cancer, leukemia, prostate cancer, etc. Gene-edited nucleic acid substances can also be introduced for treatment of various genetic diseases, such as hemophilia, thalassemia, Gaucher's disease, etc.

(2) The lipid nanoparticle of the present disclosure can be used in vaccination. The lipid nanoparticle or liposome of the present disclosure can be used for delivering antigens or nucleic acids encoding antigens. The lipid nanoparticle of the present disclosure can also be used for the initiation of immune responses against various antigens which are used for treating and/or preventing various disorders such as cancer, allergy, toxicity and pathogens (for example, viruses, bacteria, fungi and other pathogenic organisms) infection.

In another preferred embodiment, the lipid nanoparticle of the present disclosure can be used to manufacture a medicament for nucleic acid transfer, and preferably, the nucleic acid is RNA, DNA or antisense oligonucleotide; preferably, the RNA is selected from the group consisting of messenger RNA (mRNA), ribosomal RNA (rRNA), microRNA (miRNA), transfer RNA (tRNA), small interfering RNA (siRNA) and small nuclear RNA (snRNA); and preferably, the DNA is a plasmid.

EXAMPLES

To make the purposes, technical solutions and advantages of the embodiments of the present disclosure clearer, the technical solutions in the embodiments of the present disclosure will be clearly and completely described below with reference to specific examples. Obviously, the described examples are part of the embodiments of the present disclosure. Based on the examples of the present disclosure, all other examples obtained by those of ordinary skill in the art without inventive labor are within the scope of the present disclosure. The experimental methods for which specific conditions are not specified in the following examples are selected according to conventional methods and conditions, or according to the product instructions.

The structures of all compounds of the present disclosure were identified by nuclear magnetic resonance ($^1H$ NMR) and/or mass spectrometry (MS).

$^1H$ NMR chemical shift ($\delta$) was recorded in PPM (parts per million). NMR was performed on a Bruker AVANCE III-400MHz spectrometer. Suitable solvents were selected from deuterated chloroform ($CDCl_3$), deuterated methanol ($CD_3OD$), deuterated dimethyl sulfoxide (DMSO-d6), etc., and tetramethylsilane was used as an internal standard (TMS).

Low-resolution mass spectra (MS) were measured by an Agilent 1260 Infinity II-G6125C mass spectrometer.

The measurement method of specific rotation in the present disclosure is as follows. A sample was taken, precisely weighed, added with absolute ethanol to be dissolved, and quantitatively diluted into a solution containing about 10 mg per 1 ml, which was measured according to the optical rotation determination method in General testing methods 0621 in Volume IV of the "Pharmacopoeia of the People's Republic of China 2020".

The known starting materials of the present disclosure may be synthesized by using or according to methods known in the art, or may be commercially purchased.

The eluent system of column chromatography used for purifying compounds includes A: dichloromethane and

methanol system (20:1 to 5:1); B: n-hexane and ethyl acetate system (10:1 to 2:1). The volume ratio of the solvents was adjusted according to the different polarity of the compound.

Unless otherwise specified in the examples, the reaction temperature is room temperature, which ranges from 15°C to 30°C.

The HPLC analysis method disclosed in the present disclosure: HPLC-CAD method.

Table 1: Conditions of the HPLC analysis method of the present disclosure.

| | |
|---|---|
| Instrument name | Agilent 1260 |
| Detector | DAD |
| Column | ACE SuperC18, 100*4.6mm, 3um |
| Detection wavelength | 210 nm |
| Flow rate | 0.5 mL/min |
| Injection volume | 3 μl |
| Column temperature | 30 °C |
| Run time | 42 min |
| Isocratic elution | 0.1% ammonium acetate in methanol |
| Sample pretreatment | About 10 mg of the sample was weighed, dissolved in 1 mL of acetonitrile, added with 1 drop of benzoyl chloride as the derivatization reagent and then with 1 drop of triethylamine for derivatization for 15 minutes, and finally quenched with 2 drops of methanol. |

Example 1: Synthesis of Compound 1

Step 1): Synthesis of 1-octylnonyl 8-bromooctanoate

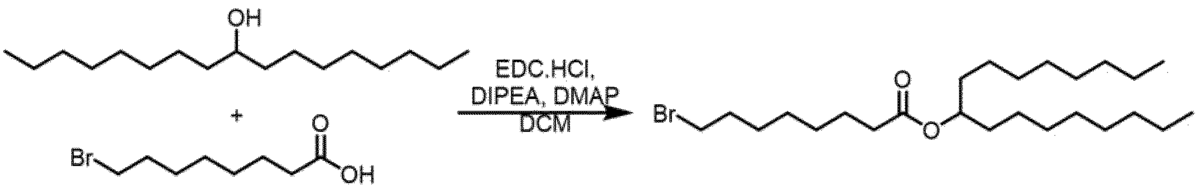


To a 250 mL flask, 8-bromooctanoic acid (22.3 g, 100 mmol), 9-heptadecanol (25.6 g, 100 mmol) and dichloromethane (100 mL) were added in sequence. The mixture was stirred to be dissolved, and then 1-(3-dimethylaminopropyl)-3-ethylcarbodiimide hydrochloride (23.0 g, 120 mmol), 4-dimethylaminopyridine (0.61 g, 5 mmol) and N,N-diisopropylethylamine (25.8 g, 200 mmol) were added. The resulting mixture was reacted at room temperature for 2h, washed with water for three times, dried over anhydrous sodium sulfate, concentrated, and purified with a flash column chromatography system (n-hexane: ethyl acetate=10: 1 to 2:1) to give 1-octylnonyl 8-bromooctanoate (41.5g, 90%).

Step 2): Synthesis of nonyl 8-((3-hydroxycyclobutyl)amino)octanoate (Intermediate II-1)

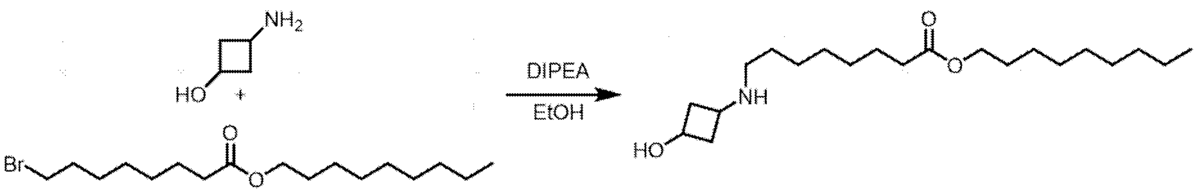


Intermediate II-1

To a 100 mL flask, nonyl 8-bromooctanoate (3.50 g, 10 mmol), 3-aminocyclobutanol (8.7 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((3-hydroxycyclobutyl)amino) octanoate (2.38 g, 67%).

$^1H$ NMR (600 MHz, $CDCl_3$) δ:4.66-4.55 (m, 0.5H), 4.14-4.06 (m, 2H), 3.61-3.51 (m, 0.5H), 3.01 (m, 0.5H), 2.82-2.71 (m, 2H), 2.71-2.63 (m, 2H), 2.58 (m, 0.5H), 2.31 (t, 2H), 2.27-2.01 (m, 2H), 1.72-1.53 (m, 6H), 1.44-1.21 (m, 18H), 0.91 (t, 3H).

LCMS: 356.3 $[M+H]^+$.

Step 3): Synthesis of Compound 1

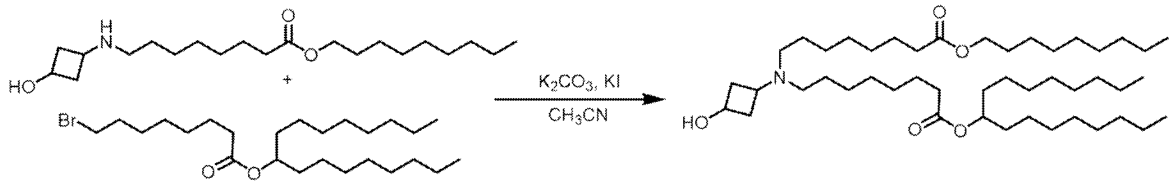


Compound 1

To a 100 mL flask, nonyl 8-((3-hydroxycyclobutyl)amino)octanoate (355 mg, 1 mmol), 1-octylnonyl 8-bromooctanoate (554 mg, 1.2 mmol) and acetonitrile (20 mL) were added in sequence. The mixture was stirred to be dissolved, and then potassium carbonate (276 mg, 2 mmol) and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Compound 1 (501 mg, 68%).

$^1H$ NMR (600 MHz, $CDCl_3$) δ: 4.94-4.78 (m, 1H), 4.42-4.37(m, 0.5H), 4.05 (t, 2H), 3.99 (m, 0.5H), 3.61-3.45 (m, 0.5H), 2.75-2.65 (s, 0.5H), 2. 60-2.52 (m, 2H), 2.50-2.45(m, 3H), 2.40(m,1H), 2.28 (m, 4H), 1.99 (m, 2H), 1.67-1.57 (m, 6H), 1.57-1.45 (m, 4H), 1.43-1.38(m,4H), (m, 49H), 0.89-0.83 (m, 9H).

LCMS: 737.2$[M+H]^+$.

Example 2: Synthesis of Compound 2

Step 1): Synthesis of undecyl 6-((3-hydroxycyclobutyl)amino)hexanoate (Intermediate II-2)

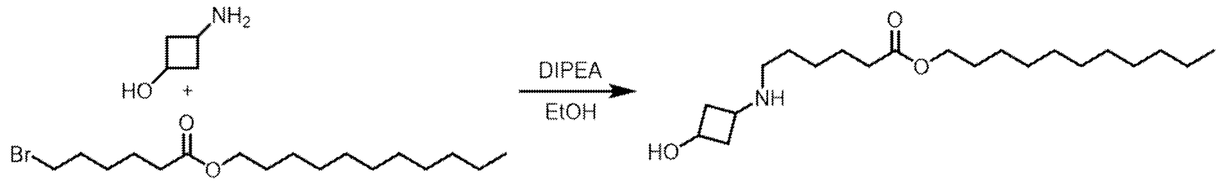


Intermediate II-2

To a 100 mL flask, undecyl 8-bromohexanoate (3.50 g, 10 mmol), 3-aminocyclobutanol (8.7 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give undecyl 6-((3-hydroxycyclobutyl) amino)hexanoate (2.38 g, 67%).

$^1H$ NMR (600 MHz, $CDCl_3$) δ:4.66-4.57 (m, 0.5H), 4.06-4.04 (t, 2H), 3.62-3.54 (m, 0.5H), 3.13-2.96 (m, 0.5H), 2.82-2.73 (m, 2H), 2.71-2.66 (m, 2H), 2.66-2.59 (m, 0.5H), 2.31 (t, 2H), 2.28-2.10 (m, 2H), 1.73-1.55 (m, 6H), 1.43-1.21 (m,

18H), 0.88 (t, 3H).

LCMS: 356.3 [M+H]+.

Step 2): Synthesis of Compound 2

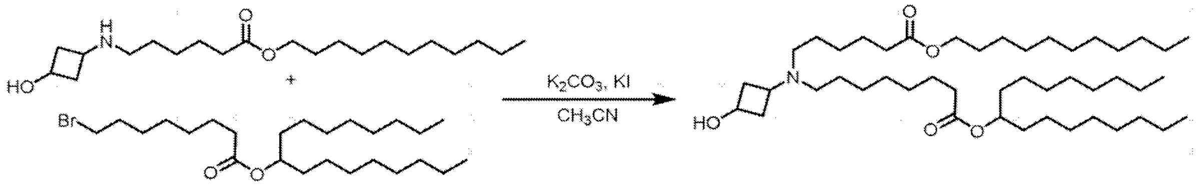


Compound 2

Compound 2 was synthesized in a similar manner to the step 3) in Example 1, except that undecyl 6-((3-hydroxycyclobutyl)amino)hexanoate (Intermediate II-2) is used instead of the intermediate nonyl 8-((3-hydroxycyclobutyl)amino)octanoate.

$^1$H NMR (600 MHz, $CDCl_3$) δ:4.95-4.77 (m, 1H), 4.42 (t, 0.5H), 4.08 (t, 2H), 4.02-3.94 (m, 0.5H), 3.51-3.42 (m, 0.5H), 2.72-2.62 (m, 0.5H), 2.56 (m, 2H), 2.50-2.44 (m, 3H), 2.42 (s, 1H), 2.38-2.29 (m, 4H), 1.94 (s, 2H), 1.71-1.58 (m, 6H), 1.53 (m, 4H), 1.44 (m, 4H), 1.40-1.21 (m, 49H), 0.90 (m, 9H).

LCMS:737.2 [M+H]+.

Example 3: Synthesis of Compound 6

Step 1): Synthesis of nonyl 8-((3-hydroxycyclohexyl)amino)octanoate (Intermediate II-6)

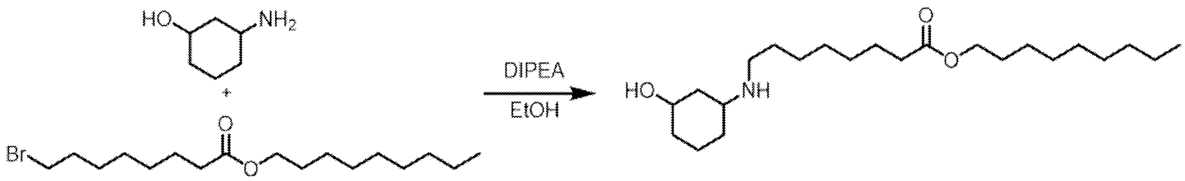


Intermediate II-6

To a 100 mL flask, nonyl 8-bromooctanoate (3.50 g, 10 mmol), 3-aminocyclohexanol (11.5 g, 100 mmol) and ethanol (30 mL) were added in sequence. The mixture was stirred to be dissolved, and then N,N-diisopropylethylamine (2.58 g, 20 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((3-hydroxycyclohexyl)amino)octanoate (2.34 g, 61%).

$^1$H NMR (600 MHz, $CDCl_3$) δ 4.20-4.13 (m, 0.5H), 4.05 (t, 2H), 3.83 (m, 0.5H), 3.09 (m, 0.5H), 2.87 (m, 0.5H), 2.76-2.59 (m, 2H), 2.29 (t, 2H), 2.00 (m, 0.5H), 1.93-1.78 (m, 1.5H), 1.78-1.65 (m, 2H), 1.65-1.46 (m, 8H), 1.40-1.18 (m, 20H), 0.88 (t, 3H).

LCMS: 384.3[M+H]+.

Step 2): Synthesis of Compound 6

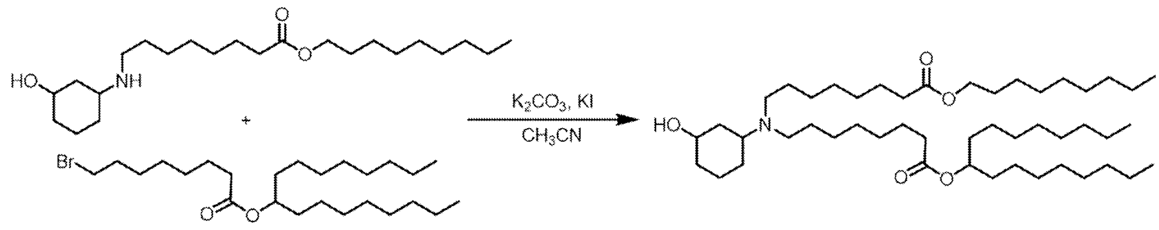


Compound 6

To a 100 mL flask, nonyl 8-((3-hydroxycyclohexyl)amino)octanoate (383 mg, 1 mmol), 1-octylnonyl 8-bromooctanoate (554 mg, 1.2 mmol) and acetonitrile (20 mL) were added in sequence. The mixture was stirred to be dissolved, and then potassium carbonate (276 mg, 2 mmol), and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Compound 6 (596 mg, 78%).

$^1H$ NMR (600 MHz, $CDCl_3$) δ 4.95-4.81 (m, 1H), 4.30-4.12 (m, 0.5H), 4.07 (t, 2H), 3.72-3.61 (m, 0.5H), 2.97 (m, 0.5H), 2.64-2.52 (m, 0.5H), 2.48-2.37 (m, 4H), 2.30 (q, 4H), 1.93-1.81 (m, 2H), 1.72-1.57 (m, 8H), 1.51 (t, 4H), 1.43-1.18 (m, 56H), 0.89 (m, 9H).

LCMS:765.3$[M+H]^+$.

Example 4: Synthesis of Compound 7

Step 1): Synthesis of undecyl 6-((3-hydroxycyclohexyl)amino)hexanoate (Intermediate II-7)

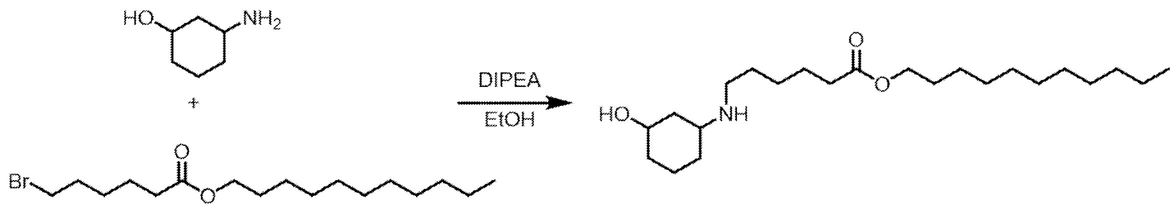


Intermediate II-7

To a 100 mL flask, undecyl 6-bromohexanoate (3.50 g, 10 mmol), 3-aminocyclohexanol (11.5 g, 100 mmol), and ethanol (30 mL) were added in sequence. The mixture was stirred to be dissolved, and then N,N-diisopropylethylamine (2.58 g, 20 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give undecyl 6-((3-hydroxycyclohexyl)amino)hexanoate (2.53 g, 66%).

$^1H$ NMR (600 MHz, $CDCl_3$) δ 4.20 (m, 0.5H), 4.05 (t, 2H), 3.93 (m, 0.5H), 3.21 (m, 0.5H), 3.03 (m, 0.5H), 2.84-2.68 (m, 2H), 2.35-2.25 (m, 2H), 2.12-2.06 (m, 0.5H), 1.91 (m, 1.5H), 1.94-1.82 (m, 2H), 1.77-1.56 (m, 8H), 1.43-1.19 (m, 20H), 0.88 (t, 3H).

LCMS:384.4$[M+H]^+$.

Step 2): Synthesis of Compound 7

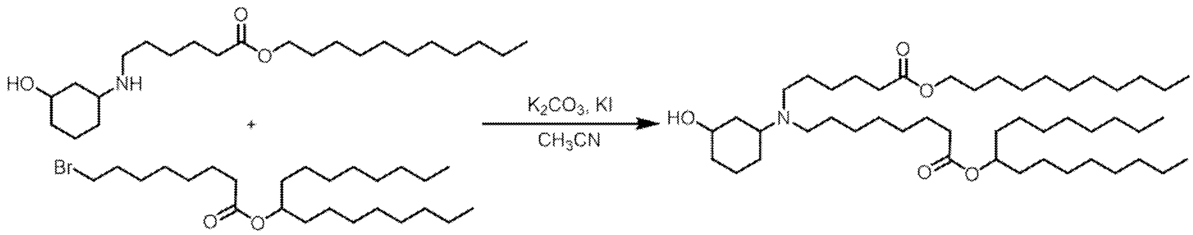


Compound 7

To a 100 mL flask, undecyl 6-((3-hydroxycyclohexyl)amino)hexanoate (383 mg, 1 mmol), 1-octylnonyl 8-bromooctanoate (554 mg, 1.2 mmol) and acetonitrile (20 mL) were added in sequence. The mixture was stirred to be dissolved, and then potassium carbonate (276 mg, 2 mmol), and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Compound 7 (572 mg, 75%).

$^1$H NMR (600 MHz, $CDCl_3$) δ 4.93-4.81 (m, 1H), 4.24 (m, 0.5H), 4.05 (t, 2H), 3.68-3.61 (m, 0.5H), 2.98 (m, 0.5H), 2.56 (m, 0.5H), 2.50-2.36 (m, 4H), 2.35-2.22 (m, 4H), 1.97-1.73 (m, 3H), 1.69-1.55 (m, 7H), 1.48 (m, 4H), 1.46-1.36 (m, 4H), 1.36-1.18 (m, 52H), 0.88 (m, 9H).

LCMS:765.0$[M+H]^+$.

Example 5: Synthesis of Compound 8

Step 1) Synthesis of pentadecan-7-yl 6-oxohexanoate

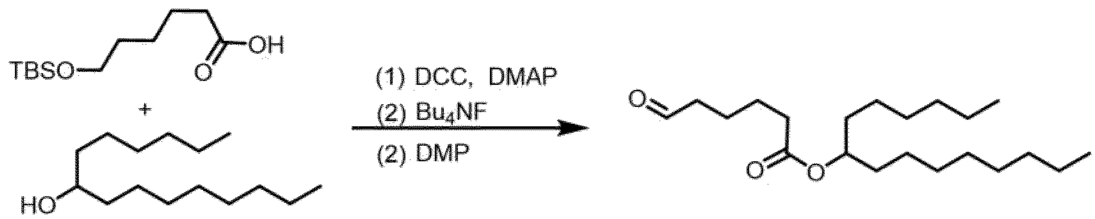


To a 250 mL flask, 6-((tert-butyldimethylsilyl)oxy)hexanoic acid (2.46 g, 10 mmol), pentadecan-7-ol (2.28 g, 10 mol) and dichloromethane (100 mL) were added in sequence. The mixture was stirred to be dissolved, and then dicyclohexylcarbodiimide (2.47 g, 12 mmol) and 4-dimethylaminopyridine (0.06 g, 0.5 mmol) were added. The resulting mixture was reacted at room temperature for 2h, washed with water for three times, dried over anhydrous sodium sulfate and concentrated to dryness. The resulting residue was added with tetrahydrofuran (50 mL) and tetrabutylammonium fluoride (2.75g, 10.5 mmol), reacted at room temperature for 1h, and concentrated to dryness. The resulting residue was dissolved with dichloromethane (100 mL), washed with water for three times, dried over anhydrous sodium sulfate, added with Dess-Martin periodinane (5.09g, 12 mmol), stirred and reacted at room temperature for 12 h, washed with saturated sodium bicarbonate for three times and then with water once, dried over anhydrous sodium sulfate, and purified with a flash column chromatography system (n-hexane: ethyl acetate=10:1 to 5:1) to give pentadecan-7-yl 6-oxohexanoate (2.76 g, 81%).

Step 2): Synthesis of Compound 8

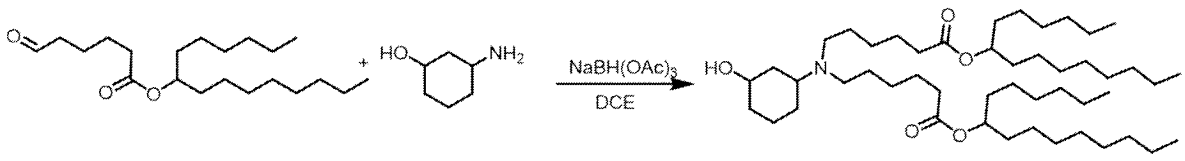


Compound 8

To a 250 mL flask, pentadecan-7-yl 6-oxohexanoate (3.41 g, 10 mmol), dichloroethane (100 mL) and sodium triacetoxyborohydride (3.18g, 15 mmol) were added in sequence, followed by addition of 3-aminocyclohexanol (0.57 g, 5 mmol). The resulting mixture was reacted at room temperature for 24h, washed with water for three times, dried over anhydrous sodium sulfate, concentrated, and purified with a flash column chromatography system (dichloromethane: methanol=20:1 to 5:1) to give Compound 8 (2.83g, 74%).

$^1$H NMR (600 MHz, $CDCl_3$) δ 4.93-4.80 (m, 2H), 4.24 (m, 0.5H), 3.66-3.58 (m, 0.5H), 3.05 (m, 0.5H), 2.54 (m, 0.5H), 2.42 (m, 4H), 2.30 (m, 4H), 1.96-1.74 (m, 3H), 1.72-1.56 (m, 6H), 1.50 (m, 11H), 1.35-1.19 (m, 48H), 0.88 (t, 12H).

LCMS:765.1$[M+H]^+$.

Example 6: Synthesis of Compound 9

Step 1): Synthesis of 6-oxohexyl 2-hexyldecanoate

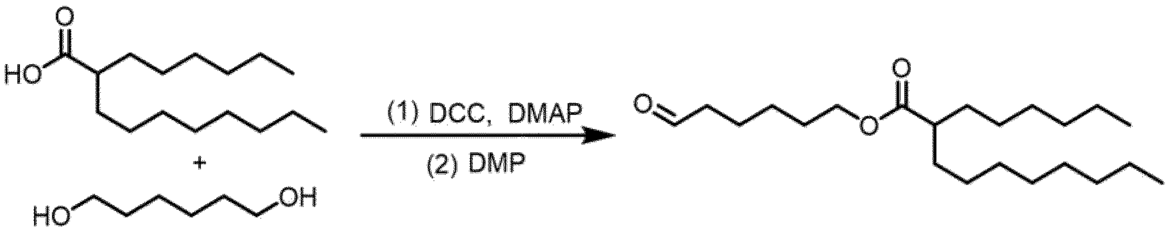


To a 250 mL flask, 2-n-hexyldecanoic acid(25.6 g, 100 mmol), 1,6-hexanediol (59.0 g, 0.5 mol) and dichloromethane (150 mL) were added in sequence. The mixture was stirred to be dissolved, and then dicyclohexylcarbodiimide (20.6 g, 100 mmol) and 4-dimethylaminopyridine (0.61 g, 5 mmol) were added. The resulting mixture was reacted at room temperature for 2h, washed with water for three times, dried over anhydrous sodium sulfate, and purified with a flash column chromatography system (n-hexane: ethyl acetate=5:1 to 1:1) to give 7-hydroxyheptyl2-hexyldecanoate. Dichloromethane (100 mL) and Dess-Martin periodinane (50.9g, 120 mmol) were added in sequence, stirred and reacted at room temperature for 12 h, washed with saturated sodium bicarbonate for three times and then with water once, dried over anhydrous sodium sulfate, and purified with a flash column chromatography system (n-hexane: ethyl acetate=10:1 to 5:1) to give 6-oxohexyl 2-hexyldecanoate (19.1g, 54%).

Step 2): Synthesis of Compound 9

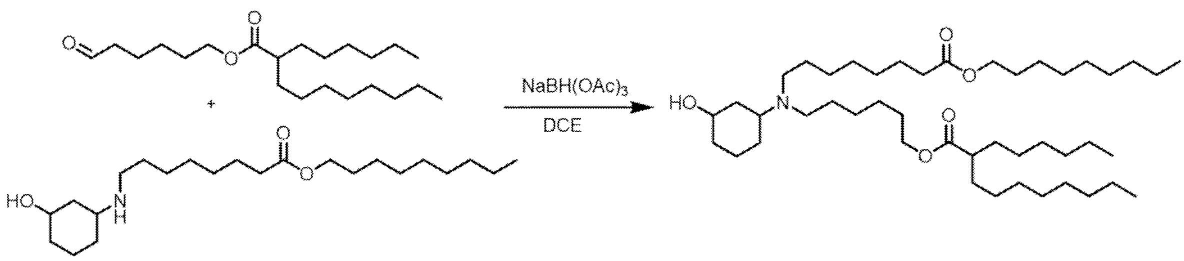


Compound 9

To a 250 mL flask, 6-oxohexyl 2-hexyldecanoate (3.55 g, 10 mmol), dichloroethane (100 mL) and sodium

triacetoxyborohydride (3.18g, 15 mmol) were added in sequence, followed by addition of nonyl 8-((3-hydroxycyclohexyl) amino)octanoate (3.83 g, 10 mmol). The resulting mixture was reacted at room temperature for 24h, washed with water for three times, dried over anhydrous sodium sulfate, concentrated, and purified with a flash column chromatography system (dichloromethane: methanol=20:1 to 5:1) to give Compound 9 (4.98 g, 69%).

$^1H$ NMR (600 MHz, $CDCl_3$) δ 4.26-4.19 (m, 0.5H), 4.12 (m, 2H), 4.09-4.02 (m, 2H), 3.65 (m, 0.5H), 2.98 (m, 0.5H), 2.59 (m, 0.5H), 2.51-2.35 (m, 4H), 2.30 (m, 3H), 1.98-1.89 (m, 1H), 1.89-1.72 (m, 3H), 1.72-1.53 (m, 9H), 1.51-1.34 (m, 10H), 1.34-1.16 (m, 42H), 0.92-0.79 (m, 9H).

LCMS:723.1$[M+H]^+$.

Example 7: Synthesis of Compound 10

Step 1): Synthesis of 8-bromooctyl oleate

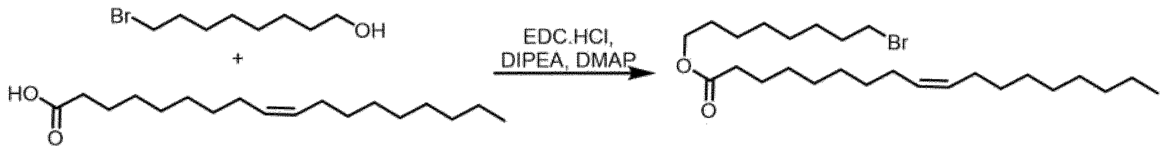


To a 250 mL flask, 8-bromooctanol (20.9 g, 100 mmol), oleic acid (28.5 g, 100 mmol) and dichloromethane (100 mL) were added in sequence. The mixture was stirred to be dissolved, and then 1-(3-dimethylaminopropyl)-3-ethylcarbodiimide hydrochloride (23.0 g, 120 mmol), 4-dimethylaminopyridine (0.61 g, 5 mmol) and N,N-diisopropylethylamine (25.8 g, 200 mmol) were added. The resulting mixture was reacted at room temperature for 2h, washed with water for three times, dried over anhydrous sodium sulfate, concentrated, and purified with a flash column chromatography system (n-hexane: ethyl acetate=10: 1 to 3:1) to give 8-bromooctyl oleate (37.4 g, 79%).

Step 2): Synthesis of 8-((3-hydroxycyclohexyl)amino)octyl oleate (Intermediate II-10)

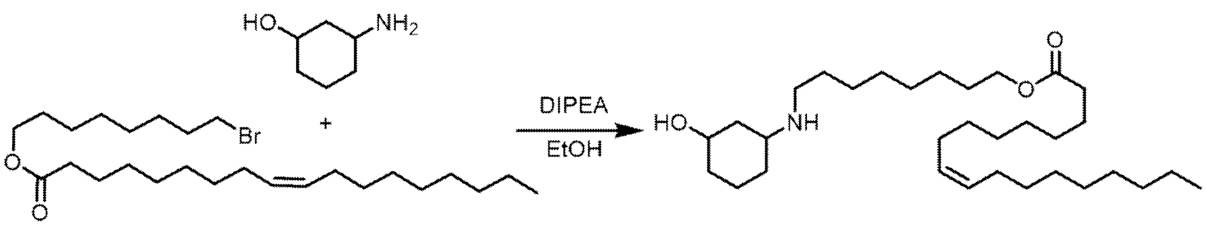


Intermediate II-10

To a 100 mL flask, 8-bromononyl oleate (4.73 g, 10 mmol), 3-aminocyclohexanol (11.5 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give 8-((3-hydroxycyclohexyl)amino)octyl oleate (3.2 g, 63%).

$^1H$ NMR (600 MHz, $CDCl_3$) δ 5.41-5.29 (m, 2H), 4.19 (m, 0.5H), 4.05 (t, 2H), 3.92 (m, 0.5H), 3.20 (m, 0.5H), 3.00 (m, 0.5H), 2.81-2.67 (m, 2H), 2.29 (t, 2H), 2.02 (m, 3H), 1.93-1.79 (m, 2H), 1.69-1.56 (m, 11H), 1.36-1.20 (m, 30H), 0.88 (t, 3H).

LCMS:508.7$[M+H]^+$.

Step 3): Synthesis of Compound 10

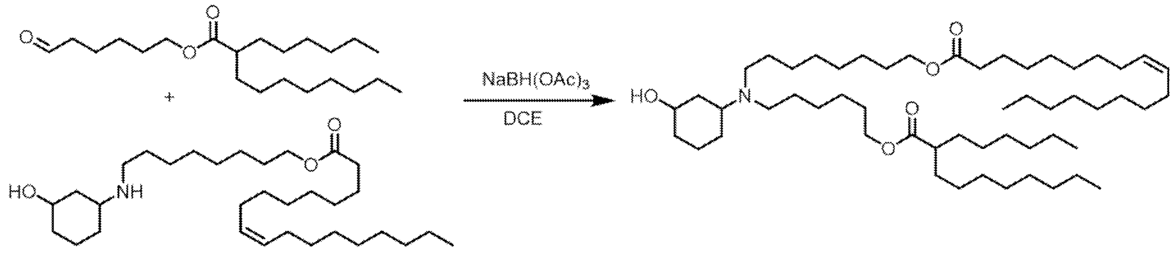


Compound 10

To a 250 mL flask, 6-oxohexyl 2-hexyldecanoate (3.55 g, 10 mmol), dichloroethane (100 mL) and sodium triacetoxyborohydride (3.18g, 15 mmol) were added in sequence, followed by addition of 8-((3-hydroxycyclohexyl)amino) octyl oleate (5.08 g, 10 mmol). The resulting mixture was reacted at room temperature for 24h, washed with water for three times, dried over anhydrous sodium sulfate, concentrated, and purified with a flash column chromatography system (dichloromethane: methanol=20:1 to 5:1) to give Compound 10 (6.26 g, 74%).

$^1$H NMR (600 MHz, $CDCl_3$) δ 5.41-5.31 (m, 2H), 4.24 (m, 0.5H), 4.06 (m, 4H), 3.70-3.60 (m, 0.5H), 3.03-2.92 (m, 0.5H), 2.57 (m, 0.5H), 2.49-2.36 (m, 4H), 2.30 (m, 3H), 2.01 (m, 3H), 1.98-1.90 (m, 2H), 1.85 (m, 1H), 1.82-1.74 (m, 1H), 1.61 (m, 9H), 1.45-1.38 (m, 6H), 1.33-1.20 (m, 56H), 0.88 (m, 9H).

LCMS:847.3$[M+H]^+$.

Example 8: Synthesis of Compound 12

Step 1): Synthesis of (6Z,9Z)-diene-octadecyl 8-((3-hydroxycyclohexyl)amino)octanoate (Intermediate II-12)

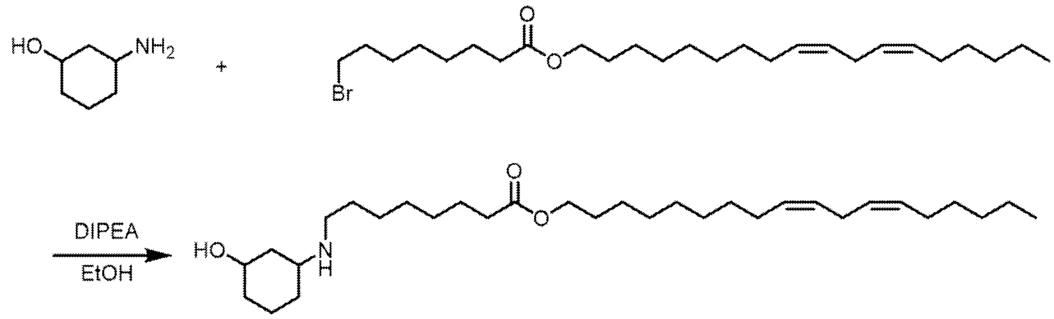


Intermediate II-12

To a 100 mL flask, (6Z,9Z)-diene-octadecyl 8-bromooctanoate (4.71 g, 10 mmol), 3-aminocyclohexanol (11.5 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give (6Z,9Z)-diene-octadecyl 8-((3-hydroxycyclohexyl)amino)octanoate (3.3 g, 66%).

$^1$H NMR (600 MHz, $CDCl_3$) δ 5.43-5.28 (m, 4H), 4.18 (m, 0.5H), 4.07-4.03 (m, 2H), 3.95 (m, 0.5H), 3.22 (m, 0.5H), 3.04 (m, 0.5H), 2.82-2.69 (m, 4H), 2.31-2.23 (m, 2H), 2.09-2.01 (m, 4H), 1.85 (m, 1H), 1.76-1.56 (m, 10H), 1.40-1.26 (m, 23H), 0.92-0.84 (t, 3H).

LCMS: 506.7$[M+H]^+$.

Step 2): Synthesis of Compound 12

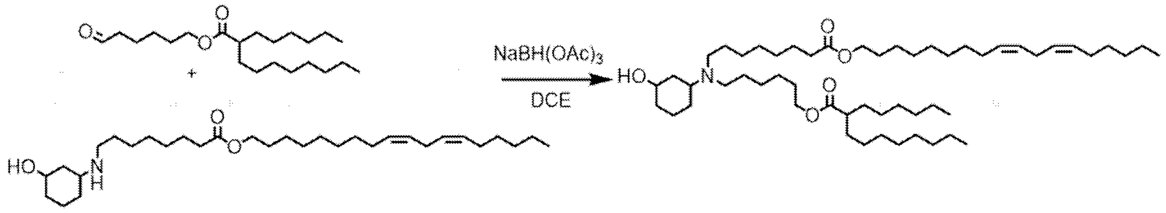


Compound 12

Compound 12 was synthesized in a similar manner to the step 3) in Example 7, except that (6Z,9Z)-diene-octadecyl 8-((3-hydroxycyclohexyl)amino) octanoate was used instead of the Intermediate compound 8-((3-hydroxycyclohexyl)amino)octyl oleate.

$^1$H NMR (600 MHz, $CDCl_3$) δ 5.43-5.28 (m, 4H), 4.24 (m, 0.5H), 4.06 (m, 4H), 3.63 (m, 0.5H), 2.98 (m, 0.5H), 2.77 (t, 2H), 2.55 (m, 0.5H), 2.45 (m, 4H), 2.30 (m, 3H), 2.10-2.01 (m, 4H), 1.88-1.73 (m, 3H), 1.66-1.53 (m, 10H), 1.35-1.16 (m, 55H), 0.88 (m, 9H).

LCMS:845.1[M+H]$^+$.

Example 9: Synthesis of Compound 14

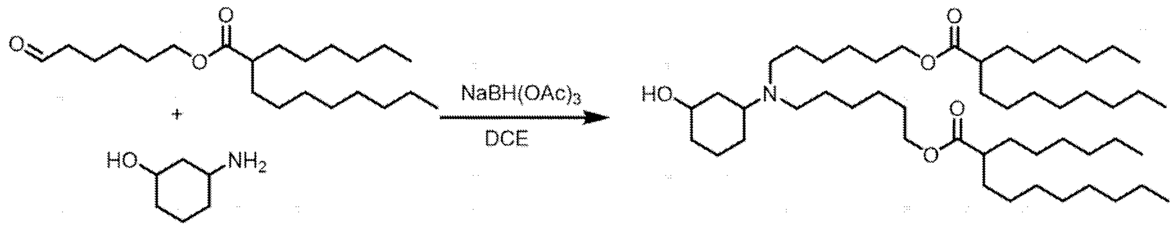


Compound 14

To a 250 mL flask, 6-oxohexyl 2-hexyldecanoate (3.55 g, 10 mmol), dichloroethane (100 mL) and sodium triacetoxyborohydride (3.18g, 15 mmol) were added in sequence, followed by addition of 3-aminocyclohexanol (0.57 g, 5 mmol). The resulting mixture was reacted at room temperature for 24h, washed with water for three times, dried over anhydrous sodium sulfate, concentrated, and purified with a flash column chromatography system (dichloromethane: methanol=20:1 to 5:1) to give Compound 14 (2.81 g, 71%).

$^1$H NMR (600 MHz, $CDCl_3$) δ 4.32-4.20 (m, 0.5H), 4.13-4.03 (m, 4H), 3.71-3.61 (m, 0.5H), 3.02 (m, 0.5H), 2.59 (m, 0.5H), 2.48 (m, 4H), 2.37-2.29 (m, 2H), 1.91-1.75 (m, 2H), 1.74-1.56 (m, 10H), 1.51-1.36 (m, 14H), 1.36-1.21 (m, 46H), 0.90 (m, 12H).

LCMS:793.2[M+H]$^+$.

Example 10: Synthesis of Compound 15

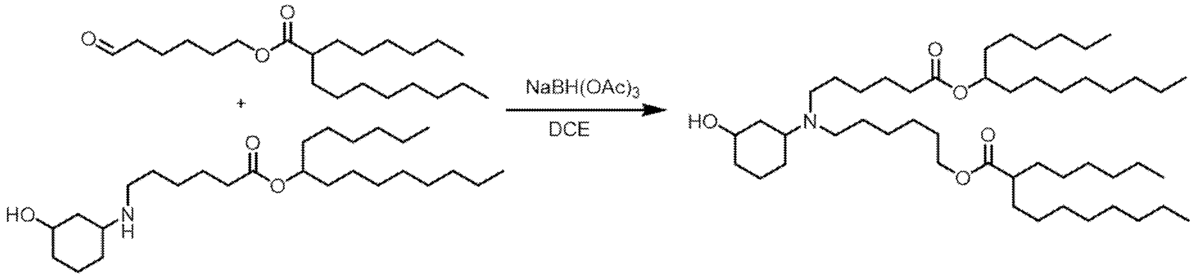


Compound 15

Compound 15 was synthesized in a similar manner to step 3) in Example 7, except that 7-pentadecyl 6-((3-hydroxycyclohexyl)amino)hexanoate was used instead of the Intermediate 8-((3-hydroxycyclohexyl)amino)octyl oleate.

$^1H$ NMR (600 MHz, $CDCl_3$) δ 4.94-4.80 (m, 1H), 4.23 (m, 0.5H), 4.08 (t, 2H), 3.72-3.62 (m, 0.5H), 3.00 (m, 0.5H), 2.55 (m, 0.5H), 2.50-2.37 (m, 4H), 2.35-2.25 (m, 3H), 1.90-1.75 (m, 2H), 1.73-1.57 (m, 9H), 1.51 (m, 4H), 1.44 (m, 7H), 1.39 (m, 2H), 1.36-1.21 (m, 46H), 0.90 (m, 12H).

LCMS:779.2[M+H]+.

Example 11: Synthesis of Compound 16

Step 1): Synthesis of nonyl 8-((3-methoxycyclohexyl)amino)octanoate (Intermediate II-16)

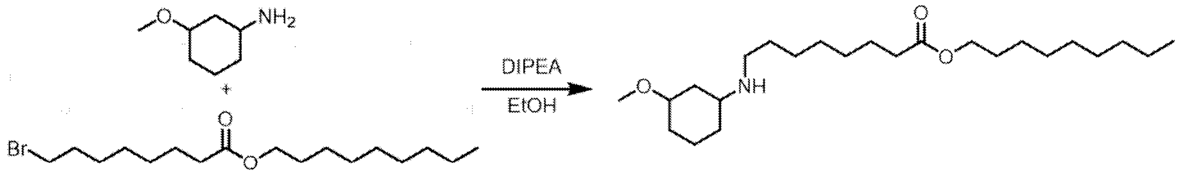


Intermediate II-16

To a 100 mL flask, nonyl 8-bromooctanoate (3.49 g, 10 mmol), 3-methoxycyclohexylamine (12.9 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((3-methoxycyclohexyl)amino)octanoate (2.35 g, 59%).

$^1H$ NMR (600 MHz, $CDCl_3$) δ 4.07 (t, 2H), 3.64 (s, 0.5H), 3.36 (s, 1.5H), 3.35 (m, 0.5H), 3.29 (s, 1.5H), 3.28-3.23 (m, 0.5H), 3.18 (s, 0.5H), 3.00-2.89 (m, 2H), 2.30 (t, 2H), 2.07-2.02 (m, 4H), 1.92-1.84 (m, 2H), 1.69 (m, 3H), 1.67-1.58 (m, 4H), 1.40-1.23 (m, 18H), 0.94-0.82 (m, 3H).

LCMS:398.5[M+H]+.

Step 2): Synthesis of Compound 16

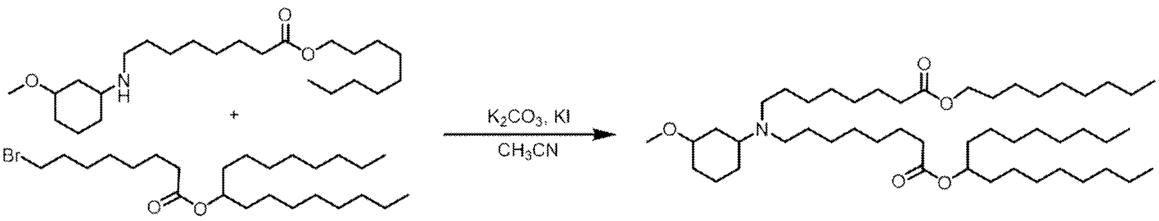


Compound 16

To a 100 mL flask, nonyl 8-((3-methoxycyclohexyl)amino)octanoate (400 mg, 1 mmol), 1-octylnonyl 8-bromooctanoate (554 mg, 1.2 mmol) and acetonitrile (20 mL) were added in sequence. The mixture was stirred to be dissolved, and then potassium carbonate (276 mg, 2 mmol) and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Compound 16 (552 mg, 71%).

$^1H$ NMR (600 MHz, $CDCl_3$) δ 4.93-4.77 (m, 1H), 4.05 (t, 2H), 3.62 (m, 0.5H), 3.36 (s, 1.5H), 3.29 (s, 1.5H), 3.15-3.07 (m, 0.5H), 2.86 (m, 0.5H), 2.55 (t, 0.5H), 2.43 (m, 4H), 2.32-2.24 (m, 4H), 2.07-1.99 (m, 1H), 1.85-1.69 (m, 2H), 1.62 (m, 6H), 1.53-1.48 (m, 4H), 1.45-1.39 (m, 4H), 1.36-1.24 (m, 54H), 0.90-0.85 (m, 9H).

LCMS:779.1[M+H]+.

Example 12: Synthesis of Compound 19

Step 1): Synthesis of nonyl 8-((3-acetoxycyclohexyl)amino)octanoate Intermediate II-19)

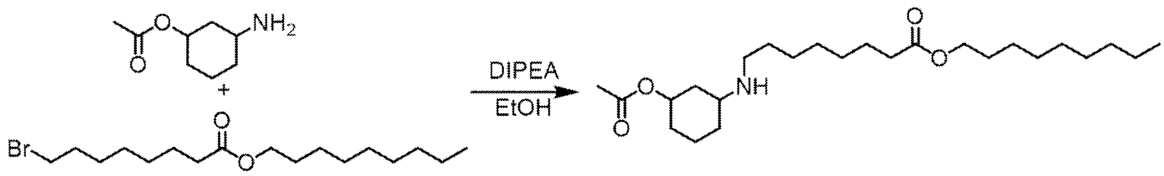


Intermediate II-19

To a 100 mL flask, nonyl 8-bromooctanoate (3.49 g, 10 mmol), 3-acetoxycyclohexylamine (15.7 g, 100 mmol) and ethanol (30 mL)were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((3-acetoxycyclohexyl)amino) octanoate (2.76 g, 65%).

$^1$H NMR (600 MHz, $CDCl_3$) δ 5.23 (m, 0.5H), 4.76-4.67 (m, 0.5H), 4.09 (t, 2H), 3.30 (m, 0.5H), 3.05 (m, 0.5H), 3.01-2.86 (m, 3H), 2.45-2.38 (m, 1H), 2.32 (t, 2H), 2.12 (m, 1H), 2.10 (s, 1.5H), 2.08 (s, 1.5H), 2.05 (m, 1H), 1.97-1.91 (m, 1H), 1.77 (m, 3H), 1.71 (m, 3H), 1.68-1.60 (m, 5H), 1.41-1.22 (m, 16H), 0.92 (t, 3H).

LCMS:426.4$[M+H]^+$.

Step 2): Synthesis of Compound 19

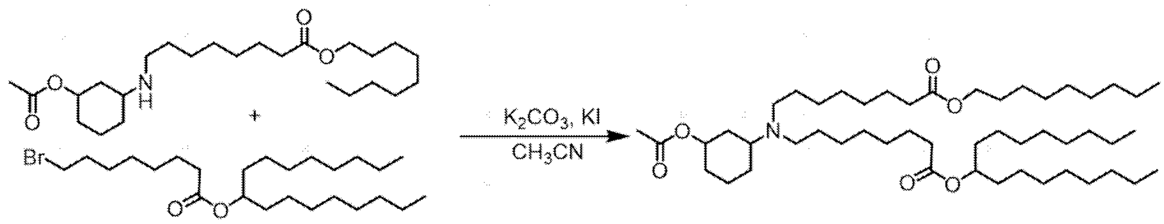


Compound 19

Compound 19 was synthesized in a similar manner to the step 2) in Example 11, except that nonyl 8-((3-acetoxycyclohexyl)amino)octanoate is used instead of the Intermediate nonyl 8-((3-methoxycyclohexyl)amino)octanoate.

$^1$H NMR (600 MHz, $CDCl_3$) δ 5.16 (m, 0.5H), 4.91-4.81 (m, 1H), 4.69 (m, 0.5H), 4.05 (t, 2H), 2.86 (m 0.5H), 2.64-2.50 (m, 0.5H), 2.45-2.32 (m, 4H), 2.32-2.22 (m, 4H), 2.04 (s, 1.5H), 2.03 (s, 1.5H), 1.93 (m, 1H), 1.84-1.75 (m, 1H), 1.74-1.69 (m, 1H), 1.66-1.58 (m, 7H), 1.49 (m, 4H), 1.37 (m, 4H), 1.34-1.21 (m, 52H), 0.90-0.85 (m, 9H).

LCMS:806.7$[M+H]^+$.

Example 13: Synthesis of Compound 20

Step 1): Synthesis of methyl 3-((8-(nonyloxy)-8-oxooctyl)amino)cyclohexane-1-carboxylate (Intermediate II-20)

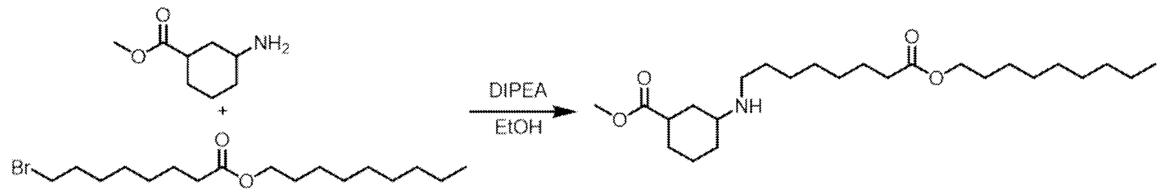


Intermediate II-20

To a 100 mL flask ,nonyl 8-bromooctanoate (3.49 g, 10 mmol), methyl 3-aminocyclohexanecarboxylate (15.7 g, 100 mmol) and ethanol (30 mL) were added in sequence . The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give methyl 3-((8-(nonyloxy)-8-oxooctyl)amino)cyclohexane-1-carboxylate (2.85 g, 67%).

$^1$H NMR (600 MHz, $CDCl_3$) δ 4.05 (t, 2H), 3.70 (m, 2H), 3.67 (s, 3H), 3.00-2.80 (m, 3H), 2.41 (m, 1H), 2.35 (m, 1H), 2.32-2.26 (t, 2H), 2.23-2.15 (m, 1H), 2.01 (m, 1H), 1.94-1.89 (m, 1H), 1.86-1.75 (m, 2H), 1.69-1.57 (m, 3H), 1.40-1.19 (m, 18H), 0.88 (t, 3H).

LCMS:426.4$[M+H]^+$.

Step 2): Synthesis of Compound 20

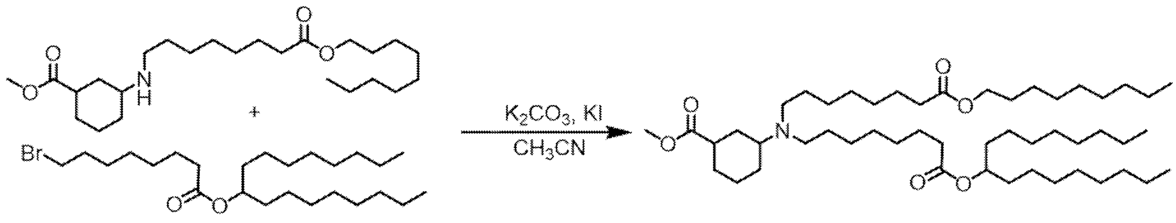


Compound 20

Compound 20 was synthesized in a similar manner to the step 2) in Example 11, except that methyl 3-((8-(nonyloxy)-8-oxooctyl)amino)cyclohexane-1-carboxylate is used instead of the Intermediate nonyl 8-((3-methoxycyclohexyl) amino)octanoate.

$^1$H NMR (600 MHz, $CDCl_3$) δ 4.95-4.78 (m, 1H), 4.05 (t, 2H), 3.67 (s, 3H), 2.55-2.45 (m, 1H), 2.45-2.34 (m, 4H), 2.32-2.24 (m, 5H), 1.99 (m, 1H), 1.94-1.82 (m, 2H), 1.74 (m, 1H), 1.65-1.58 (m, 6H), 1.57-1.45 (m, 4H), 1.43-1.19 (m, 56H), 0.88 (m, 9H).

LCMS:806.8$[M+H]^+$.

Example 14: Synthesis of Compound (I-6-II)

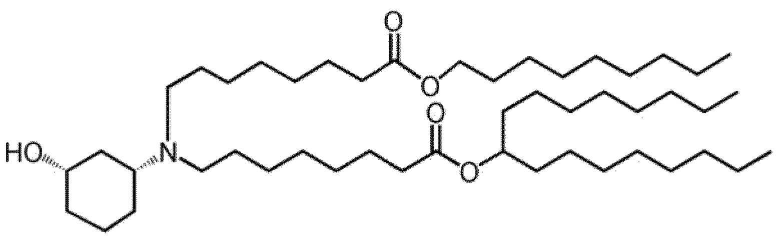

(I-6-II)

To a 250 mL single-necked flask, 9-heptadecyl 8-bromooctanoate (8g, 17.3mmol), (1S,3R)-3-aminocyclohexanol (10g ,76.5mmol) and ethanol (100 mL) were added. The resulting mixture was reacted at 50 °C for 15h, and the raw material 9-heptadecyl-8-bromooctanoate was depleted. After the solvent was removed by rotary evaporation, the

resulting crude was added with EA (200 mL), washed with water (100 mL) twice to thoroughly wash off the (1S,3R)-3-aminocyclohexanol. The EA phase was dried by rotary evaporation to obtain the crude product of 9-heptadecyl-8-(((1R,3S)-3-hydroxycyclohexyl)amino)octanoate (Compound (6-VII-I)). The crude product was purified by column chromatography (dichloromethane: methanol=20:1 to 5:1) to give 6.77g of Compound (6-VII-I), with a purity of 99.2% and a yield of 78.6%.

NMR data:

$^1$H NMR (600 MHz, $CDCl_3$): δ 4.91-4.81 (m, 1H), 3.86-3.82 (m, 1H), 2.84-2.81 (m, 1H), 2.69-2.54 (qt, J =11.2, 7.3 Hz, 2H), 2.29-2.26 (t, J =7.5 Hz, 2H), 1.93-1.86 (m, 1H), 1.77-1.74(d, J =12.0 Hz, 1H), 1.72-1.57 (m, 6H), 1.54-1.45 (m, 6H), 1.37-1.30 (m, 8H), 1.30-1.22 (m, 24H), 0.88 (t, J =7.0 Hz, 6H).

LCMS:496.7 $[M+H]^+$.

The above Compound (6-VII-I) (6g, 12.1mmol) was taken into a 250mL single-necked flask, added with acetonitrile (90ml) and cyclopentyl methyl ether (60ml), followed by addition of potassium carbonate powder (6.7g, 48.4mmol), potassium iodide (2g, 12.1mmol) and nonyl 8-bromooctanoate (5g, 17.5mmol). The resulting mixture was reacted at 90 °C for 24h, and the raw material Compound (6-VII-I) was depleted. The reaction mixture was removed from oil bath and cooled to room temperature, then filtered to remove the solid. The filtrate was dried by rotary evaporation to obtain a crude product, which was finally purified by column chromatography (dichloromethane: methanol=20:1 to 5:1) to give 5.67g of Compound (I-6-II), with a purity of 98.6% and a yield of 61.4%.

NMR data:

$^1$H NMR (600 MHz, $CDCl_3$): δ 4.93-4.84 (m, 1H), 4.09 (t, J =6.8 Hz, 2H), 3.68-3.61 (m, 1H), 2.62-2.54 (m, 1H), 2.53-2.41 (m, 4H), 2.31-2.26 (q, J =7.4 Hz, 4H), 1.97 (m, 1H), 1.91-1.78 (m, 2H), 1.71-1.61 (m, 7H), 1.54 (d, J =6.0 Hz, 4H), 1.43-1.37 (m, 4H), 1.39-1.23 (m, 52H), 0.91 (m, 9H).
Specific rotation: +4.3°

LCMS:765.2 $[M+H]^+$.

Example 15: Synthesis of Compound (I-6-III)

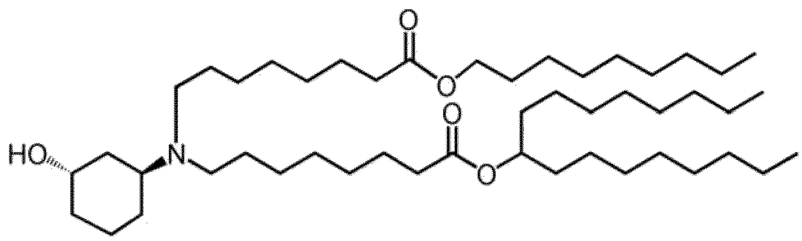

(I-6-III)

To a 100 mL single-necked flask, 9-heptadecyl 8-bromooctanoate (2g, 4.3mmol), (1S,3S)-3-aminocyclohexanol (2.5g, 20.2mmol) and ethanol (20ml) were added. The resulting mixture was reacted at 50 °C for 15h, and the raw material 9-heptadecyl-8-bromooctanoate was depleted. After the solvent was removed by rotary evaporation, the resulting crude was added with EA (50 mL), and washed with water (25 mL) twice to thoroughly wash off the (1S,3S)-3-aminocyclohexanol. The EA phase was dried by rotary evaporation to obtain the crude product of 9-heptadecyl-8-(((1S,3S)-3-hydroxycyclohexyl)amino)octanoate (Compound (6-VII-II)). The crude product was purified by column chromatography (dichloromethane: methanol=20:1 to 5:1) to give 1.63g of Compound (6-VII-II) ,with a purity of 98.5% and a yield of 75.5%.

NMR data:

$^1$H NMR (600 MHz, $CDCl_3$): δ 4.91-4.82 (m, 1H), 4.16-4.10 (m, 1H), 2.96-2.87 (m, 1H), 2.65-2.55 (qt, J =11.2, 7.3 Hz, 2H), 2.28-2.26 (t, J =7.5 Hz, 2H), 1.93-1.77 (m, 4H), 1.67-1.55 (m, 6H), 1.54-1.45 (m, 6H), 1.33-1.31 (m, 6H), 1.30-1.22 (m, 24H), 0.88 (t, J =7.0 Hz, 6H).

LCMS:496.7 $[M+H]^+$.

The above Compound (6-VII-II) (1.5g, 3.0mmol) was taken into a 100mL single-necked flask, added with acetonitrile (25mL) and cyclopentyl methyl ether (15mL), followed by addition of potassium carbonate powder (1.67g,

12mmol), potassium iodide (0.5g, 3mmol) and nonyl 8-bromooctanoate (1.26g, 3.6mmol), The resulting mixture was reacted at 90 °C for 24h, and the raw material Compound (6-VII-II) was depleted. The reaction mixture was removed from oil bath and cooled to room temperature, then filtered to remove the solid. The filtrate was dried by rotary evaporation to obtain a crude product, which was finally purified by column chromatography (dichloromethane: methanol=20:1 to 5:1) to give 1.37g of Compound (I-6-III) , with a purity of 99.3% and a yield of 59.5%.

NMR data:

$^1$H NMR (600 MHz, $CDCl_3$): δ 4.91-4.80 (m, 1H), 4.28-4.22 (m, 1H), 4.06-4.04 (t, J =6.8 Hz, 2H), 3.03-2.97 (m, 1H), 2.47-2.41 (m, 4H), 2.33-2.25 (q, J =7.5 Hz, 4H), 1.89-1.85 (d, J =12.1 Hz, 1H), 1.80-1.77 (t, J =12.2 Hz, 1H), 1.73-1.65 (m, 2H), 1.64-1.59 (m, 6H), 1.54-1.48 (m, 4H), 1.48-1.39 (m, 4H), 1.34-1.29 (m, 14H), 1.29-1.23 (m, 38H), 0.89-0.87 (m, 9H).

Specific rotation:-12.9°

LCMS: 765.2 $[M+H]^+$.

Example 16: Synthesis of Compound (I-6-IV)

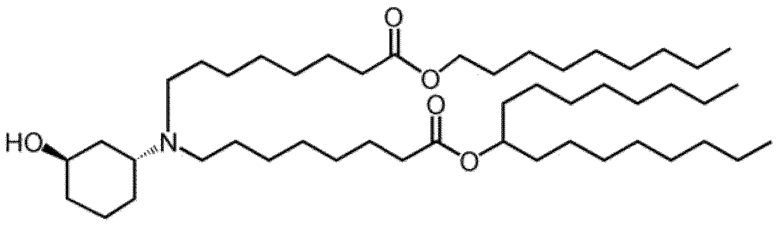

(I-6-IV)

To a 100 mL single-necked flask, 9-heptadecyl-8-bromooctanoate (2g, 4.3mmol), (1R,3R)-3-aminocyclohexanol (2.5g, 20.2mmol) and ethanol (20ml) were added. The resulting mixture was reacted at 50 °C for 15h, and the raw material 9-heptadecyl-8-bromooctanoate was depleted. After the solvent was removed by rotary evaporation, the resulting crude was added with EA (50 mL), washed with water (25 mL) twice to thoroughly wash off the (1R,3R)-3-aminocyclohexanol. The EA phase was dried by rotary evaporation to obtain a crude product of 9-heptadecyl 8-(((1R,3R)-3-hydroxycyclohexyl)amino)octanoate (Compound (6-VII-III)). The crude product was purified by column chromatography (dichloromethane: methanol=20:1 to 5:1) to give 1.58g of Compound (6-VII-III) ,with a purity of 99.2% and a yield of 73.2%.

NMR data:

$^1$H NMR (600 MHz, $CDCl_3$): δ 4.91-4.81 (m, 1H), 4.17-4.08 (m, 1H), 2.94-2.84 (m, 1H), 2.64-2.5 (qt, J =11.2, 7.3 Hz, 2H), 2.28-2.26 (t, J =7.5 Hz, 2H), 1.88-1.76 (m, 2H), 1.75-1.54 (m, 8H), 1.54-1.40 (m, 6H), 1.33-1.3 (m, 6H), 1.30-1.23 (m, 24H), 0.88 (t, J =7.0 Hz, 6H).

LCMS: 496.7 $[M+H]^+$.

The above Compound (6-VII-III) (1.5g, 3.0mmol) was taken into a 100mL single-necked flask, added with acetonitrile (25mL) and cyclopentyl methyl ether (15mL), followed by addition of potassium carbonate powder (1.67g, 12mmol), potassium iodide (0.5g, 3mmol) and nonyl 8-bromooctanoate (1.26g, 3.6mmol). The resulting mixture was reacted at 90 °C for 24h, and the raw material compound (6-VII-III) was depleted. The reaction mixture was removed from oil bath and cooled to room temperature, then filtered to remove the solid. The filtrate was dried by rotary evaporation to obtain a crude product, which was finally purified by column chromatography (dichloromethane: methanol=20:1 to 5:1) to give 1.29g of Compound (I-6-IV) , with a purity of 98.6% and a yield of 55.8%.

NMR data:

$^1$H NMR (600 MHz, $CDCl_3$): δ 4.89-4.82 (m, 1H), 4.24-4.22 (m, 1H), 4.06-4.04 (t, J =6.8 Hz, 2H), 3.00-2.95 (m, 1H), 2.47-2.36 (m, 4H), 2.30-2.26 (q, J =7.5 Hz, 4H), 1.86-1.84 (d, J =12.1 Hz, 1H), 1.79-1.77 (t, J =12.2 Hz, 1H), 1.70-1.66 (m, 2H), 1.65-1.57 (m, 6H), 1.53-1.48 (m, 4H), 1.45-1.38 (m, 4H), 1.35-1.30 (m, 14H), 1.29-1.22 (m, 38H), 0.89-0.87 (m, 9H).

Specific rotation:+12.6°

LCMS: 765.2 $[M+H]^+$.

Example 17 : Synthesis of Compound (I-6-V)

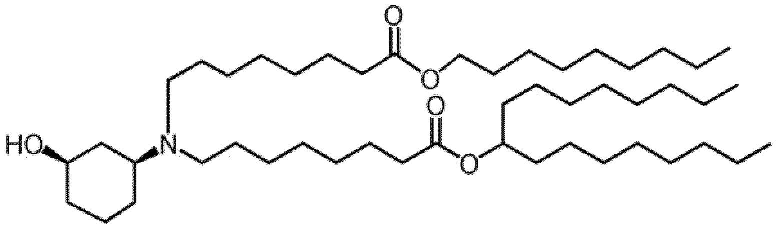

(I-6-V)

To a 250 mL single-necked flask, 9-heptadecyl 8-bromooctanoate (8g, 17.3mmol), (1R,3S)-3-aminocyclohexanol (10g, 76.5mmol) and ethanol (100 mL) were added. The resulting mixture was reacted at 50 °C for 15h, and the raw material 9-heptadecyl-8-bromooctanoate was depleted. After the solvent was removed by rotary evaporation, the resulting crude was added with EA (200 mL), washed with water (100 mL) twice to thoroughly wash off the (1R,3S)-3-aminocyclohexanol. The EA phase was dried by rotary evaporation to obtain a crude product of 9-heptadecyl 8-(((1S,3R)-3-hydroxycyclohexyl)amino)octanoate (Compound (6-VII-IV)). The crude product was purified by column chromatography (dichloromethane: methanol=20:1 to 5:1) to give 6.4g of Compound (6-VII-IV), with a purity of 98.5% and a yield of 74.8%.

NMR data:

$^1$H NMR (600 MHz, $CDCl_3$): δ 4.90-4.83 (m, 1H), 3.86-3.82 (m, 1H), 2.86-2.82 (m, 1H), 2.69-2.58 (qt, J =11.2, 7.3 Hz, 2H), 2.29-2.26 (t, J =7.5 Hz, 2H), 1.94-1.86 (m, 1H), 1.79-1.77 (d, J =12.0 Hz, 1H), 1.74-1.64 (m, 3H), 1.64-1.59 (m, 3H), 1.51-1.46 (m, 6H), 1.36-1.30 (m, 8H), 1.30-1.22 (m, 24H), 0.88 (t, J =7.0 Hz, 6H).

LCMS: 496.7 $[M+H]^+$.

The above Compound (6-VII-IV) (6g, 12.1mmol) was taken into a 250mL single-necked flask, added with acetonitrile (90ml) and cyclopentyl methyl ether (60ml), followed by addition of potassium carbonate powder (6.7g, 48.4mmol), potassium iodide (2g, 12.1mmol) and nonyl 8-bromooctanoate (5g, 17.4mmol). The resulting mixture was reacted at 90 °C for 24h, and the raw material Compound (6-VII-IV) was depleted. The reaction mixture was removed from oil bath and cooled to room temperature ,then filtered to remove the solid. The filtrate was dried by rotary evaporation to obtain a crude product, which was finally purified by column chromatography (dichloromethane: methanol=20:1 to 5:1) to give 5.35g of Compound (I-6-V) ,with a purity of 99.1% and a yield of 57.9%.

NMR data:

$^1$H NMR (600 MHz, $CDCl_3$): δ 4.90-4.82 (m, 1H), 4.05 (t, J =6.8 Hz, 2H), 3.70-3.60 (m, 1H), 2.64-2.54 (m, 1H), 2.51-2.41 (m, 4H), 2.30-2.26 (q, J =7.5 Hz, 4H), 1.95 (m, 1H), 1.91-1.77 (m, 2H), 1.69-1.56 (m, 7H), 1.50 (d, J =6.0 Hz, 4H), 1.41-1.37 (m, 4H), 1.34-1.18 (m, 52H), 0.88 (m, 9H).

Specific rotation: -4.5°

LCMS: 765.2 $[M+H]^+$.

Example 18: Synthesis of Compound 4

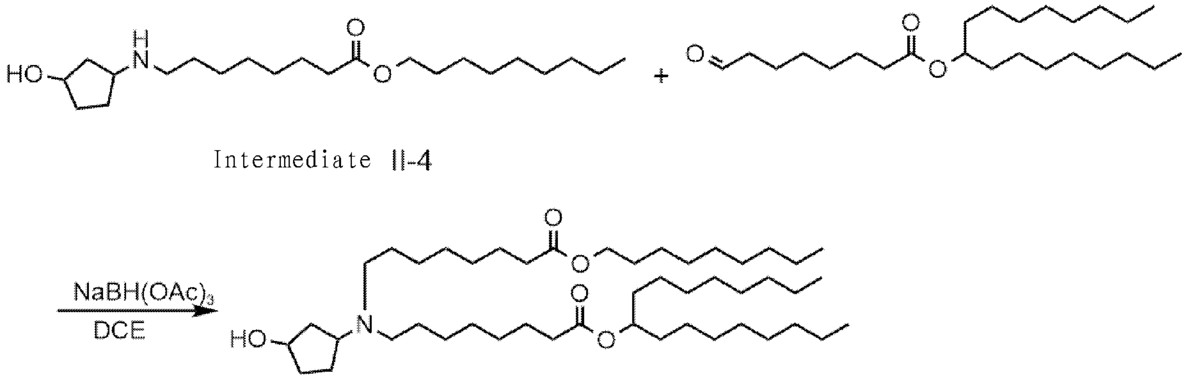


Compound 4

Compound 4 was synthesized in a similar manner to the step 3) in Example 7. Compound 4 (152mg, yield 40.75%) was synthesized from Intermediate II-4 (184mg, 0.50mmol) and 9-heptadecyl 8-oxooctanoate (296mg).

$^1$H NMR (600 MHz, $CDCl_3$): δ 4.89-4.84 (m, 1H), 4.44-4.38 (m, 0.5H), 4.27-4.19 (m, 0.5H), 4.05 (t, J =6.8 Hz, 2H), 3.53-3.41 (m, 0.5H), 3.24-3.14 (m, 0.5H), 2.76 (t, J =6.0 Hz, 2H), 2.72-2.63 (t, J =6.0 Hz, 2H), 2.33-2.24 (m, 4H), 2.21-2.00 (m, 2H), 1.91-1.74 (m, 2H), 1.72-1.45 (m, 15H), 1.39-1.19 (m, 49H), 0.88 (m, 9H).

LC-MS:751.2[M+H]$^+$.

Example 19: Synthesis of Compound 5

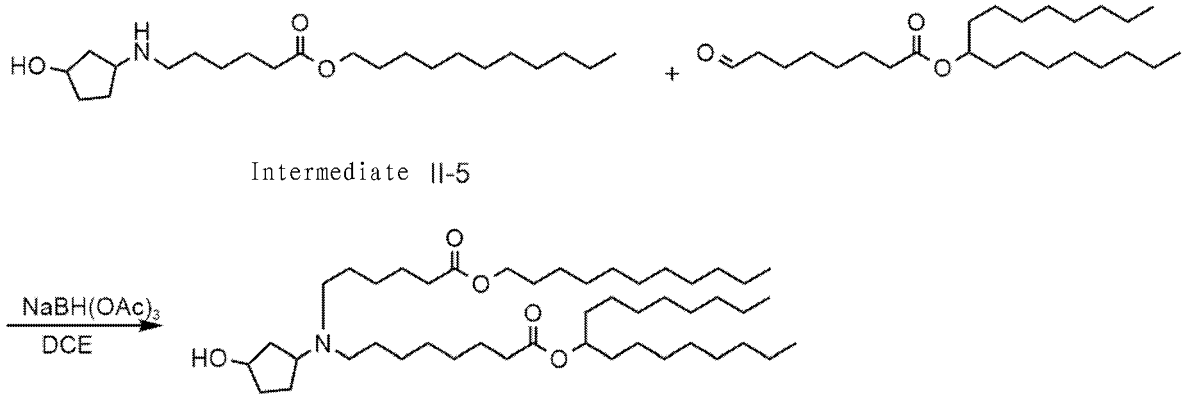


Compound 5

Compound 5 was synthesized in a similar manner to the step 3) in Example 7. Compound 5 (250mg, yield 81.9%) was synthesized from Intermediate II-5 (150mg, 0.38mmol) and 9-heptadecyl 8-oxooctanoate (225mg).

$^1$H NMR (600 MHz, $CDCl_3$): δ 4.93-4.81 (m, 1H), 4.44-4.36 (m, 0.5H), 4.29-4.20 (m, 0.5H), 4.10-4.01 (m, 2H), 3.54-3.42 (m, 0.5H), 3.24-3.08 (m, 0.5H), 2.73 (m, 3H), 2.36-2.25 (m, 4H), 2.17-2.08 (m, 1H), 2.08-2.00 (m, 1H), 1.95-1.75 (m, 2H), 1.71-1.57 (m, 8H), 1.57-1.42 (m, 8H), 1.40-1.18 (m, 49H), 0.88 (m, 9H).

LC-MS: 751.0 [M+H]$^+$.

Example 20: Synthesis of Compound 11

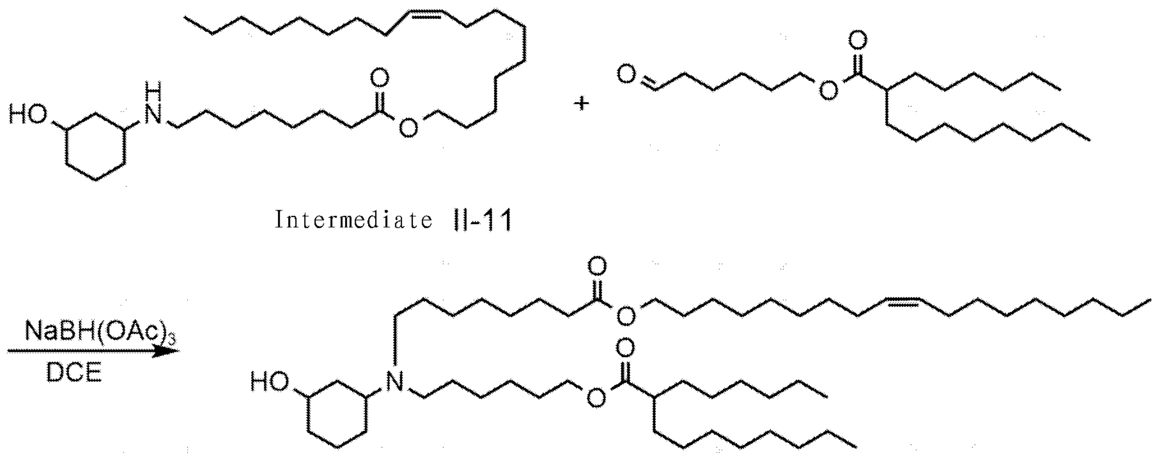


Intermediate II-11

Compound 11

Compound 11 was synthesized in a similar manner to the step 3) in Example 7. Compound 5 (96mg, yield 42.5%) was synthesized from Intermediate II-11 (140mg, 0.28mmol) and 9-heptadecyl 8-oxooctanoate(146mg).

$^{1}$H NMR (600 MHz, $CDCl_3$) δ 5.37-5.29 (m, 2H), 4.21-4.19 (m, 0.5H), 4.02-4.00 (m, 4H), 3.67-3.60 (m, 0.5H), 2.99-2.91 (m, 0.5H), 2.57-2.53 (m, 0.5H), 2.46-2.34 (m, 4H), 2.29-2.25 (m, 3H), 2.01 -1.97(m, 3H), 1.96-1.88 (m, 2H), 1.83-1.80 (m, 1H), 1.79-1.72 (m, 1H), 1.58-1.52 (m, 9H), 1.43-1.35 (m, 6H), 1.31-1.18 (m, 56H), 0.87-0.84 (m, 9H).

LC-MS: 847.3 $[M+H]^+$.

Example 21: Synthesis of Compound 18

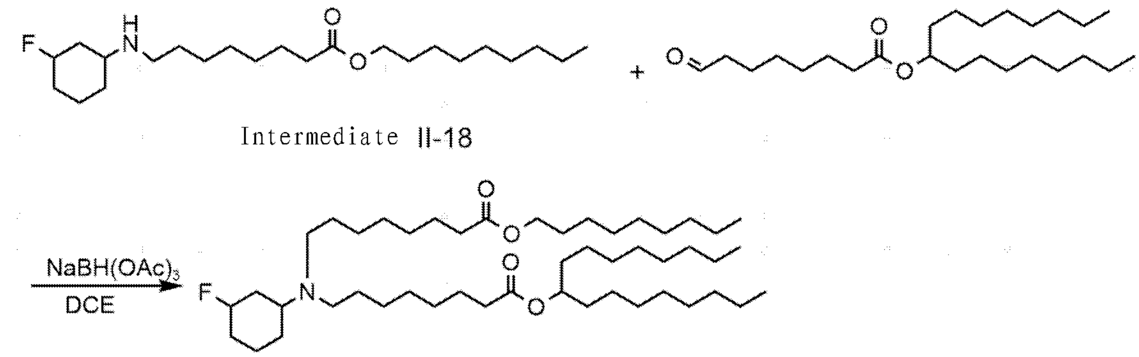


Intermediate II-18

Compound 18

Compound 18 was synthesized in a similar manner to the step 3) in Example 7. Compound 18 (177mg, yield 74.4%) was synthesized from Intermediate II-18 (120mg, 0.31mmol) and 9-heptadecyl 8-oxooctanoate (185mg).

$^{1}$H NMR (600 MHz, $CDCl_3$): δ 5.04-5.02 (m, 0.5H), 4.96-5.04 (m, 0.5H), 4.95-4.81 (m, 1H), 4.30-4.12 (m, 0.5H), 4.08-4.06 (t, J =6.8 Hz, 2H), 3.72-3.61 (m, 0.5H), 2.98-2.96 (m, 0.5H), 2.64-2.52 (m, 0.5H), 2.48-2.37 (m, 4H), 2.31-2.29 (m, 4H), 1.93-1.81 (m, 2H), 1.72-1.57 (m, 8H), 1.52-1.50 (m, 4H), 1.43-1.18 (m, 55H), 0.91-0.87 (m, 9H).

LC-MS: 767.3$[M+H]^+$.

Example 22 : Synthesis of Compound 21

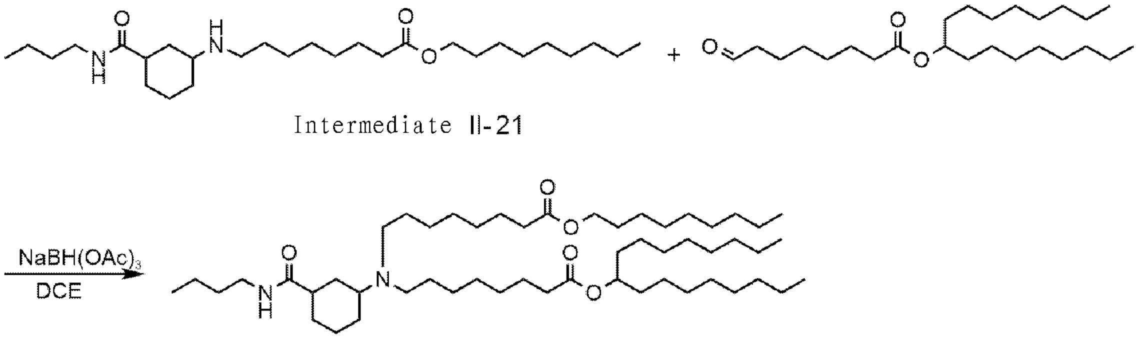


Intermediate II-21

Compound 21

Compound 21 was synthesized in a similar manner to the step 3) in Example 7. Compound 18 (100mg, yield 13.8%) was synthesized from Intermediate II-21 (400mg, 0.86mmol) and 9-heptadecyl 8-oxooctanoate (510mg).

$^1$H NMR (600 MHz, $CDCl_3$): δ 4.90-4.81 (m, 1H), 4.06-4.04 (t, J =6.8 Hz, 2H), 3.26-3.22 (dq, J =8.9, 6.9 Hz, 2H), 2.66-2.55 (m, 1H), 2.53-2.35 (m, 4H), 2.30-2.26 (q, J =7.5 Hz, 4H), 2.12-2.04 (m, 1H), 1.95-1.93 (m, 1H), 1.90-1.85 (m, 1H), 1.78-1.70 (m, 2H), 1.66-1.57 (m, 6H), 1.55-1.45 (m, 6H), 1.43-1.38 (m, 5H), 1.37-1.20 (m, 54H), 0.94-0.90 (t, J =6.0 Hz, 3H), 0.88-0.86 (m, 9H).

LC-MS: 848.3[M+H]$^+$.

Example 23: Synthesis of Compound 22

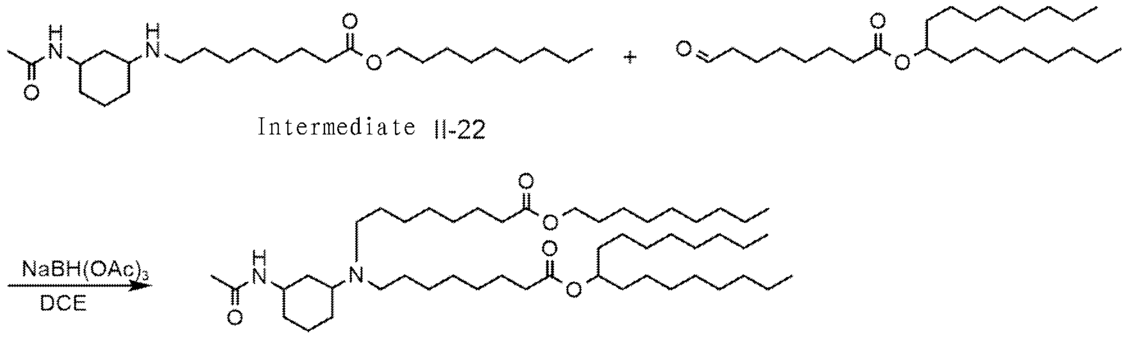


Intermediate II-22

Compound 22

Compound 22 was synthesized in a similar manner to the step 3) in Example 7. Compound 18 (266mg, yield 46.8%) was synthesized from Intermediate II-22 (300mg, 0.71mmol) and 9-heptadecyl 8-oxooctanoate (422mg).

$^1$H NMR (600 MHz, $CDCl_3$): δ 4.88-4.80 (m, 1H), 4.03-3.98 (t, J =6.8 Hz, 2H), 3.96-3.94(m, 1H), 2.49-2.32 (m, 4H), 2.28-2.23 (m, 4H), 2.09-2.01 (m, 1H), 1.93-1.91 (m, 1H), 1.88-1.83 (m, 1H), 1.76-1.68 (m, 2H), 1.64-1.53 (m, 6H), 1.53-1.43 (m, 6H), 1.40-1.35 (m, 5H), 1.34-1.16 (m, 52H), 0.84-0.81 (m, 9H).

LC-MS: 806.1[M+H]$^+$.

Comparative Examples:

Comparative Example 1: Synthesis of Comparative Compound 1

Step 1): Synthesis of nonyl 8-((2-hydroxycyclopentyl)amino)octanoate

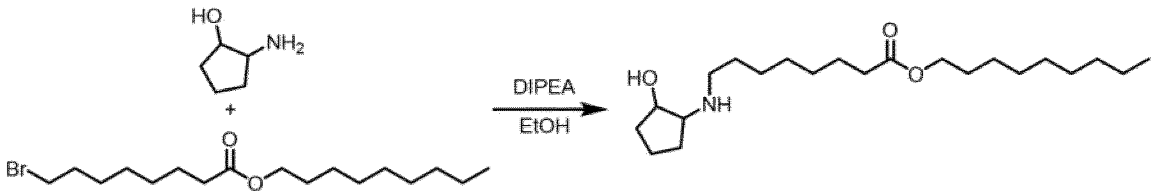


To a 100 mL flask, nonyl 8-bromooctanoate (3.50 g, 10 mmol), 2-aminocyclopentanol (10.12 g, 100 mmol) and ethanol (30 mL)were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((2-hydroxycyclopentyl)amino) octanoate (2.29 g, 62%).

$^1$H NMR (600 MHz, $CDCl_3$): δ 4.07 (t, 2H), 4.01-3.94 (q, 1H), 2.91 (q, 1H), 2.72 (m, 1H), 2.63 (m, 1H), 2.31 (t, 2H), 2.09-1.97 (m, 2H), 1.72 (m, 2H), 1.67-1.59 (m, 4H), 1.56 (m, 3H), 1.45-1.38 (m, 1H), 1.38-1.22 (m, 18H), 0.90 (t, 3H).

LCMS:370.3[M+H]$^+$.

Step 2): Synthesis of Comparative compound 1

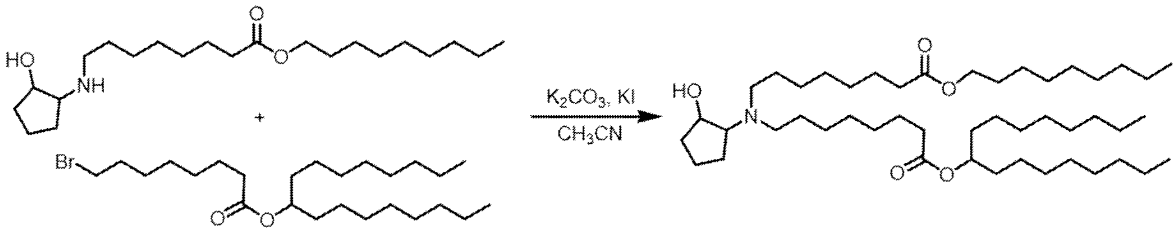


Comparative compound 1

Comparative compound 1 was synthesized in a similar manner to the step 3) in Example 1, except that the Intermediate nonyl 8-((2-hydroxycyclopentyl)amino)octanoate instead of the Intermediate nonyl 8-((3-hydroxycyclobutyl) amino)octanoate was used.

$^1$H NMR (600 MHz, $CDCl_3$): δ 4.91-4.80 (q, 1H), 4.05 (t, 2H), 3.92 (q, 1H), 2.85 (q, 1H), 2.50 (m, 2H), 2.44-2.35 (m, 2H), 2.28 (q, 4H), 1.95-1.87 (m, 2H), 1.83 (br, 2H), 1.75-1.66 (m, 2H), 1.64-1.56 (m, 6H), 1.55-1.47 (m, 5H), 1.47-1.38 (m, 5H), 1.36-1.18 (m, 46H), 0.88 (m, 9H).

LCMS:750.9[M+H]$^+$.

Comparative Example 2: Synthesis of Comparative Compound 2

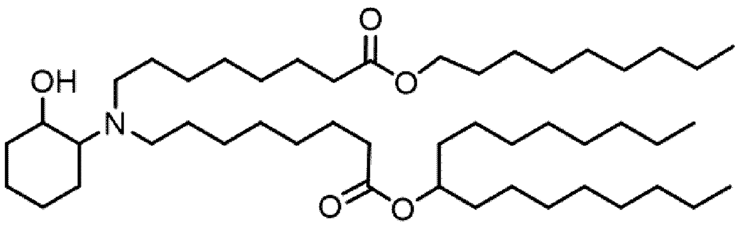

Comparative compound 2

Comparative compound 2 was prepared according to the synthesis method of Compound 22 in Chinese Patent Application No.CN110520409A.

$^1$H NMR (600 MHz, $CDCl_3$): δ4.10- 4.07 (t, 2H), 3.26-3.20 (m, 1H), 2.85-2.79 (m, 1H), 2.54-2.48 (m, 1H), 2.34-2.30 (t, 2H), 2.28-2.19 (m, 1H), 2.15-2.01 (m, 2H), 1.75 (m, 2H), 1.69-1.58 (m, 4H), 1.58-1.46 (m, 2H), 1.41-1.25 (m, 20H), 0.91 (t, 3H).

LCMS:765.0[M+H]+.

Comparative Example 3: Synthesis of Comparative Compound 3

Step 1): Synthesis of undecyl 6-((2-hydroxycyclohexyl)amino)hexanoate

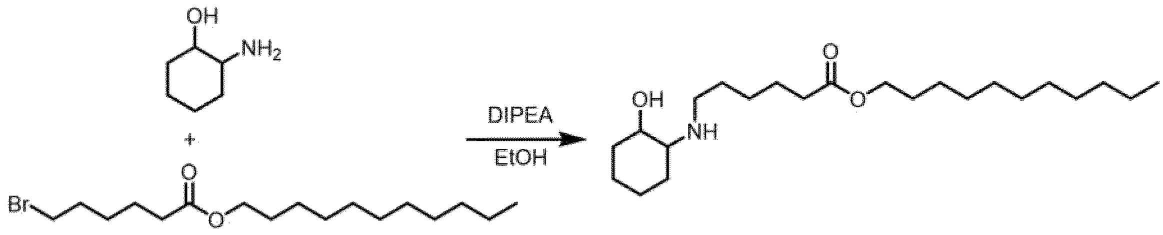


To a 100 mL flask, undecyl 6-bromohexanoate (3.50 g, 10 mmol), 2-aminocyclohexanol (11.5 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give undecyl 6-((2-hydroxycyclohexyl) amino)hexanoate (2.57 g, 67%).

$^1$H NMR (600 MHz, $CDCl_3$): δ 4.05 (t, 2H), 3.31-3.20 (m, 1H), 2.90-2.75 (m, 1H), 2.53 (m, 1H), 2.35 -2.26 (m, 3H), 2.13-2.01 (m, 2H), 1.80-1.68 (m, 2H), 1.67-1.58 (m, 4H), 1.57-1.46 (m, 2H), 1.44-1.34 (m, 2H), 1.34-1.15 (m, 20H), 0.88 (t, 3H).

LCMS:384.4[M+H]+.

Step 2): Synthesis of Comparative Compound 3

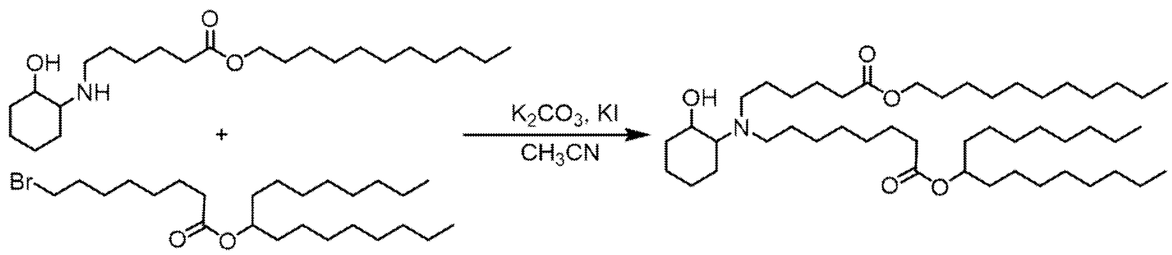


Comparative compound 3

To a 100 mL flask ,undecyl 6-((2-hydroxycyclohexyl)amino)hexanoate (383 mg, 1 mmol), 1-octylnonyl 8-bromooctanoate (554 mg, 1.2 mmol) and acetonitrile (20 mL)were added in sequence. The mixture was stirred to be dissolved, and then potassium carbonate (276 mg, 2 mmol) and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Comparative Compound 3 (550 mg, 72%).

$^1$H NMR (600 MHz, $CDCl_3$): δ 4.86 (m, 1H), 4.05 (t, 2H), 3.39-3.21 (m, 1H), 2.55-2.43 (m, 2H), 2.35- 2.23 (m, 6H), 2.10 (m, 1H), 1.76 (m, 2H), 1.70 (m, 1H), 1.67-1.57 (m, 6H), 1.49 (m, 6H), 1.38-1.19 (m, 55H), 0.88 (m, 9H).

LCMS:765.0[M+H]+.

Comparative Example 4: Synthesis of Comparative Compound 4

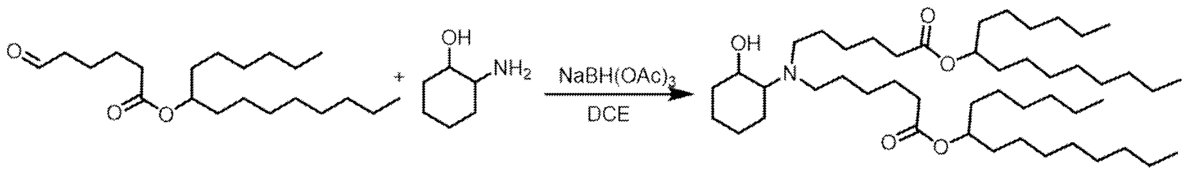


Comparative compound 4

To a 250 mL flask, pentadecan-7-yl 6-oxohexanoate (3.40 g, 10 mmol), dichloroethane (100 mL) and sodium triacetoxyborohydride (3.18g, 15 mmol) were added in sequence, followed by addition of 2-aminocyclohexanol (0.57 g, 5 mmol). The resulting mixture was reacted at room temperature for 24h, washed with water for three times, dried over anhydrous sodium sulfate, concentrated, and purified with a flash column chromatography system (dichloromethane: methanol=20:1 to 5:1) to give Comparative Compound 4 (2.67g, 70%).

$^1$H NMR (600 MHz, $CDCl_3$): δ 4.91-4.80 (m, 2H), 3.28 (m, 1H), 2.48 (m, 2H), 2.30 (m, 6H), 2.24 (m, 1H), 2.11 (m, 1H), 1.75 (m, 2H), 1.68 (m, 1H), 1.66-1.60 (m, 4H), 1.48 (m, 10H), 1.44-1.07 (m, 50H), 0.88 (t, 12H).

LCMS:765.2$[M+H]^+$.

Comparative Example 5: Synthesis of Comparative Compound 5 (SM102)

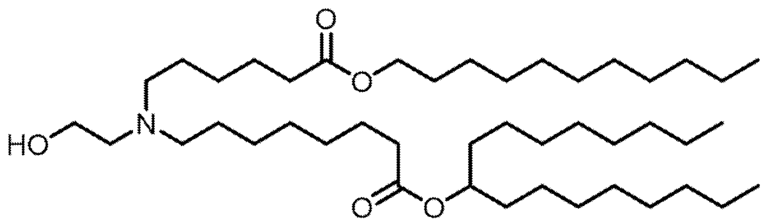

Comparative compound 5

Comparative compound 5 was prepared according to the synthesis method of Compound 25 in Chinese Patent Application No. CN110520409A.

$^1$H NMR (600 MHz, $CDCl_3$): δ 4.86-4.74 (m, 1H), 3.99 (t, 2H), 3.46 (t, 2H), 2.51 (t, 2H), 2.39 (q, 4H), 2.26-2.18 (m, 4H), 1.62-1.52 (m, 6H), 1.47-1.36 (m, 8H), 1.28-1.14 (m, 48H), 0.81 (m, 9H).

LCMS:711.0$[M+H]^+$.

Comparative Example 6: Synthesis of Comparative Compound 6

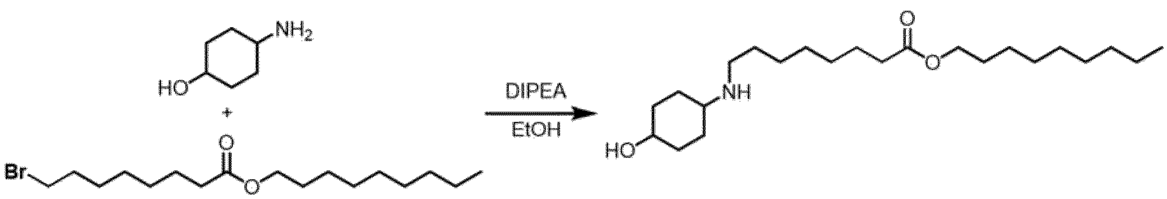


To a 100 mL flask, nonyl 8-bromooctanoate (3.50 g, 10 mmol), p-aminocyclohexanol (11.5 g, 100 mmol) and ethanol (30 mL) were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give nonyl 8-((4-hydroxycyclohexyl)amino) hexanoate (2.65 g, 69%).

$^1$H NMR (600 MHz, $CDCl_3$): δ 4.05 (t, 2H), 3.96-3.90 (m, 0.5H), 3.67-3.57 (m, 0.5H), 2.75-2.67 (t, 2H), 2.66 (m, 0.5H), 2.54 (m, 0.5H), 2.28 (t, 2H), 2.03-1.96 (m, 1H), 1.86-1.78 (m, 1H), 1.78-1.71 (m, 3H), 1.66-1.50 (m, 7H), 1.40-1.20 (m, 20H), 0.88 (t, 3H).

LCMS:384.4[M+H]+.

Step 2): Synthesis of Comparative Compound 6

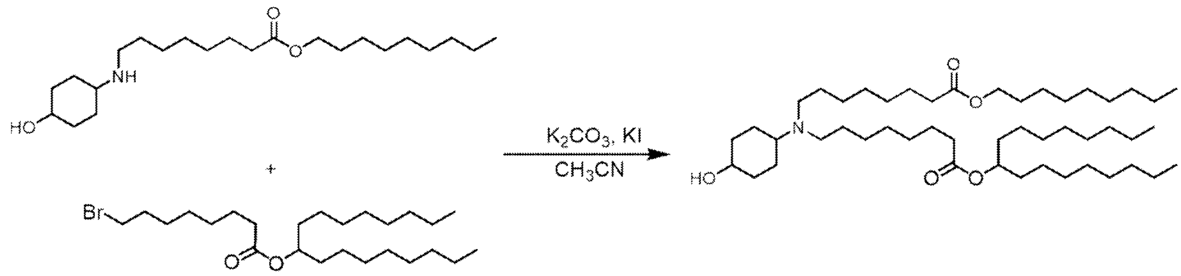


Comparative compound 6

To a 100 mL flask, nonyl 8-((4-hydroxycyclohexyl)amino)hexanoate (383 mg, 1 mmol), 1-octylnonyl 8-bromooctanoate (554 mg, 1.2 mmol) and acetonitrile (20 mL) were added in sequence. The mixture was stirred to be dissolved, and then potassium carbonate (276 mg, 2 mmol) and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Comparative compound 6 (535 mg, 70%).

$^1$H NMR (600 MHz, $CDCl_3$): δ 4.90-4.82 (m, 1H), 4.05 (t, 2H), 3.99 (m, 0.5H), 3.59-3.51 (m, 0.5H), 2.47 (m, 0.5H), 2.45-2.42 (m, 3H), 2.39-2.32 (m, 1.5H), 2.28 (m, 4H), 2.01 (m, 1H), 1.82 (m, 1H), 1.76 (m, 1H), 1.68-1.58 (m, 7H), 1.52 (m, 4H), 1.43-1.36 (m, 4H), 1.36-1.20 (m, 52H), 0.88 (m, 9H).

LCMS:765.0[M+H]+.

Comparative Example 7: Synthesis of Comparative Compound 7

Step 1): Synthesis of undecyl 6-((4-hydroxycyclohexyl)amino)hexanoate

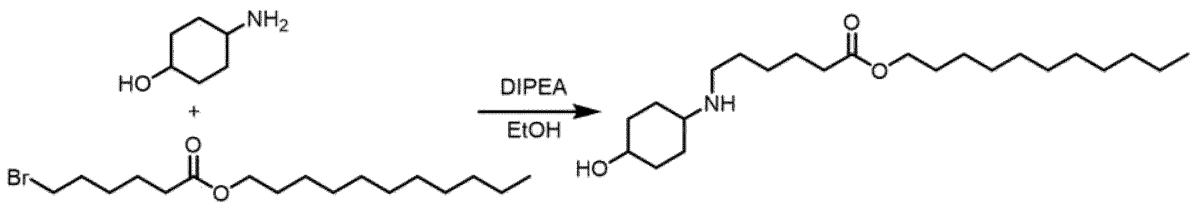


To a 100 mL flask, undecyl 6-bromohexanoate (3.50 g, 10 mmol), p-aminocyclohexanol (11.5 g, 100 mmol) and ethanol (30 mL)were added in sequence. The mixture was stirred to be dissolved, and then N,N-diisopropylethylamine (2.58 g, 20 mmol) was added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give undecyl 6-((4-hydroxycyclohexyl) amino)hexanoate (2.57 g, 67%).

$^1$H NMR (600 MHz, $CDCl_3$): δ 4.05 (t, 2H), 3.94 (m, 0.5H), 3.62 (m, 0.5H), 2.73 (t, 2H), 2.70-2.65 (m, 0.5H), 2.59-2.51 (m, 0.5H), 2.30 (t, 2H), 1.99 (m, 1H), 1.85-1.80 (m, 1H), 1.80-1.73 (m, 3H), 1.66 -1.50 (m, 7H), 1.39-1.21 (m, 20H), 0.88 (t, 3H).

LCMS:384.4[M+H]+.

Step 2): Synthesis of Comparative Compound 7

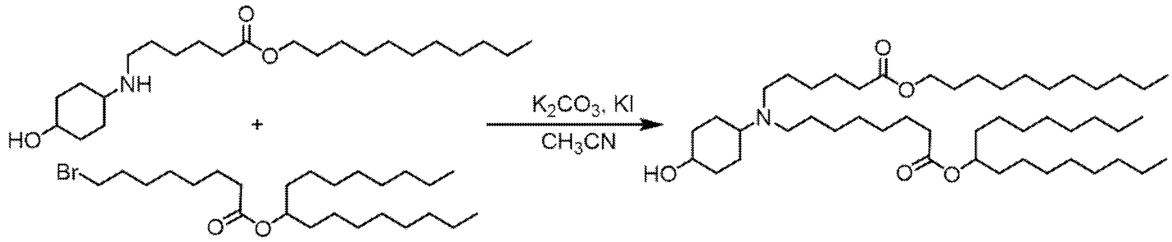


Comparative compound 7

To a 100 mL flask, undecyl 6-((4-hydroxycyclohexyl)amino)hexanoate (383 mg, 1 mmol), 1-octylnonyl 8-bromooctanoate (554 mg, 1.2 mmol) and acetonitrile (20 mL)were added in sequence. The mixture was stirred to be dissolved, and then potassium carbonate (276 mg, 2 mmol) and potassium iodide (166 mg, 1 mmol) were added. The resulting mixture was reacted at room temperature for 24h, added with dichloromethane (100 mL), washed with water for three times, dried over anhydrous sodium sulfate, concentrated, and purified with a flash column chromatography system (dichloromethane: methanol=20:1 to 5:1) to give Comparative compound 7 (550 mg, 72%).

$^1H$ NMR (600 MHz, $CDCl_3$): δ 4.93-4.83 (m, 1H), 4.07 (t, 2H), 3.99 (m, 0.5H), 3.63-3.49 (m, 0.5H), 2.52 (m, 0.5H), 2.49-2.44 (m, 3H), 2.40 (m, 1.5H), 2.30 (m, 4H), 2.02 (m, 1H), 1.85 (m, 1H), 1.78 (m, 1H), 1.69-1.60 (m, 7H), 1.53 (m, 4H), 1.46-1.39 (m, 4H), 1.35-1.23 (m, 52H), 0.90 (m, 9H).

LCMS:765.2$[M+H]^+$.

BIOLOGICAL EXPERIMENTS

The Lipid MC3, SM102, and ALC-0315 used in the biological experiments of the present disclosure have structures as shown below:

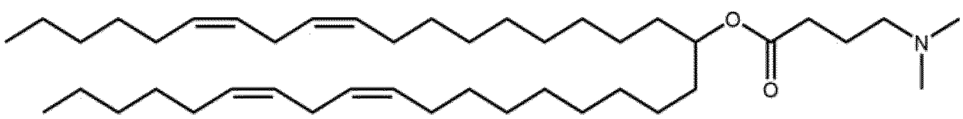

MC3

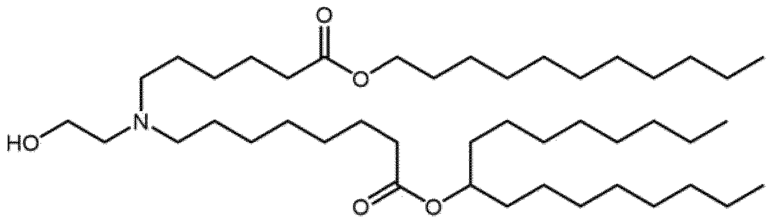

SM102

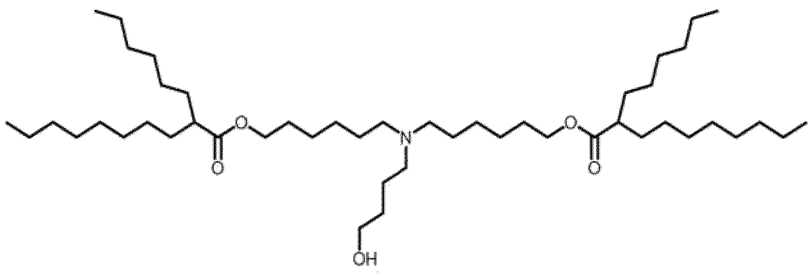

ALC-0315

,

wherein, MC3 and ALC-0315 are commercially available, or can be prepared according to techniques known in the art.

PREPARATION OF LIPID NANOPARTICLES:

Formulation method 1:

The amino lipid compounds of the present disclosure were mixed with DOPE, cholesterol, and PEG2000-DMG in a molar ratio of 45:10:42.5:2.5 and dissolved in absolute ethanol. By using a microinjection pump, the resultant ethanol solution and a sodium acetate solution (50 mM, pH = 4.0) were mixed in a ratio of 1:3 in a microfluidic chip to prepare a crude solution of lipid nanoparticles, which were then dialyzed in 1X PBS at a controlled temperature of 4°C for 6 h using a dialysis box (Fisher, MWCO 20,000), and filtered through a 0.22 μm microporous filter membrane before use.

Formulation method 2:

The formulation method was the same as the formulation method 1, except that the molar ratio of the amino lipid compounds to DSPC, cholesterol and PEG2000-DMG was 50:10:38.5:1.5.

The lipid nanoparticles prepared according to the above formulation method 1 or 2 were used in the biological experiments below for evaluating the performances of in vivo delivery of luciferase mRNA (Fluc mRNA). For the lipid nanoparticles obtained in the formulation method 1, the mass ratio of the amino lipid compounds to the luciferase mRNA was about 10: 1, and the lipid nanoparticles were administered subcutaneously; and the lipid nanoparticles obtained in the formulation method 2 were administered by tail vein and intramuscular injection.

Test Example 1: Evaluation of the performances of lipid nanoparticles prepared from the amino lipid compounds of the present disclosure in in vivo delivery of luciferase mRNA

Animal preparation:

6-week-old female BALB/c mice weighed about 20 g were selected and fed in a SPF-grade feeding room. Animal tests were conducted strictly according to the guidelines of National Health institutions and the requirements of animal ethics.

In vivo delivery:

9 mice were assigned randomly to each group and injected with the lipid nanoparticles at a dosage of 0.5 mg/kg by three administration routes, i.e., subcutaneous, intramuscular and tail vein injection (three mice for each administration route). After 12 hours, D-luciferin potassium salt (200 μL, 10 mg/mL) was injected into each mouse through the tail vein. After 10 minutes, the total fluorescence intensity of each mouse was observed on a living imaging system (IVIS-200, Xenogen) and taken photographs for record. The expression intensities of Fluc mRNA delivered by representative amino lipid compounds through the three administration routes are shown in Table 2-4. MC3 was used as a control and SM102 was used as a comparative compound.

Table 2: Expression intensity of Fluc mRNA delivered by subcutaneous administration of representative amino lipid compounds

| No. | Structure | Fluorescence intensity |
|---|---|---|
| 1 | 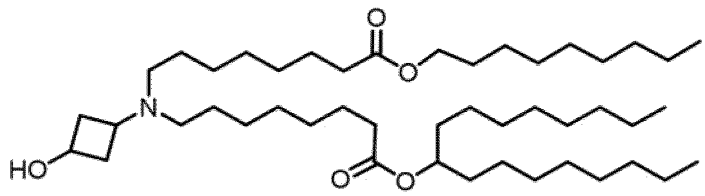 | 2.7E+07 |
| 2 | 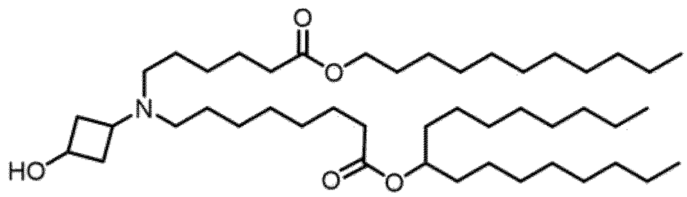 | 1.9E+08 |

(continued)

| No. | Structure | Fluorescence intensity |
|---|---|---|
| 5 | 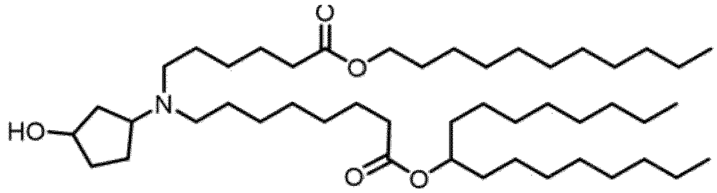 | 1.4E+07 |
| 6 | 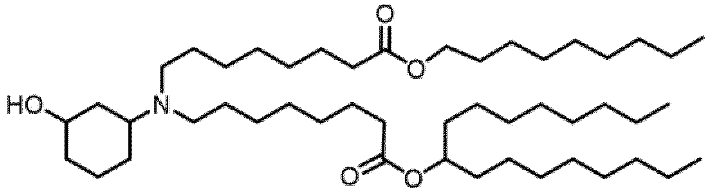 | 3.7E+08 |
| 7 | 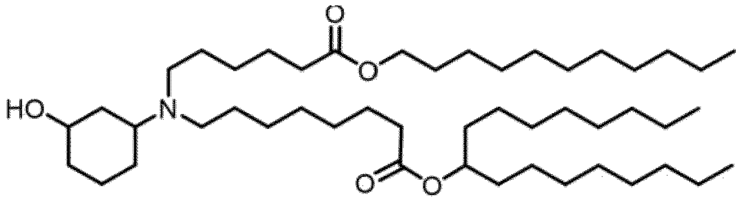 | 2.9E+08 |
| 9 | 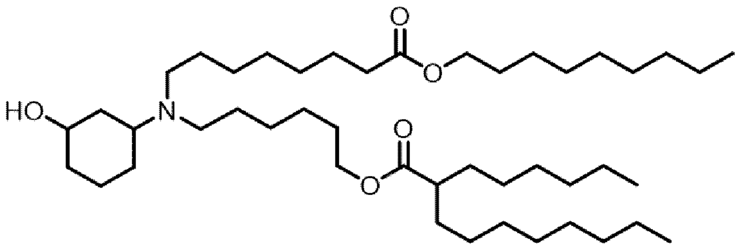 | 4.2E+07 |
| 10 | 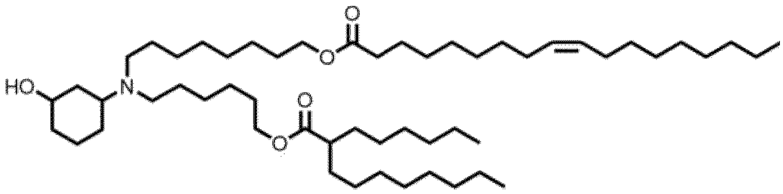 | 1.1E+07 |
| 12 | 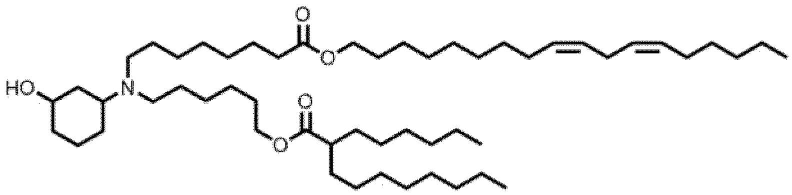 | 4.2E+06 |
| 15 | 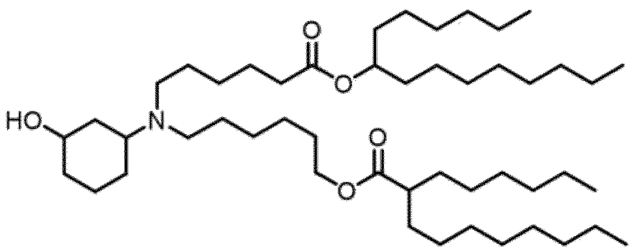 | 4.7E+07 |

(continued)

| No. | Structure | Fluorescence intensity |
| --- | --- | --- |
| 16 | 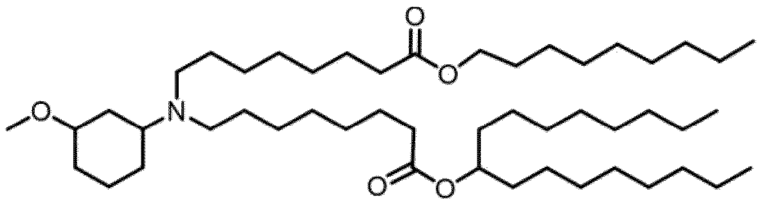 | 1.3E+06 |
| 21 | 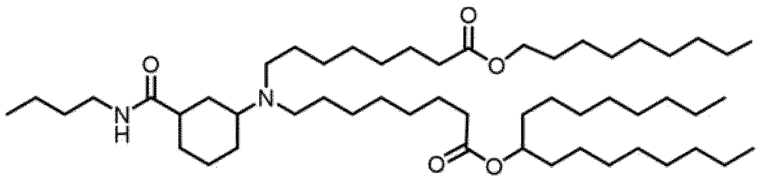 | 1.1E+06 |
| 23 | MC3 | 3.7E+06 |
| 24 | SM102 | 2.4E+07 |

Table 3: Expression intensity of Fluc mRNA delivered by intramuscular injection of representative amino lipid compounds

| No. | Structure | Fluorescence intensity |
| --- | --- | --- |
| 2 | 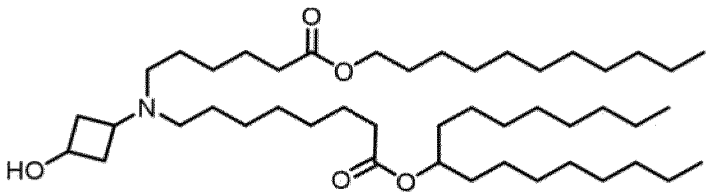 | 1.3E+07 |
| 6 | 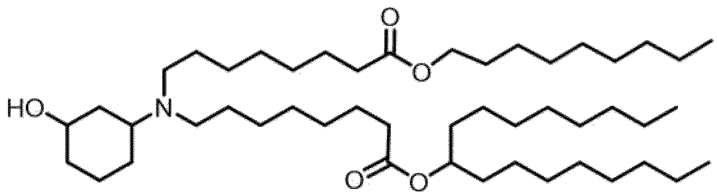 | 1.2E+08 |
| 7 | 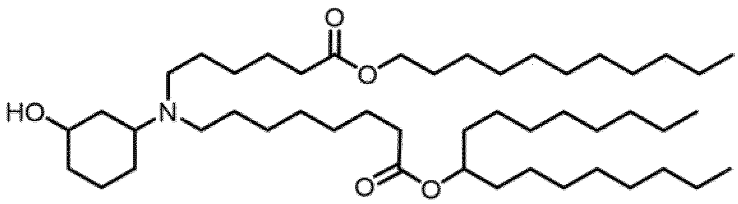 | 3.7E+07 |
| 8 | 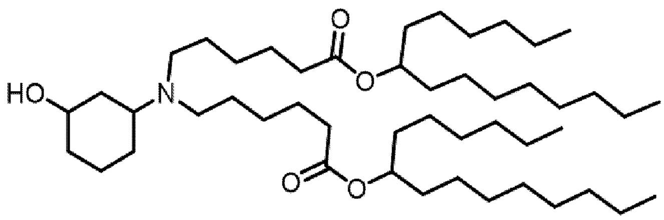 | 9.2E+06 |

(continued)

| No. | Structure | Fluorescence intensity |
|---|---|---|
| 9 | 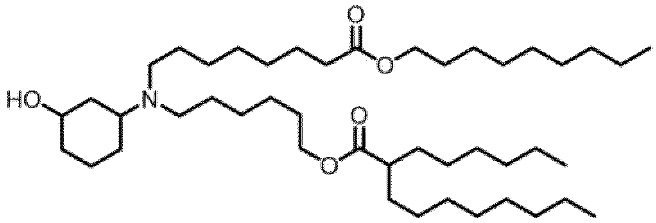 | 3.7E+07 |
| 14 | 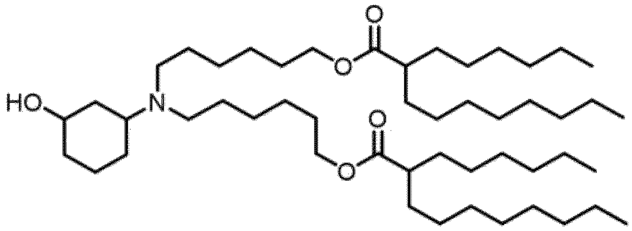 | 8.2E+06 |
| 15 | 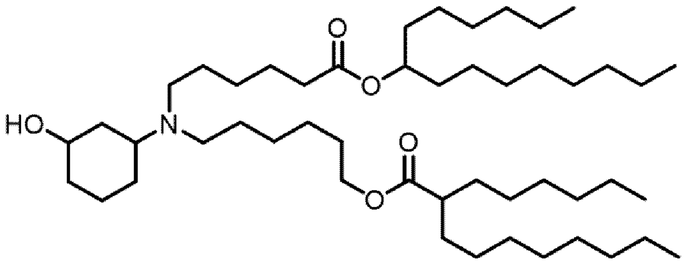 | 1.8E+07 |
| 17 | 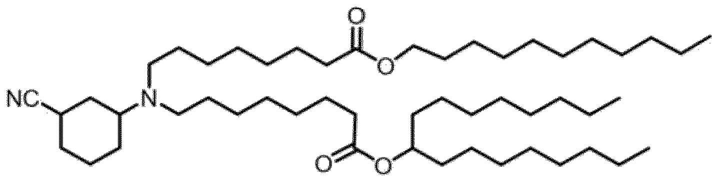 | 6.2E+06 |
| 18 | 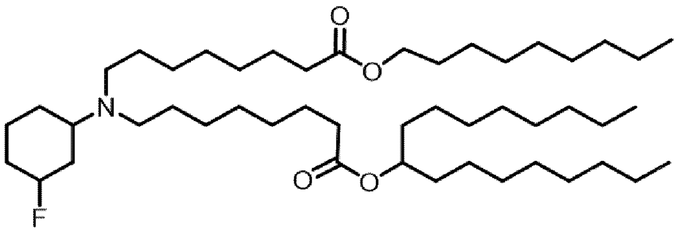 | 7.8E+06 |
| 23 | MC3 | 2.7E+07 |
| 24 | SM102 | 4.6E+07 |

Table 4: Expression intensity of Fluc mRNA delivered by tail vein administration of representative amino lipid compounds

| No. | Structure | Fluorescence intensity |
|---|---|---|
| 2 | 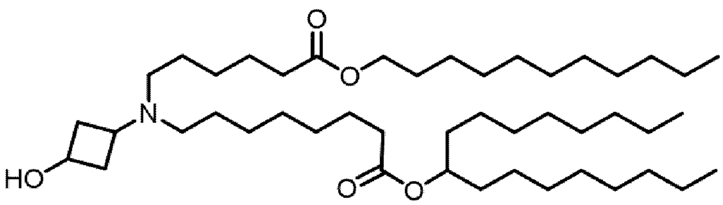 | 2.9E+06 |

(continued)

| No. | Structure | Fluorescence intensity |
|---|---|---|
| 6 | 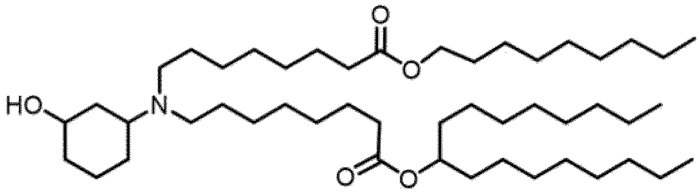 | 1.7E+08 |
| 7 | 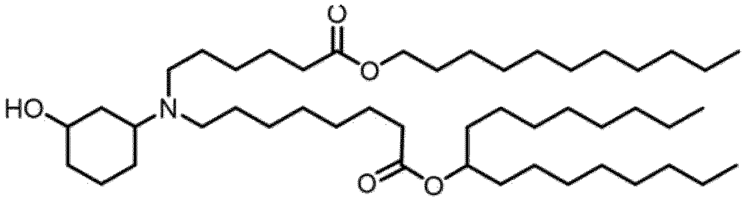 | 2.9E+07 |
| 8 | 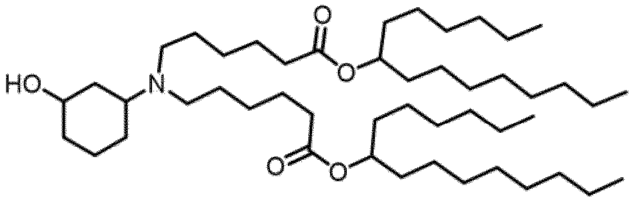 | 5.1E+06 |
| 12 | 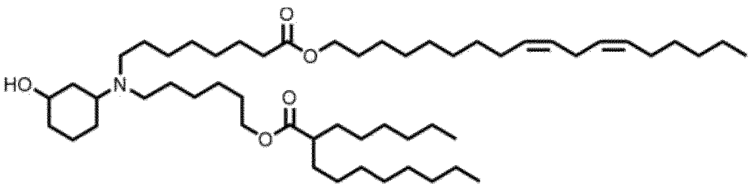 | 6.2E+06 |
| 14 | 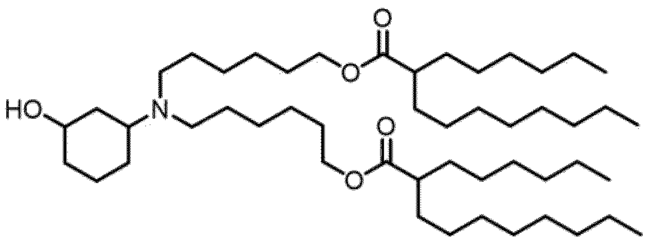 | 4.1E+06 |
| 15 | 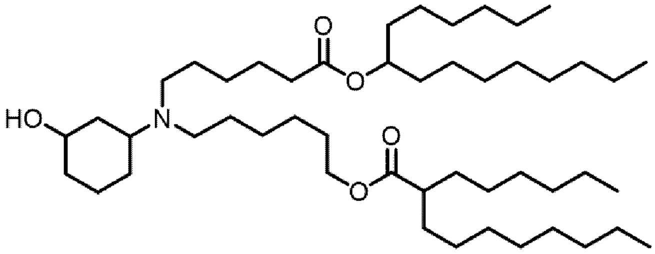 | 3.5E+06 |
| 23 | MC3 | 2.7E+07 |
| 24 | SM102 | 7.4E+06 |

Test Example 2: Evaluation of the performances of lipid nanoparticles prepared from the amino lipid compounds of the present disclosure in in vivo delivery of ovalbumin mRNA and immunity

Preparation of lipid nanoparticles:

Formulation method:

The amino lipid compounds of formula (I) of the present disclosure were mixed with DOPE, cholesterol, and PEG2000-DMG in a molar ratio of 50:10:38.5:1.5 and dissolved in absolute ethanol. Ovalbumin mRNA (OVA mRNA) was dissolved in a sodium acetate solution (50 mM, pH = 4.0). By using a microinjection pump, the resultant ethanol solution and the sodium acetate solution (50 mM, pH = 4.0) were mixed in a ratio of 1:3 in a microfluidic chip to prepare a crude solution of lipid nanoparticles, which were then dialyzed in 1X PBS at a controlled temperature of 4°C for 6 h using a dialysis box (Fisher, MWCO 20,000), and filtered through a 0.22 μm microporous filter membrane before use.
The mass ratio of the amino lipid compounds to the ovalbumin mRNA (OVA mRNA) in the obtained lipid nanoparticles was about 10:1.

Animal preparation:

6-week-old female BALB/c mice weighed about 20 g were selected and fed in a SPF-grade feeding room. Animal tests were conducted strictly according to the guidelines of National Health institutions and the requirements of animal ethics.

In vivo delivery:

3 mice were assigned randomly to each group and injected intramuscularly into the leg with the lipid nanoparticles at a dosage of 0.5 mg/kg (Day 0). After 7 days, one booster injection was performed at the same dosage (Day 7). On Day 21, blood was taken from the tail vein for serological analysis. MC3 was used as a control.

Enzyme-linked immunosorbent assay (ELISA):

A flat-bottomed 96-well plate (Nunc) was pre-coated with OVA protein at a concentration of 0.5 μg protein per well in a 50 mM carbonate buffer (pH 9.6) at 4 °C, and allowed to stay overnight, and then blocked with 5% glycine. Antiserum obtained from the immunized animals was diluted from 102 to 106 using PBS-0.05% Tween (PBS-T) at pH 7.4, and added to the wells and incubated at room temperature and held at 37 °C for 1 h. Horseradish peroxidase (HRP)-conjugated goat anti-mouse IgG was used for labeling in PBS-T-1% BSA at a dilution ratio of 1:10,000. After addition of the HRP substrate, the absorbance at 450 nm was measured on an ELISA plate reader (Bio-Rad).
The test results are shown in FIG. 1, the IgG antibody titers generated from Lipid2 (compound 2) were comparable to those of MC3 and SM102, while the IgG antibody titers of other compounds were significantly superior to those of the control. It should be pointed out that the protein expression level of Lipid7 (compound 7) was similar to that of SM102, but with a stronger immune response.
It can be seen that the amino lipid compounds provided by the present disclosure have excellent immune activities, and possess strong adjuvant effects at the same time.
Test Example 3: Evaluation of the performances of lipid nanoparticles prepared from the amino lipid compounds of the present disclosure in in vivo delivery of influenza mRNA vaccine and immunity

Formulation method:

The amino lipid compounds of formula (I) of the present disclosure were mixed with DSPC, cholesterol, and PEG2000-DMG in a molar ratio of 45:10:42.5:2.5 and dissolved in absolute ethanol. The mRNA expressing influenza was dissolved in a sodium acetate solution (50 mM, pH = 4.0). By using a microinjection pump, the resultant ethanol solution and the sodium acetate solution (50 mM, pH = 4.0) were mixed in a ratio of 1:3 in a microfluidic chip to prepare a crude solution of lipid nanoparticles, which were then dialyzed in 1X PBS at a controlled temperature of 4°C for 6 h using a dialysis box (Fisher, MWCO 20,000), and filtered through a 0.22 μm microporous filter membrane before use.
The mass ratio of the amino lipid compounds to the influenza mRNA (OVA mRNA)in the obtained lipid nanoparticles was about 10:1.

Animal preparation:

6-week-old female BALB/c mice weighed about 20 g were selected and fed in a SPF-grade feeding room. Animal tests were conducted strictly according to the guidelines of National Health institutions and the requirements of animal ethics.

In vivo delivery:

3 mice were assigned randomly to each group and injected subcutaneously on the back with the lipid nanoparticles at a dosage of 0.5 mg/kg (Day 0). After 7 days, one booster injection was performed at the same dosage (Day 7). On Day 21, blood was taken from the tail vein for serological analysis. MC3 was used as a control.

Enzyme-linked immunosorbent assay (ELISA): The measurement method was the same as that in test example 2.

The test results are as shown in FIG. 2, wherein the MC3 control group had the lowest IgG antibody titers, the Lipid10 (compound 10) generated IgG antibody titers comparable to those of SM102, while the IgG antibody titers of other compounds were significantly superior to those of the control, which also demonstrated that the amino lipid compounds provided by the present disclosure have excellent immune activities, and possess strong adjuvant effects at the same time.

Test Example 4: Evaluation of stability performances of lipid nanoparticles prepared from the amino lipid compounds of the present disclosure using formulation method 1

Preparation of lipid nanoparticles: The formulation method 1 was used to prepare LNPs for subcutaneous administration.

Characterization of lipid nanoparticles: The particle size and PDI of the prepared lipid nanoparticles were measured by Nano-ZSZEN 3600 (Malvern). 40 μL of the LNP solution was taken for particle size measurement with circulation for three times, 30 seconds for each circulation.

The particle size measurements were carried out on the day of preparation (week 0), and after one week (week 1), two weeks (week 2), and four weeks (week 4) of storage at 25°C, and the expression intensities of Fluc mRNA delivered by subcutaneous administration were determined on the day of preparation (week 0) and after four weeks (week 4). The measurement results are as shown in Table 5.

Table 5: DLS characterization and subcutaneous expression of Fluc-mRNA of LNPs prepared from representative amino lipid compounds of the present disclosure

| | No. of Amino lipid compound | week 0 | | | week 1 | | week 2 | | week 4 | | |
|---|---|---|---|---|---|---|---|---|---|---|---|
| | | **Z-Average (d.nm)** | **PDI** | **Flue** Expression intensities | **Z-Average (d.nm)** | **PDI** | **Z-Average (d.nm)** | **PDI** | **Z-Average (d.nm)** | **PDI** | **Fluc** Expression intensities |
| **1** | **5** | **105** | **0.05** | **1.4E+07** | **110** | **0.05** | **110** | **0.06** | **115** | **0.07** | **1.1E+07** |
| **2** | **6** | **110** | **0.02** | **3.7E+08** | **110** | **0.05** | **125** | **0.04** | **125** | **0.06** | **3.6E+08** |
| **3** | **7** | **95** | **0.03** | **2.9E+08** | **105** | **0.05** | **115** | **0.06** | **120** | **0.05** | **2.6E+08** |
| **4** | **9** | **115** | **0.02** | **4.2E+07** | **120** | **0.03** | **125** | **0.06** | **125** | **0.08** | **3.1E+07** |
| **5** | **SM102** | **120** | **0.05** | **2.4E+07** | **145** | **0.16** | **195** | **0.21** | **245** | **0.28** | **3.6E+06** |

As can be seen from Table 5, at the test temperature of 25°C, in terms of particle size, all of the representative compounds 5, 6, 7, 9 of the present disclosure and SM102 had relatively uniform nanoparticles with a particle size of about 100 nm and a PDI of less than 0.1 on the preparation day (week 0), and had increased particle size after four weeks of storage, wherein the particle size of SM102 increased by more than one time, from 120 to 245, while the particle sizes of the representative compounds 5, 6, 7 and 9 of the present disclosure did not increase significantly; in terms of PDI, after four weeks, the PDIs of all the representative compounds 5, 6, 7 and 9 of the present disclosure were less than 0.1, while the PDI of SM102 increased from 0.05 on the preparation day (week 0) to 0.28 (week 4); in terms of Fluc expression, after four weeks, the Fluc expression of the representative compounds 5, 6, 7, and 9 of the present disclosure did not decrease significantly, while the Fluc expression of SM102 decreased significantly, from 2.1E+07 to 3.6E+06. It can be seen that the amino lipid compounds of the present disclosure have excellent stability.

Test Example 5: Evaluation of the activity performances of lipid nanoparticles prepared from the amino lipid compounds of the present disclosure using the formulation method 2

Preparation of lipid nanoparticles: LNPs were prepared using the formulation method 2 for intramuscular administration.

The measurement method was the same as that of Test Example 1. The measurement results are as shown in Table 6.

Table 6: Expression intensity of Fluc mRNA delivered by intramuscular administration of representative amino lipid compounds of the present disclosure

| Compound No. | Structure | Fluorescence intensity |
|---|---|---|
| Compound 4 | 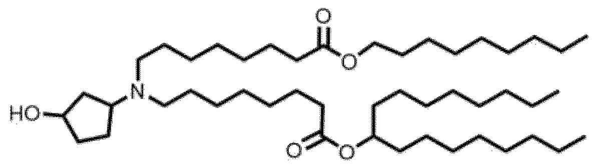 | 6.7E+06 |
| Comparative compound 1 | 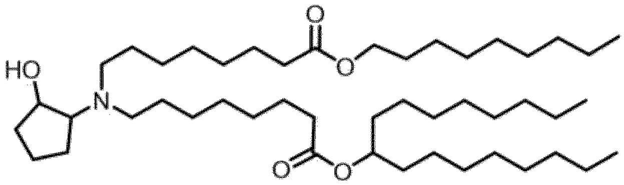 | 1.7E+05 |
| Compound 6 | 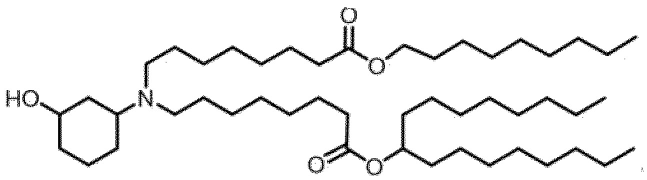 | 1.2E+08 |
| Comparative compound 2 | 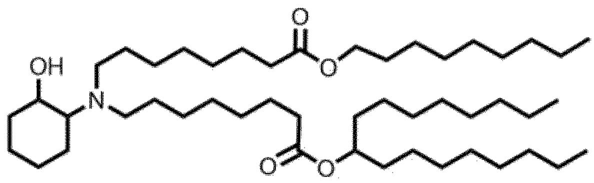 | 2.8E+06 |
| Comparative compound 6 | 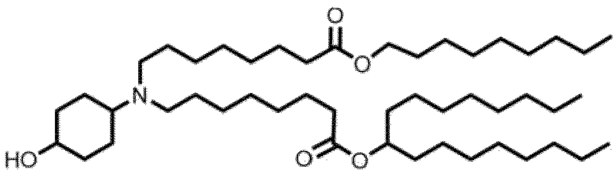 | 1.9E+07 |
| Compound 7 | 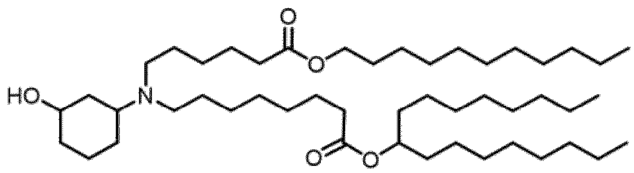 | 3.7E+07 |

(continued)

| Compound No. | Structure | Fluorescence intensity |
|---|---|---|
| Comparative compound 3 | 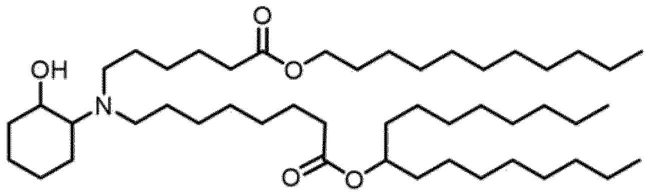 | 2.9E+05 |
| Comparative compound 7 | 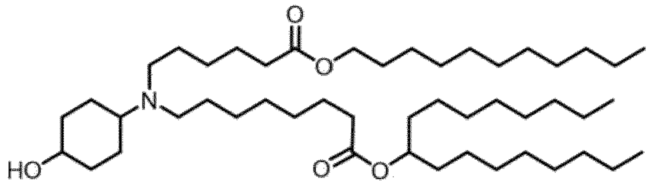 | 4.5E+06 |
| Compound 8 | 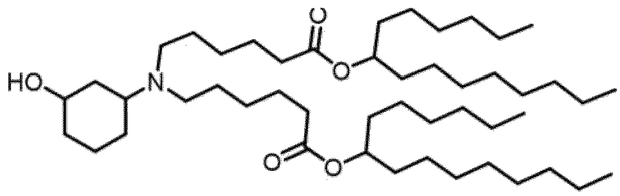 | 9.2E+06 |
| Comparative compound 4 | 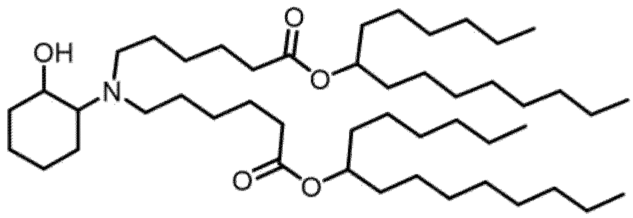 | 1.3E+05 |
| Comparative compound 8 | 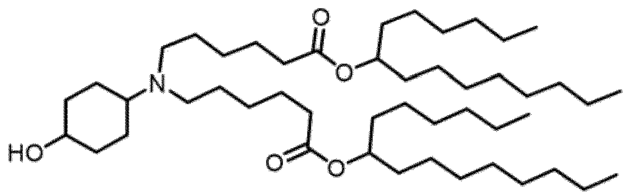 | 2.1E+06 |

It can be seen from Table 6 that, in the case that the other structural units are same, the compounds where the hydroxyl on the cycloalkyl structural unit is located at the meta position of the amine substituent has higher expression intensity of Fluc mRNA than those where the hydroxyl on the cycloalkyl structural unit is located at other substitution positions of the amine substituent. Specifically, for example, the expression intensity of Fluc mRNA of the representative compound 6 of the present disclosure was 1.2E+08; in contrast, the expression intensity of Fluc mRNA of the comparative compound 2, which differs from the representative compound 6 of the present disclosure only in that the hydroxyl on the cycloalkyl structural unit is located at the adjacent position of the amine substituent, is 2.8E+06; further, the expression intensity of Fluc mRNA of the comparative compound 6, which differs from the representative compound 6 of the present disclosure only in that the hydroxyl on the cycloalkyl structural unit is located at the position two carbon atom away from the amine substituent, was 1.9E+07. The same conclusion can be seen, for example, between the representative compound 7 of the present disclosure and the comparative compounds 3 and 7, between the representative compound 8 of the present disclosure and the comparative compounds 4 and 8, etc. It can be seen that, in the case that the other structural units are same, the lipid nanoparticles containing amino lipid compounds where the substituent on the cycloalkyl structural unit is located at the meta position of the amine substituent have the highest activity performance.

Test Example 6: Targeting Assay of Amino Lipid Compounds

Formulation method of mRNA lipid nanoparticle (LNP) preparations:

The lipids were mixed with DSPC, CHO-HP, M-DMG2000 (i.e. PEG2000-DMG) in a molar ratio of 50:10:38.5:1.5 and dissolved in absolute ethanol. mRNA was dissolved in a sodium acetate solution (0.2M, pH = 5.0). The samples of various prescriptions were prepared at a water phase: alcohol phase = 9 ml/min: 3 ml/min by using a microfluidic equipment (MPE-L2) and a chip (SN.000035). The two phases were mixed by injecting them into the microfluidic chip according to the interface requirements, and then the drug solution was loaded into a 100KD dialysis bags, immersed in a beaker containing 1L dialysis solution and wrapped with aluminum foil, and dialyzed at 100rpm for 1h at room temperature, and then the dialysis solution was replaced and the dialysis continued for 1 h.

Formulation of dialysis solution: 1×PBS + 8% sucrose solution: 2 packets of 1×PBS powder were taken into a beaker, dissolved with 2L DEPC water, added with 160g of sucrose additionally, and mixed to obtain the 1×PBS + 8% sucrose solution.

The mass ratio of mRNA : Lipids in the obtained lipid nanoparticles were about 1:10.

Fluorescent protein-encoding mRNA-LNP preparations containing Compound (I-6-II), MC3 or SM102, respectively were prepared by using the formulation method of mRNA lipid nanoparticle (LNP) preparations as described above, selecting a fluorescent protein-encoding mRNA as the mRNA and the compound (I-6-II), MC3 or SM102, respectively as the lipid.

Test method:

Each preparation to be tested was administered to mice by intramuscular injection, with each animal receiving 15μg mRNA preparation sample. 6h after sample injection, the mice were anesthetized by isoflurane inhalation, and injected intraperitoneally with 200 μL of D-Luciferin (concentration 10mg/ml) luciferase development substrate. The animals were placed in a supine position and observed the signal distribution and expression intensity of Luciferase in vivo in the mice under an IVIS living imaging system 10min after substrate injection. After development of living imaging of the intramuscularly injected animals, they were immediately dissected, followed by observation of the fluorescence in the liver immediately after the dissection.

The specific fluorescence intensity values are as follows:

Fluorescent protein-encoding mRNA-LNP preparation containing MC3: 1.23E+05
Fluorescent protein-encoding mRNA-LNP preparation containing SM102: 3.23E+06
Fluorescent protein-encoding mRNA-LNP preparation containing Compound (I-6-II): 2.49E+05

The test results are as shown in FIG. 8. When administered via intramuscular injection, the compound (I-6-II) group showed significantly lower liver fluorescence intensity than SM102, indicating a relatively low distribution of fluorescent protein-encoding mRNA in the important organ liver, and thus showed good local targeting effect and effectively reduced the risk of liver toxicity.

Test Example 7 Adverse reaction assay of amino lipid compounds by intramuscular administration

Formulation of Covid-19 S protein-encoding mRNA-LNP preparation:

Covid-19 S protein-encoding mRNA-LNP preparations containing Compound 6, Compound (I-6-II), ALC-0315 or SM102, respectively were prepared by using the formulation method of mRNA lipid nanoparticle (LNP) preparations in test example 6, selecting a Covid-19 S protein-encoding mRNA as the mRNA and the Compound 6, Compound (I-6-II), ALC-0315 or SM102, respectively as the lipid.

The sequence of the Covid-19 S protein-encoding mRNA was a sequence obtained by replacing all uracil (u) in SEQ ID NO. 1 with N1-methylpseudouridine. It should be noted that, the t (thymine) in the RNA sequence SEQ ID NO. 1 in the sequence listings is actually u (uracil) according to the WIPO standard ST.26 for nucleotide and amino acid sequence listings.

Test method:

SD rats were randomly assigned into 4 groups, half male and half female, i.e., Compound 6 high-dose group (100 μg mRNA/animal), Compound (I-6-II) high-dose group (100 μg mRNA/animal), ALC- 0315 high-dose group (the cationic lipid in Pfizer vaccine BNT162-b2) (100 μg mRNA/animal), SM-102 high-dose group (the cationic lipid in Moderna vaccine mRNA-1273) (100 μg mRNA/animal), with 3 animals per gender in each group. The administration was carried out by intramuscular injection once a week for 3 consecutive weeks. Swelling and other abnormalities at the injection site were

observed clinically.

The test results are as shown in FIG. 9. It can be seen from FIG. 9 that, compared with the cationic lipid components of the marketed mRNA vaccines at the same level, Compound (I-6-II) had significantly reduced or decreased severe swelling at the injection site and significantly reduced or decreased moderate claudication, and thus can be considered as having a higher safety.

Test Example 8 Toxicokinetic

Formulation of empty liposome preparation:

1.9078g of Compound I-6-II, 0.3985g of DSPC, 0.7575g of CHO-HP and 0.1972g of PEG-DMG (average molecular weight of 2000) were weighed in the molar ratio of 49.5:10:39:1.5, dissolved in absolute ethanol and diluted to 237.5mL to obtain an alcohol phase. The alcohol phase was mixed and encapsulated by using a microfluidic device (MPE-L2), with an acetic acid-sodium acetate buffer (0.2mol/L, pH=5) as the aqueous phase, and a volume ratio of aqueous phase: alcohol phase of 3:1. The drug solution after encapsulation was replaced by ultrafiltration at a TMP of 0.2 bar and a flow rate of 300 ml/min using a dialysis solution containing 8 mg/ml sodium chloride, 0.2 mg/ml potassium chloride, 0.2 mg/ml potassium dihydrogen phosphate, 1.15 mg/ml disodium hydrogen phosphate dihydrate and 80 mg/ml sucrose, followed by sterilization filtration to obtain an I-6-II empty liposome preparation.

Test method:

The animal experiment was set up with a total of 5 groups (5 animals/gender/group), and the groups and the dosages thereof were as follows: negative control group (0 dosage/animal), empty liposome low-dosage group (1 dosage/animal) and empty liposome high-dosage group (4 dosages/animal). The animals in each group were administered intramuscularly once every 2 weeks for a total of 3 times, i.e., administered on D1 (the day of the first administration), D15 (Day 15 after the first administration), and D29 (Day 29 after the first administration).

The animal experiment was set up with a total of 5 groups (5 animals/gender/group), and the groups and the dosages thereof were as follows: negative control group (0μg compound I-6-II/animal), empty liposome low-dosage group (300μg compound I-6-II/animal) and empty liposome high-dosage group (1200μg compound I-6-II/animal). The animals in each group were administered intramuscularly once every 2 weeks for a total of 3 times, i.e., administered on D1, D15 and D29.

Blood samples were collected from animals before and 4 h after the first (D1) and the last (D29) administration in the negative control group; and Blood samples were collected from animals before and 15 min, 1 h, 2 h, 4 h, 8 h, 24 h, 32 h and 48 h after the first (D1) and the last (D29) administration in the empty liposome group. The plasma concentration of compound I-6-II in cynomolgus monkeys was detected to investigate the exposure of compound I-6-II in cynomolgus monkeys after administration in each group of animals.

The main TK parameters of compound I-6-II in animals of the empty liposome low- and high-dosage groups after the first administration (D1) and the last administration (D29) are as shown in Table 7 below.

Table 7 Main TK parameters of compound I-6-II in animals of empty liposome low- and high-dosage groups

| groups | Day on administration | gender | parameters | $T_{max}$ (h) | $C_{max}$ (ng/mL) | $AUC_{last}$ (h·ng/mL) | $t_{1/2}$ (h) |
|---|---|---|---|---|---|---|---|
| empty liposome low-dosage group | D1 | male | Mean | 4.80 | 50.65 | 652.31 | 5.10 |
| | | | SD | 1.79 | 7.88 | 126.61 | 0.88 |
| | | female | Mean | 3.20 | 79.06 | 837.50 | 5.28 |
| | | | SD | 1.10 | 27.18 | 214.11 | 1.26 |
| | D29 | male | Mean | 7.20 | 42.39 | 623.24 | 5.59 |
| | | | SD | 1.79 | 17.90 | 211.52 | 2.62 |
| | | female | Mean | 6.00 | 59.26 | 783.22 | 5.58 |
| | | | SD | 2.83 | 17.51 | 158.41 | 1.65 |

(continued)

| groups | Day on administration | gender | parameters | $T_{max}$ (h) | $C_{max}$ (ng/mL) | $AUC_{last}$ (h·ng/mL) | $t_{1/2}$ (h) |
|---|---|---|---|---|---|---|---|
| empty liposome high-dosage group | D1 | male | Mean | 3.20 | 198.24 | 2240.75 | 6.07 |
| | | | SD | 1.10 | 100.32 | 1133.04 | 0.14 |
| | | female | Mean | 5.00 | 333.54 | 4568.49 | 5.07 |
| | | | SD | 3.00 | 148.18 | 1430.91 | 0.43 |
| | D29 | male | Mean | 8.00 | 146.37 | 2280.44 | 6.45 |
| | | | SD | 0.00 | 41.08 | 670.99 | 1.38 |
| | | female | Mean | 8.00 | 193.68 | 3089.27 | 5.29 |
| | | | SD | 0.00 | 31.77 | 465.33 | 0.24 |

The experimental data revealed that:
No I-6-II liposome was detected in the first (D1) and the last (D29) plasma samples of animals in the negative control group.
As can be seen from Table 7, compound I-6-II can be rapidly cleared in vivo from cynomolgus monkeys, with a plasma half-life of 4.68-6.45 h; comparing the first (D1) and the last (D29) administrations in animals, the $C_{max}$ ratios (D29/D1) of compound I-6-II in empty liposome groups were between 0.58 and 0.84, and the $AUC_{last}$ ratios (D29/D1) were between 0.68 and 1.02, indicating that after 4 weeks of continuous administration (3 times in total), compound I-6-II did not accumulate in cynomolgus monkeys.
In summary, the amino lipid compound having a cycloalkyl structural unit and an additional substituent at the meta position of the amine substituent of the cycloalkyl provided by the present disclosure can be used to deliver bioactive agents into cells. Compared with the amino lipid compounds with similar structures known in the prior art, the amino lipid compound of the present disclosure has one or more of the following advantages: good delivery ability, good stability and high safety, and can be used to deliver bioactive agents (such as nucleic acids) into cells with increased protein expression levels to more effectively induce the body to produce an immune response; and it has excellent targeting ability, and can be stored, transported and used at room temperature.

List of abbreviations

| | |
|---|---|
| DIPEA | N,N-diisopropylethylamine |
| DNA | Deoxyribonucleic acid |
| RNA | Ribonucleic acid |
| DOPE | Dioleoyl phosphatidyl ethanolamine |
| DSPC | Distearoylphosphatidylcholine |
| PEG2000-DMG | 1,2-dimyristoyl-rac-glycerol-3-methoxypolyethylene glycol 2000 |
| kD | kilodalton |
| PBS | Phosphate buffered saline. |

It is obvious to those skilled in the art that the present disclosure is not limited to the above illustrative embodiments, and may be implemented in other specific embodiments without departing from the substantive feature of the present disclosure. Therefore, it is intended that these embodiments are considered illustrative and non-restrictive in all respects, and reference should be made to the appended claims rather than to the above embodiments, and all changes within the equivalent meaning and scope of the claims should be included therein.

## Claims

1. A compound represented by the following structural formula I or a pharmaceutically acceptable salt or stereoisomer thereof:

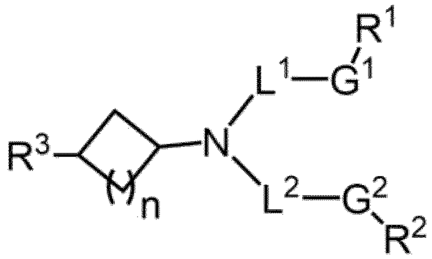

(I)

wherein:

$L^1$ and $L^2$ are identical or different, and each independently selected from the group consisting of $C_1$-$C_{12}$ alkylene, $C_2$-$C_{12}$ alkenylene and $C_2$-$C_{12}$ alkynylene; preferably, $L^1$ and $L^2$ are identical or different, and each independently selected from the group consisting of $C_3$-$C_{10}$ alkylene, $C_3$-$C_{10}$ alkenylene and $C_3$-$C_{10}$ alkynylene; further preferably, $L^1$ and $L^2$ are identical or different, and each independently selected from the group consisting of $C_3$-$C_{10}$ alkylene; most preferably, $L^1$ and $L^2$ are identical or different, and each independently selected from the group consisting of $C_5$-$C_8$ alkylene;

$G^1$ and $G^2$ are identical or different, and each independently selected from the group consisting of -O-(C=O)-, -(C=O)-O-, -C(=O)-, -O-, -C(=O)-S-, and -S-C(=O)-; preferably, $G^1$ and $G^2$ are identical or different, and each independently selected from the group consisting of -O-(C=O)-, -(C=O)-O-, -C(=O)-, and -O-; most preferably, $G^1$ and $G^2$ are identical or different, and each independently selected from the group consisting of -O-(C=O)-, and -(C=O)-O-;

$R^1$ and $R^2$ are identical or different, and each independently selected from the group consisting of $C_5$-$C_{27}$ alkyl, and $C_5$-$C_{27}$ alkenyl comprising one or more double bonds, which are attached via any one carbon thereof; preferably, $R^1$ and $R^2$ are identical or different, and each independently selected from the group consisting of $C_8$-$C_{20}$ alkyl, and $C_8$-$C_{20}$ alkenyl comprising one or more double bonds, which are attached via any one carbon thereof; further preferably, $R^1$ and $R^2$ are identical or different, and each independently selected from the group consisting of $C_9$-$C_{17}$ alkyl, and $C_9$-$C_{18}$ alkenyl comprising one or two double bonds, which are attached via any one carbon thereof; most preferably, $R^1$ and $R^2$ are identical or different, and each independently selected from the group consisting of

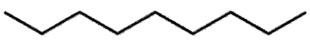
,
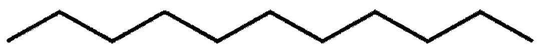
,

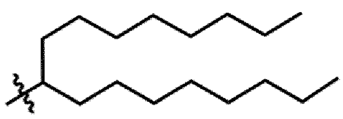
,
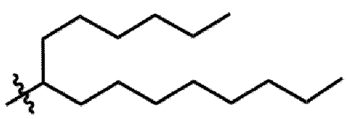
,

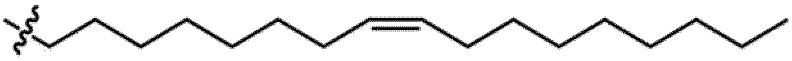
,

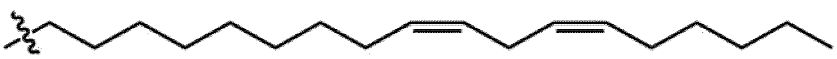
,

and

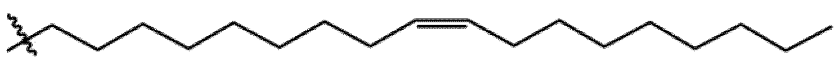
;

$R^3$ is selected from the group consisting of halogen, hydroxy, cyano, $C_1$-$C_6$ alkyl, nitro, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino; preferably, $R^3$ is selected from the group consisting of halogen, hydroxy, cyano, $C_1$-$C_6$ alkyloxy, $C_1$-$C_6$ alkylcarbonyloxy,

$C_1$-$C_6$ alkyloxycarbonyl, $C_1$-$C_6$ alkylaminocarbonyl, and $C_1$-$C_6$ alkylcarbonylamino; further preferably, $R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl, and $C_1$-$C_4$ alkylcarbonylamino; most preferably, $R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

2. The compound according to claim 1 or a pharmaceutically acceptable salt or stereoisomer thereof, wherein:

$L^1$ and $L^2$ are identical or different, and each independently selected from the group consisting of $C_3$-$C_{10}$ alkylene, $C_3$-$C_{10}$ alkenylene and $C_3$-$C_{10}$ alkynylene;
$G^1$ and $G^2$ are identical or different, and each independently selected from the group consisting of -O-(C=O)-, -(C=O)-O-, -C(=O)-, and -O-;
$R^1$ and $R^2$ are identical or different, and each independently selected from the group consisting of $C_8$-$C_{20}$ alkyl, and $C_8$-$C_{20}$ alkenyl comprising one or more double bonds, which are attached via any one carbon thereof;
$R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino;
n is selected from the group consisting of 1, 2, and 3.

3. The compound according to claim 1 or a pharmaceutically acceptable salt or stereoisomer thereof, wherein:

$L^1$ and $L^2$ are identical or different, and each independently selected from the group consisting of $C_3$-$C_{10}$ alkylene;
$G^1$ and $G^2$ are identical or different, and each independently selected from the group consisting of -O-(C=O)-, and -(C=O)-O-;
$R^1$ and $R^2$ are identical or different, and each independently selected from the group consisting of $C_9$-$C_{17}$ alkyl, and $C_9$-$C_{18}$ alkenyl comprising one or two double bonds, which are attached via any one carbon thereof;
$R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl, and $C_1$-$C_4$ alkylcarbonylamino;
n is selected from the group consisting of 1, 2, and 3.

4. The compound according to claim 1 or a pharmaceutically acceptable salt or stereoisomer thereof, wherein:

$L^1$ and $L^2$ are identical or different, and each independently selected from the group consisting of $C_5$-$C_8$ alkylene;
$G^1$ and $G^2$ are identical or different, and each independently selected from the group consisting of -O-(C=O)-, and -(C=O)-O-;
$R^1$ and $R^2$ are identical or different, and each independently selected from the group consisting of

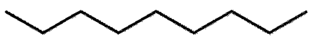 , 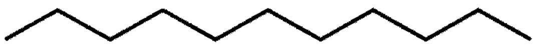 ,

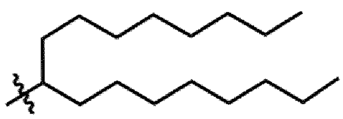 , 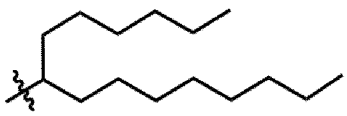 ,

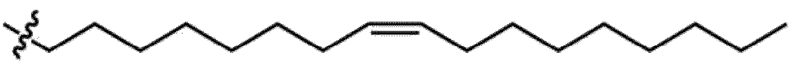 ,

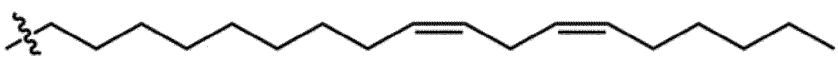 ,

and

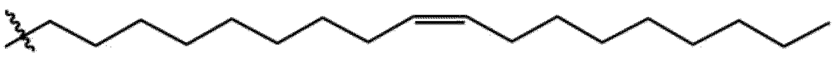 ;

$R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

**5.** The compound according to claim 1 or a pharmaceutically acceptable salt or stereoisomer thereof, wherein said compound is selected from the group consisting of:

| No. | Structure |
|---|---|
| 1 | 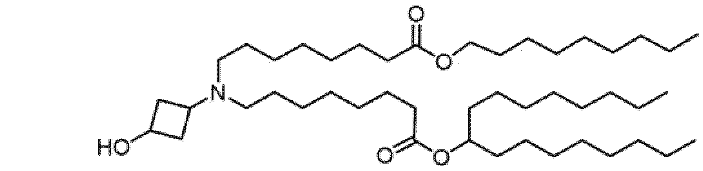 |
| 2 | 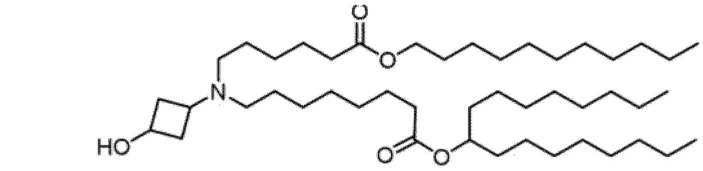 |
| 4 | 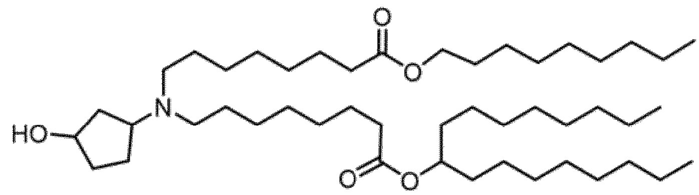 |
| 5 | 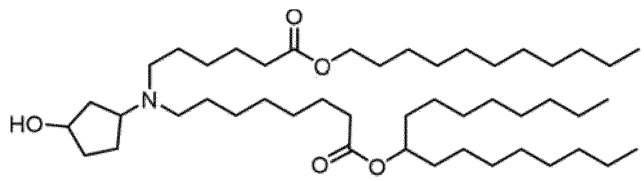 |
| 6 | 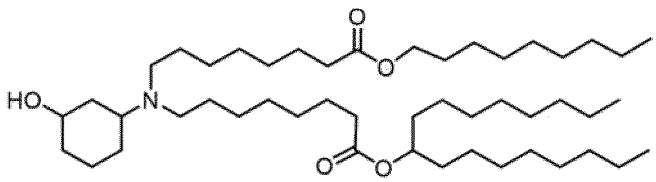 |
| 7 | 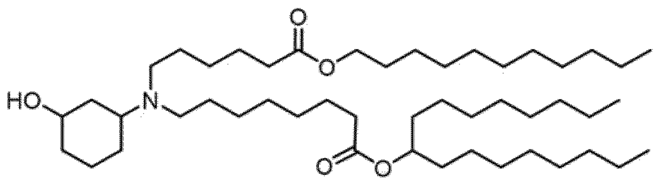 |
| 8 | 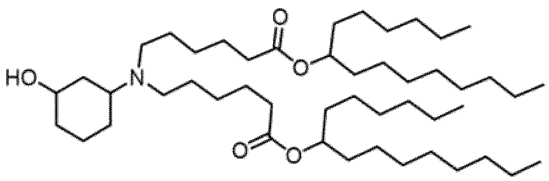 |

(continued)

| No. | Structure |
|---|---|
| 9 | 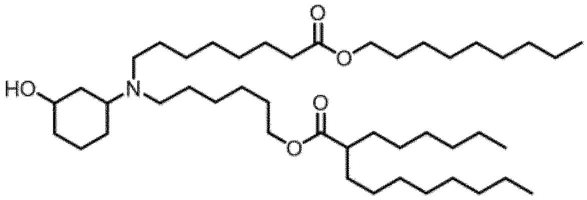 |
| 10 | 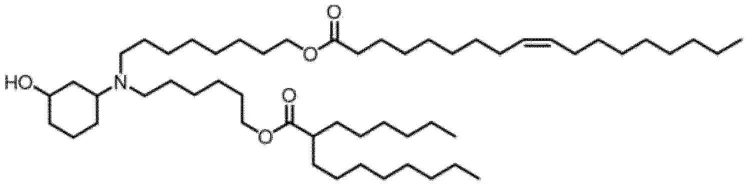 |
| 11 | 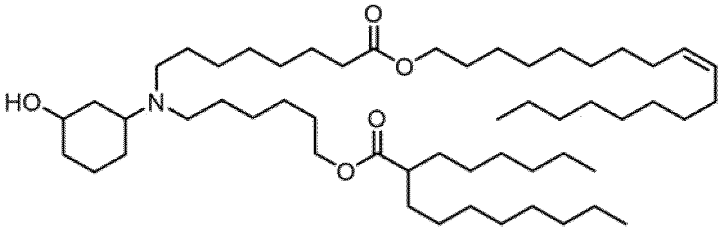 |
| 12 | 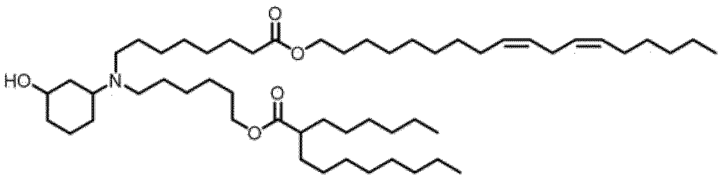 |
| 14 | 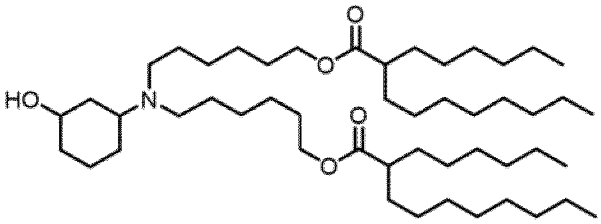 |
| 15 | 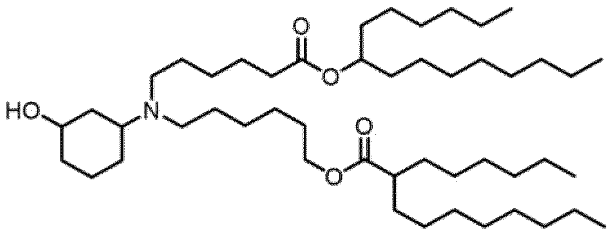 |
| 16 | 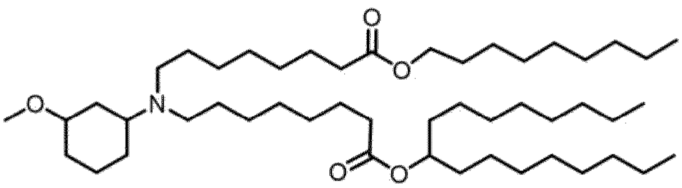 |

(continued)

| No. | Structure |
|---|---|
| 17 | 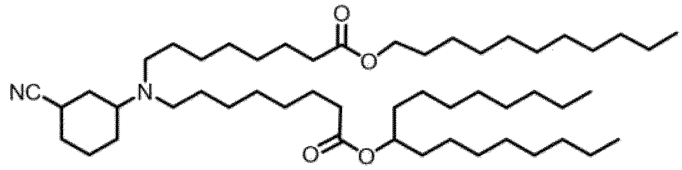 |
| 18 | 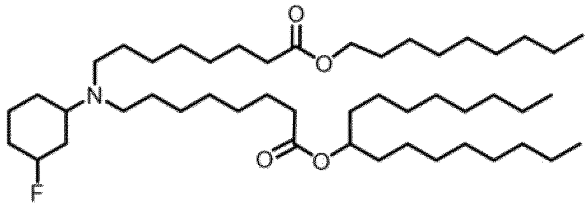 |
| 19 | 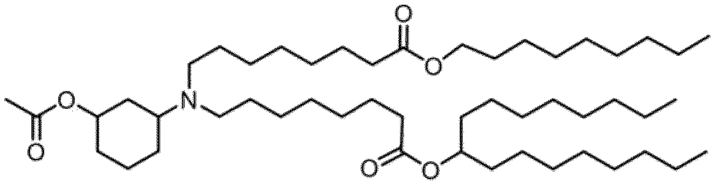 |
| 20 | 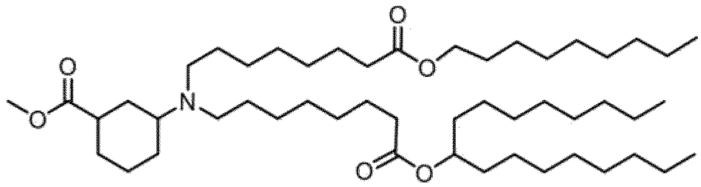 |
| 21 | 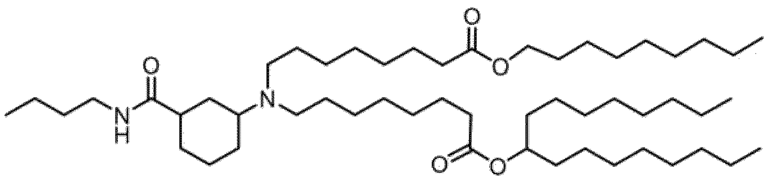 |
| 22 | 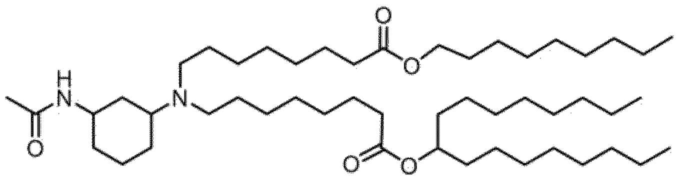 |
| 25 | 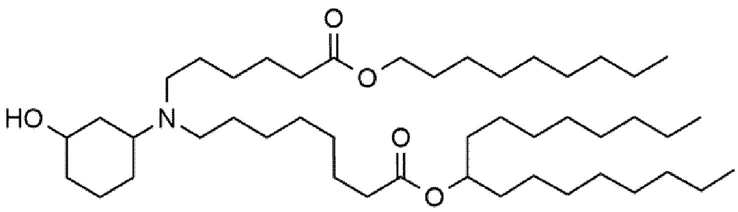 |
| 26 | 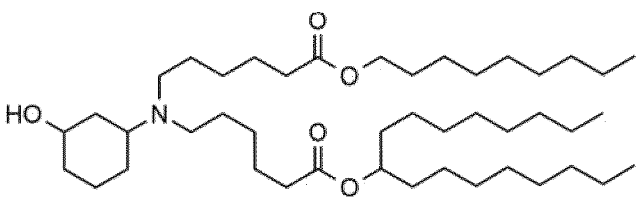 |

(continued)

| No. | Structure |
|---|---|
| 27 | 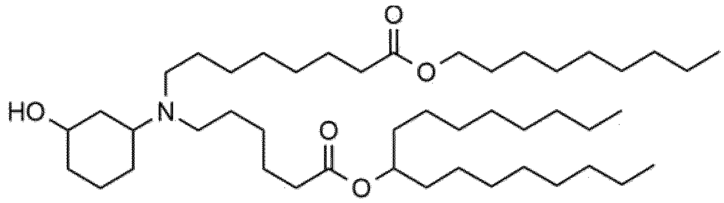 |

.

**6.** A compound having a structure selected from the following structures, or an optical isomer thereof or a pharmaceutically acceptable salt thereof:

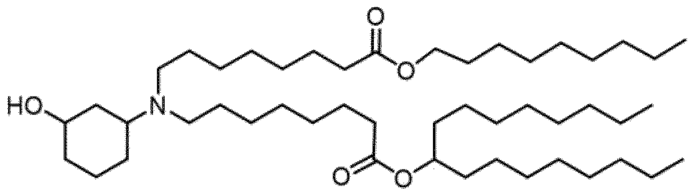

,

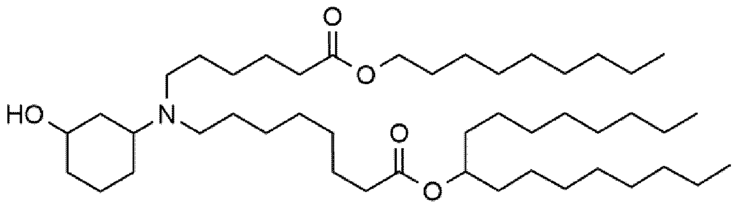

,

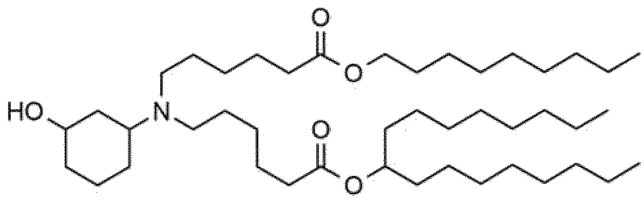

,

and

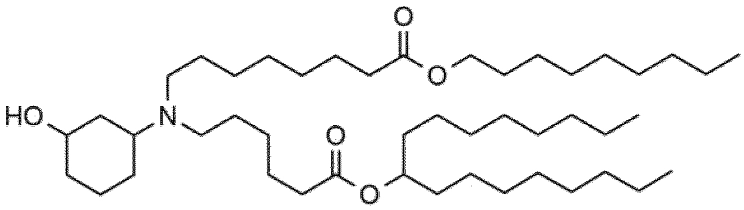

.

**7.** A compound having a structure selected from the following structures, or a pharmaceutically acceptable salt thereof:

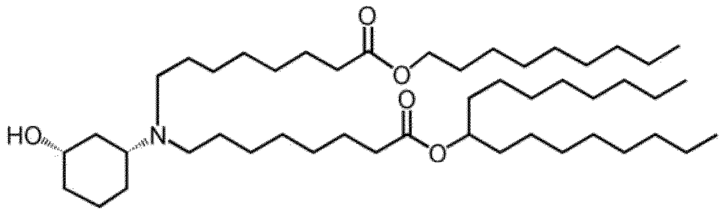

(I-6-II) ,

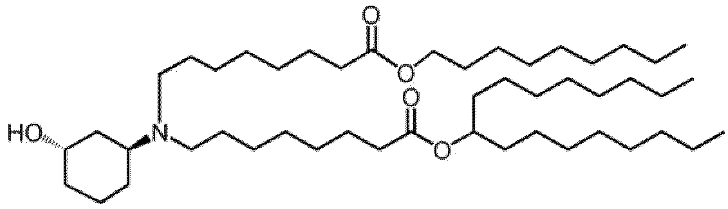

(I-6-III) ,

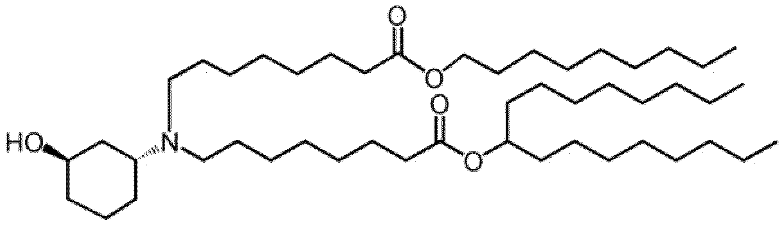

(I-6-IV) ,

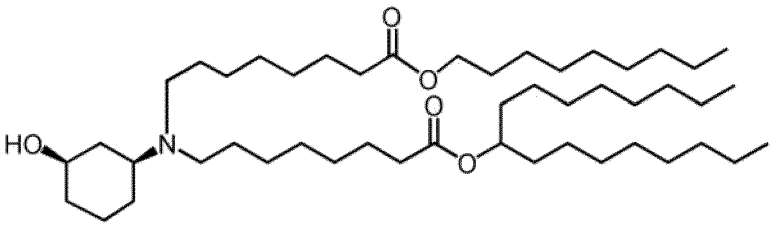

(I-6-V) ,

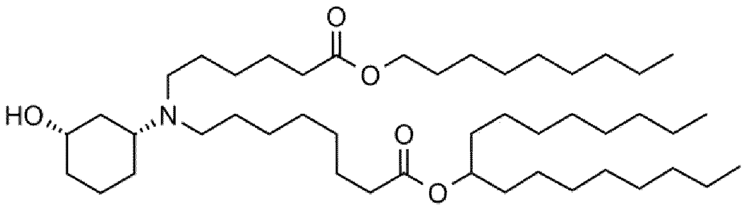

(I-25-II) ,

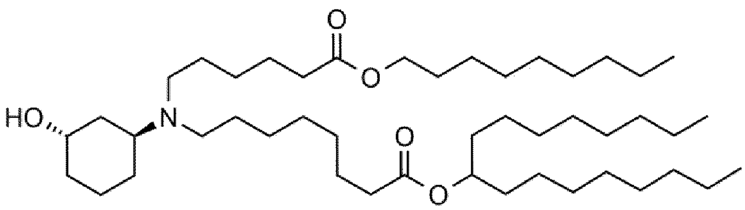

(I-25-III) ,

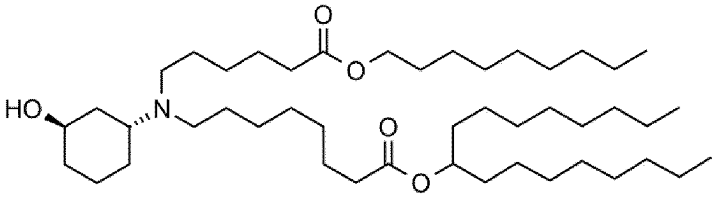

(I-25-IV) ,

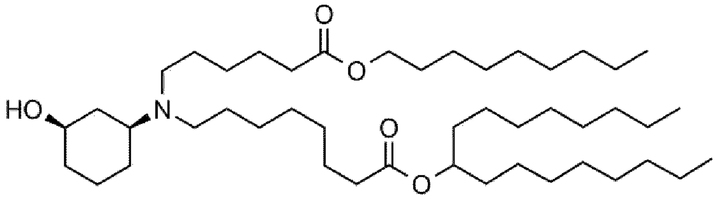

(I-25-V) ,

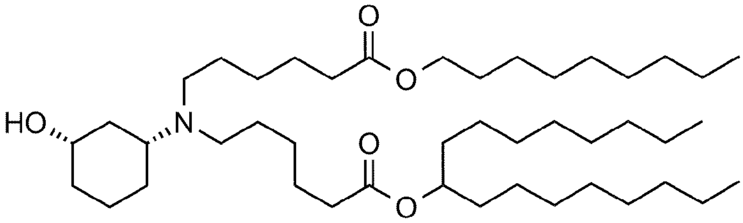

(I-26-II) ,

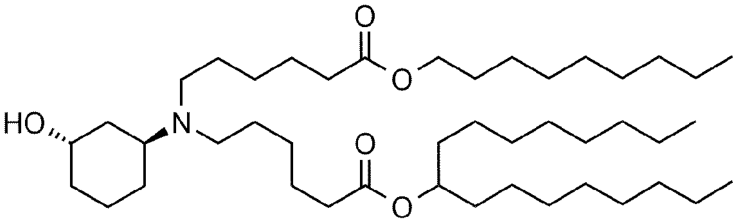

(I-26-III) ,

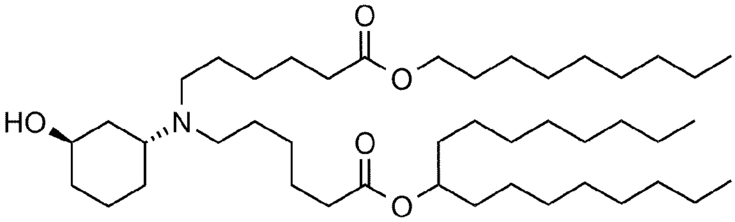

(I-26-IV) ,

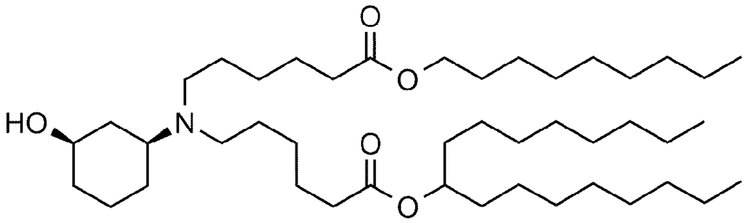

(I-26-V) ,

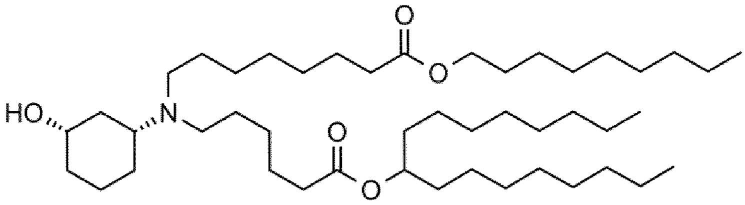

(I-27-II) ,

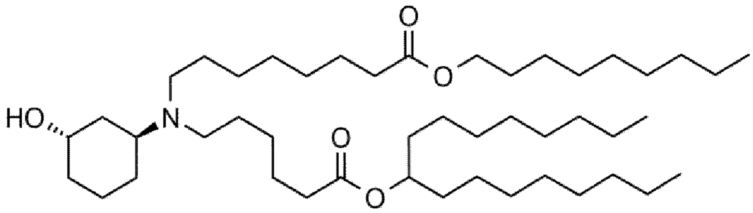

(I-27-III) ,

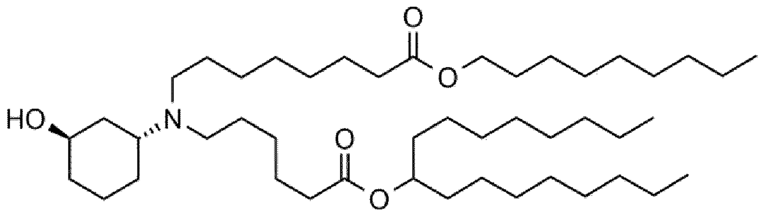

(I-27-IV) ,

and

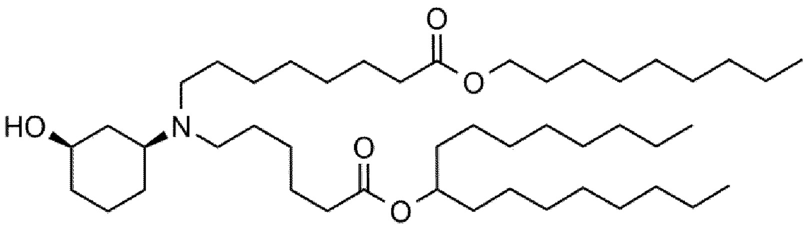

(I-27-V) .

**8.** A compound having a structure selected from the following structures, or an optical isomer thereof or a pharmaceutically acceptable salt thereof:

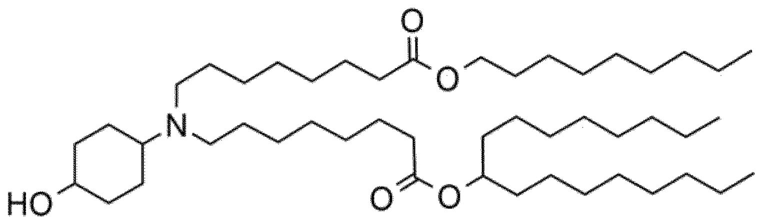

,

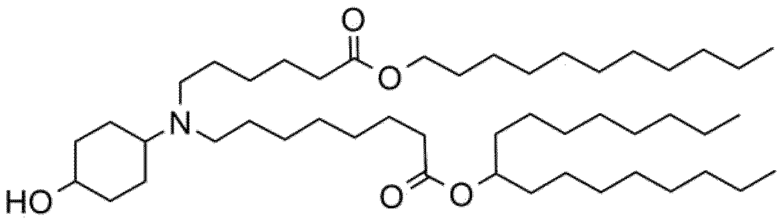

,

and

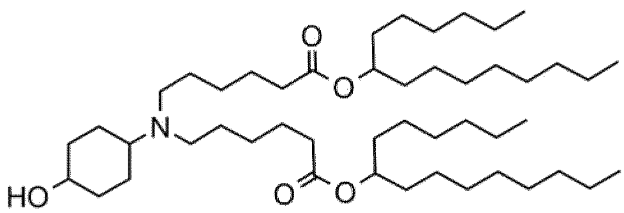

.

**9.** A lipid nanoparticle comprising the compound according to any one of claims 1-8 or a pharmaceutically acceptable salt or stereoisomer thereof.

**10.** A pharmaceutical composition comprising the compound according to any one of claims 1-8 or a pharmaceutically acceptable salt or stereoisomer thereof and a pharmaceutically acceptable carrier, diluent or excipient.

**11.** Use of the compound according to any one of claims 1-8 or a pharmaceutically acceptable salt or stereoisomer thereof, the lipid nanoparticle according to claim 9 or the pharmaceutical composition according to claim 10 in the manufacture of a medicament, wherein said medicament is used in gene therapy, genetic vaccination, antisense therapy or therapy by interfering RNA.

**12.** The use according to claim 11, wherein the gene therapy is used for the treatment of a cancer or a genetic disease.

**13.** The use according to claim 12, wherein the cancer is selected from one or more of lung cancer, stomach cancer, liver cancer, esophagus cancer, colon cancer, pancreatic cancer, brain cancer, lymphatic cancer, blood cancer and prostatic cancer; the genetic disease is selected from one or more of hemophilia, thalassemia and Gaucher's disease.

**14.** The use according to claim 11, wherein the genetic vaccination is used for the treatment of cancer, allergy, toxicity or infection by a pathogen.

**15.** The use according to claim 14, wherein the pathogen is selected from one or more of virus, bacteria and fungi.

**16.** Use of the lipid nanoparticle according to claim 9 or the compound according to any one of claims 1-8 or a pharmaceutically acceptable salt or stereoisomer thereof in the manufacture of a medicament for nucleic acid transfer, wherein the nucleic acid is RNA, DNA, or antisense oligonucleotide; preferably, the RNA is selected from the group consisting of messenger RNA (mRNA), ribosomal RNA (rRNA), micro RNA (miRNA), transfer RNA (tRNA), small interferingRNA (siRNA) and small nuclear RNA (snRNA); preferably, the DNA is a plasmid.

**17.** A compound represented by the following structural formula II or a pharmaceutically acceptable salt or stereoisomer thereof:

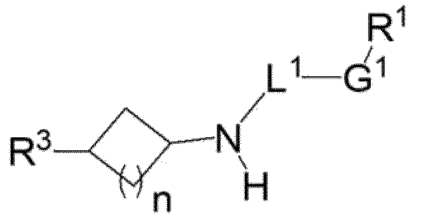

II

wherein:

$L^1$ is selected from the group consisting of $C_1$-$C_{12}$ alkylene, $C_2$-$C_{12}$ alkenylene and $C_2$-$C_{12}$ alkynylene; preferably, $L^1$ is selected from the group consisting of $C_3$-$C_{10}$ alkylene, $C_3$-$C_{10}$ alkenylene and $C_3$-$C_{10}$ alkynylene; further preferably, $L^1$ is selected from the group consisting of $C_3$-$C_{10}$ alkylene; most preferably, $L^1$ is selected from the group consisting of $C_5$-$C_8$ alkylene;

$G^1$ is selected from the group consisting of -O-(C=O)-, -(C=O)-O-, - C(=O)-, -O-, -C(=O)-S-, and -S-C(=O)-; preferably, $G^1$ is selected from the group consisting of -O-(C=O)-, -(C=O)-O-, -C(=O)-, and -O-; most preferably, $G^1$ is selected from the group consisting of -O-(C=O)-, and - (C=O)-O-;

$R^1$ is selected from the group consisting of $C_5$-$C_{27}$ alkyl, and $C_5$-$C_{27}$ alkenyl comprising one or more double bonds, which are attached via any one carbon thereof; preferably, $R^1$ is selected from the group consisting of $C_8$-$C_{20}$ alkyl, and $C_8$-$C_{20}$ alkenyl comprising one or more double bonds, which are attached via any one carbon thereof; further preferably, $R^1$ is selected from the group consisting of $C_9$-$C_{17}$ alkyl, and $C_9$-$C_{18}$ alkenyl comprising one or two double bonds, which are attached via any one carbon thereof; most preferably, $R^1$ is selected from the group consisting of

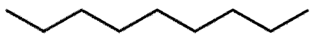 , 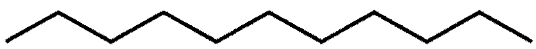 ,

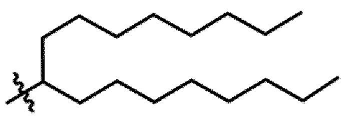 , 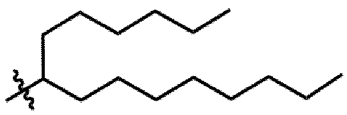 ,

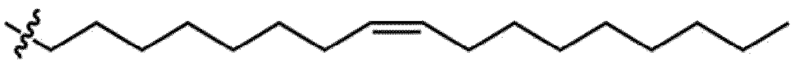 ,

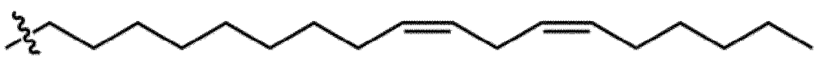 ,

and

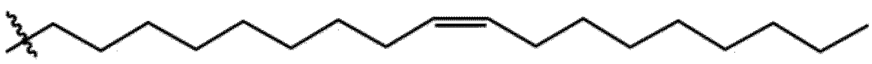 ;

$R^3$ is selected from the group consisting of halogen, hydroxy, cyano, $C_1$-$C_6$ alkyl, nitro, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino; preferably, $R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino; further preferably, $R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl, and $C_1$-$C_4$ alkylcarbonylamino; most preferably, $R^3$ is selected from the group consisting of fluoro, hydroxy, cyano, methoxy, acetoxy, methoxycarbonyl, butylaminocarbonyl and acetamido;

n is selected from the group consisting of 1, 2, and 3.

**18.** The compound according to claim 17 or a pharmaceutically acceptable salt or stereoisomer thereof, wherein:

$L^1$ is selected from the group consisting of $C_3$-$C_{10}$ alkylene, $C_3$-$C_{10}$ alkenylene and $C_3$-$C_{10}$ alkynylene;
$G^1$ is selected from the group consisting of -O-(C=O)-, -(C=O)-O-, - C(=O)-, and -O-;
$R^1$ is selected from the group consisting of $C_8$-$C_{20}$ alkyl, and $C_8$-$C_{20}$ alkenyl comprising one or more double bonds, which are attached via any one carbon thereof;
$R^3$ is selected from the group consisting of halogen, hydroxy, cyano, $C_1$-$C_6$ alkyloxy, $C_1$-$C_6$ alkylcarbonyloxy, $C_1$-$C_6$ alkyloxycarbonyl, $C_1$-$C_6$ alkylaminocarbonyl, and $C_1$-$C_6$ alkylcarbonylamino;
n is selected from the group consisting of 1, 2, and 3.

**19.** The compound according to claim 17 or a pharmaceutically acceptable salt or stereoisomer thereof, wherein:

$L^1$ is selected from the group consisting of $C_3$-$C_{10}$ alkylene;
$G^1$ is selected from the group consisting of -O-(C=O)-, and -(C=O)-O-;
$R^1$ is selected from the group consisting of $C_9$-$C_{17}$ alkyl, and $C_9$-$C_{18}$ alkenyl comprising one or two double bonds, which are attached via any one carbon thereof;
$R^3$ is selected from the group consisting of halogen, hydroxy, cyano, $C_1$-$C_4$ alkyloxy, $C_1$-$C_4$ alkylcarbonyloxy, $C_1$-$C_4$ alkyloxycarbonyl, $C_1$-$C_4$ alkylaminocarbonyl, and $C_1$-$C_4$ alkylcarbonylamino;
n is selected from the group consisting of 1, 2, and 3.

**20.** The compound according to claim 17 or a pharmaceutically acceptable salt or stereoisomer thereof, wherein:

$L^1$ is selected from the group consisting of $C_5$-$C_8$ alkylene;
$G^1$ is selected from the group consisting of -O-(C=O)-, and -(C=O)-O-;
$R^1$ is selected from the group consisting of

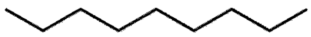 , 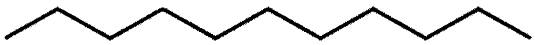 ,

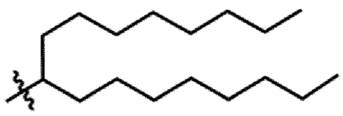 , 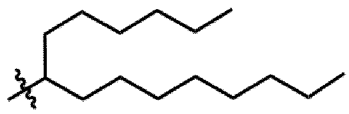 ,

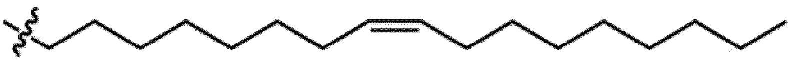 ,

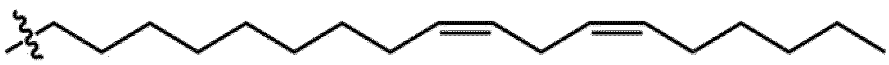 ,

and

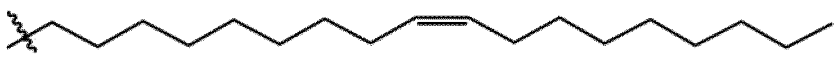 ;

$R^3$ is selected from the group consisting of fluoro, hydroxy, cyano, methoxy, acetoxy, methoxycarbonyl, butylaminocarbonyl and acetamido;
n is selected from the group consisting of 1, 2, and 3.

**21.** The compound according to claim 17 or a pharmaceutically acceptable salt or stereoisomer thereof, wherein said compound is selected from the group consisting of:

| No. | Structure |
|---|---|
| II-1 | 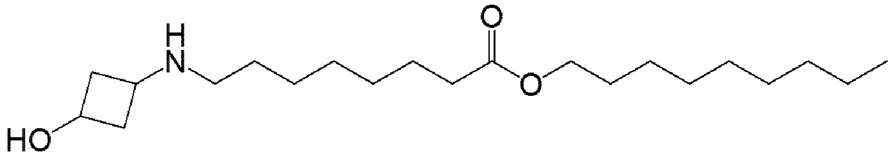 |
| II-2 | 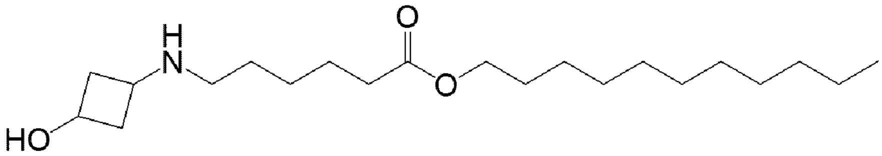 |
| II-4 | 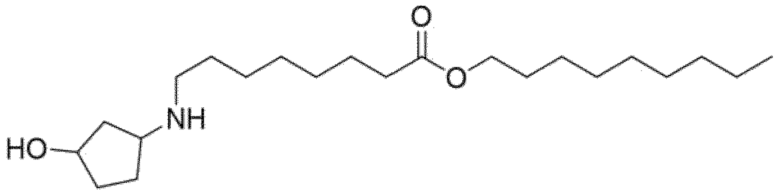 |
| II-5 | 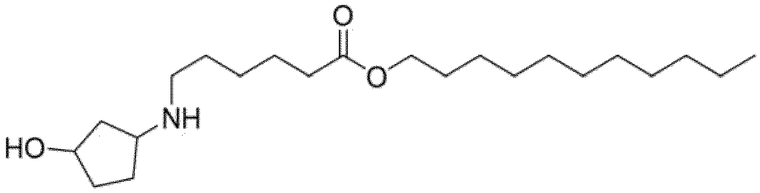 |
| II-6 | 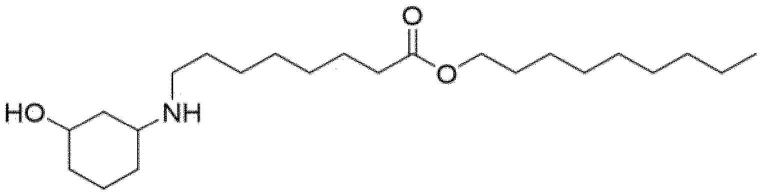 |
| II-7 | 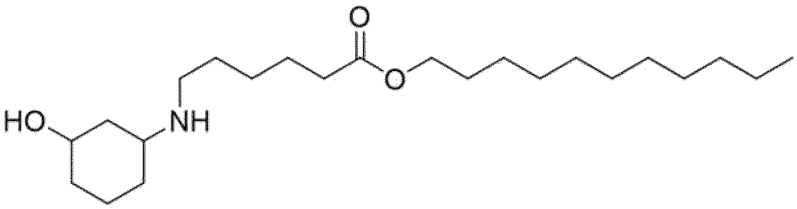 |
| II-8 | 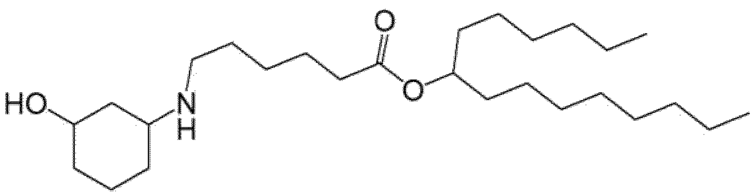 |
| II-9 | 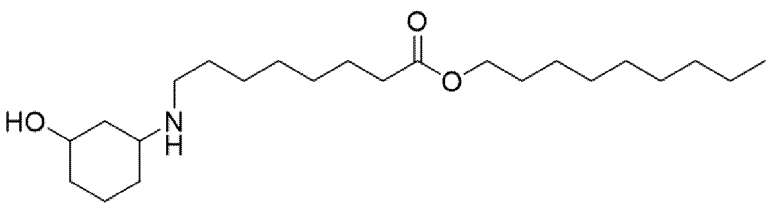 |

(continued)

| No. | Structure |
|---|---|
| II-10 | 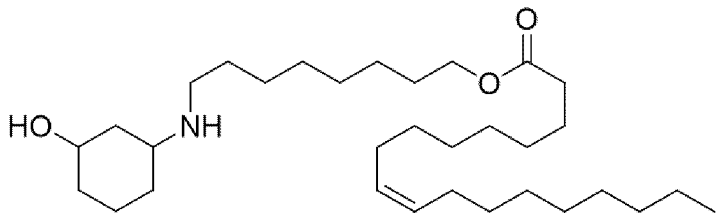 |
| II-11 | 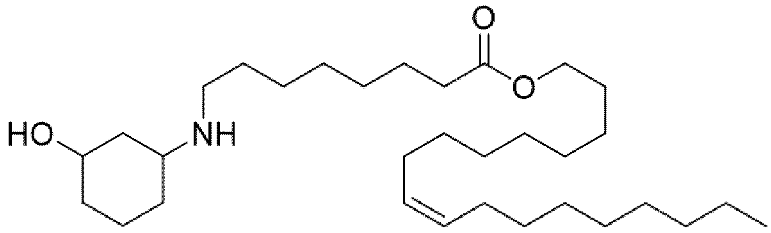 |
| II-12 | 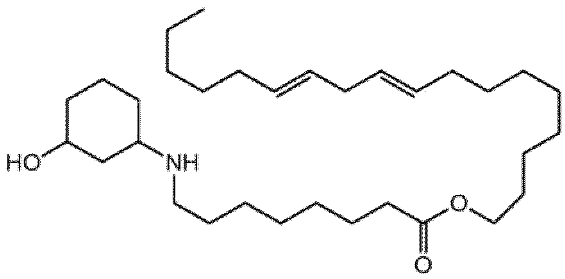 |
| II-14 | 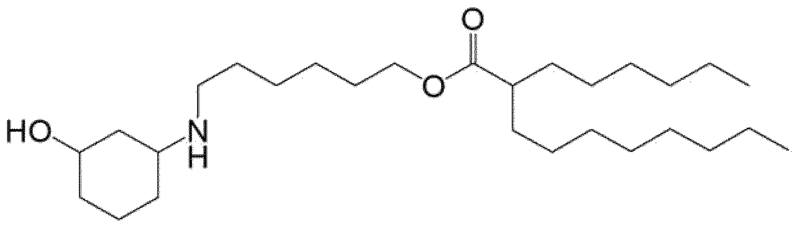 |
| II-15 | 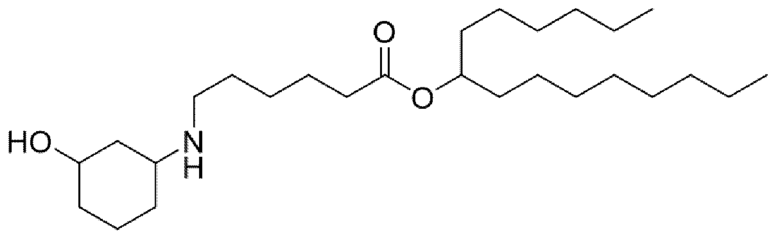 |
| II-16 | 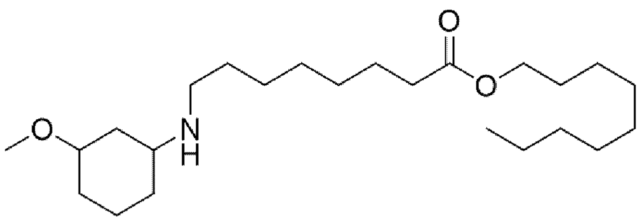 |

(continued)

| No. | Structure |
|---|---|
| II-17 | 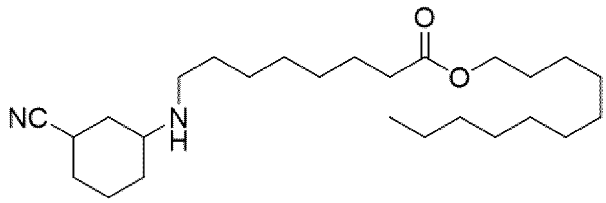 |
| II-18 | 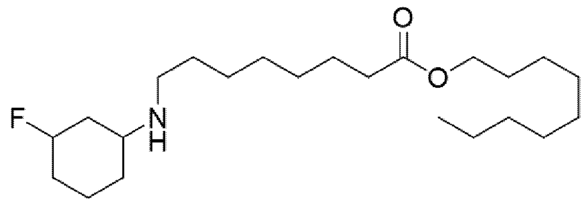 |
| II-19 | 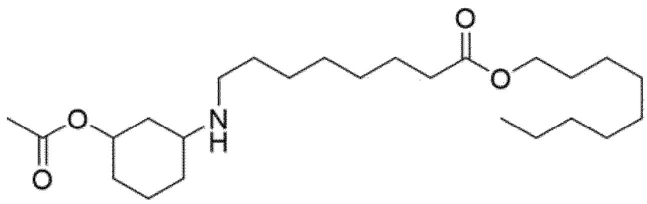 |
| II-20 | 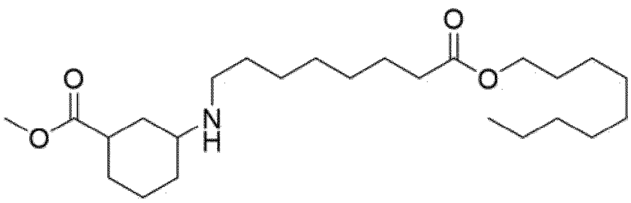 |
| II-21 | 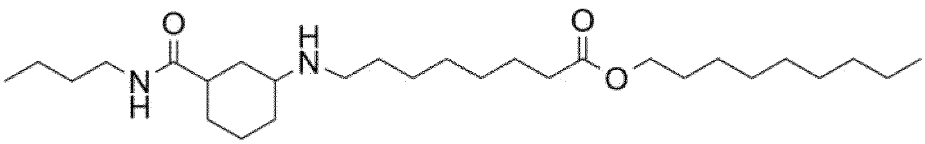 |
| II-22 | 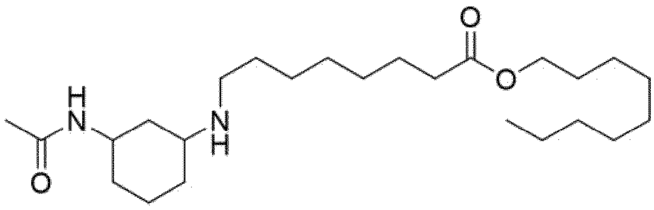 |

22. The compound according to claim 17 or a pharmaceutically acceptable salt thereof, wherein said compound is selected from the group consisting of:

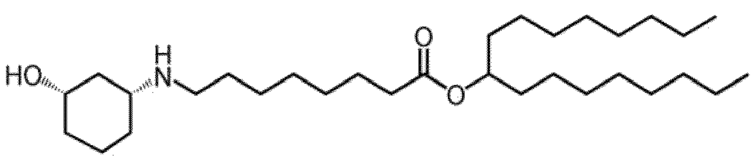

(6-VII-I)

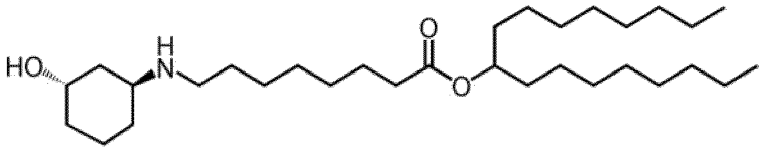

(6-VII-II)

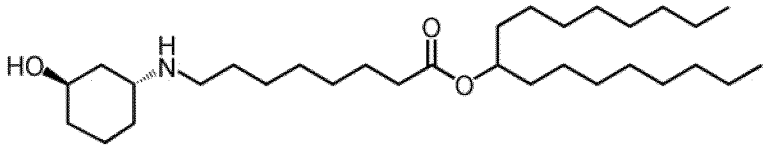

(6-VII-III)

and

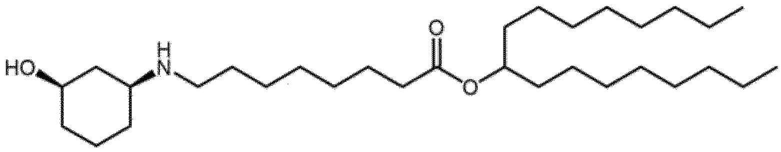

(6-VII-IV) .

**23.** Use of the compound according to any one of claims 17-22 in the preparation of the compound as defined in any one of claims 1-8 or a pharmaceutically acceptable salt or stereoisomer thereof.

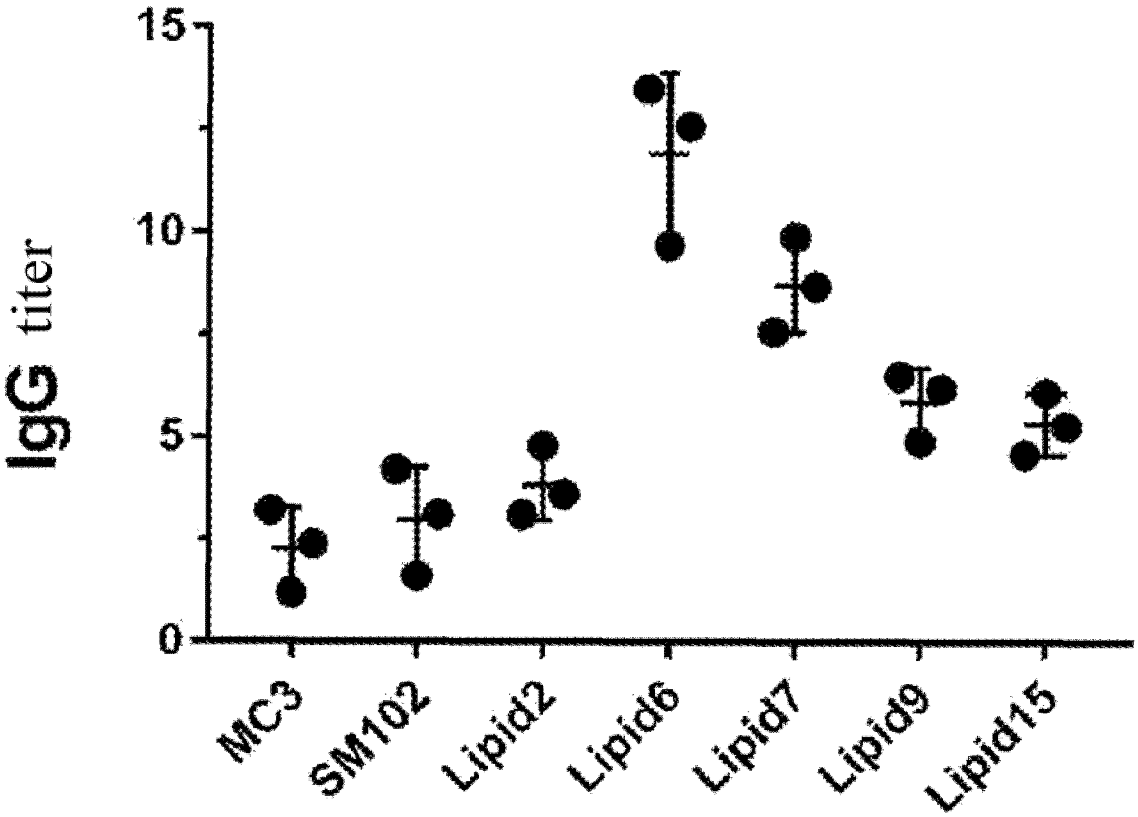
15
10
5
0
IgG titer
MC3
SM102
Lipid2
Lipid6
Lipid7
Lipid9
Lipid15

FIG.1

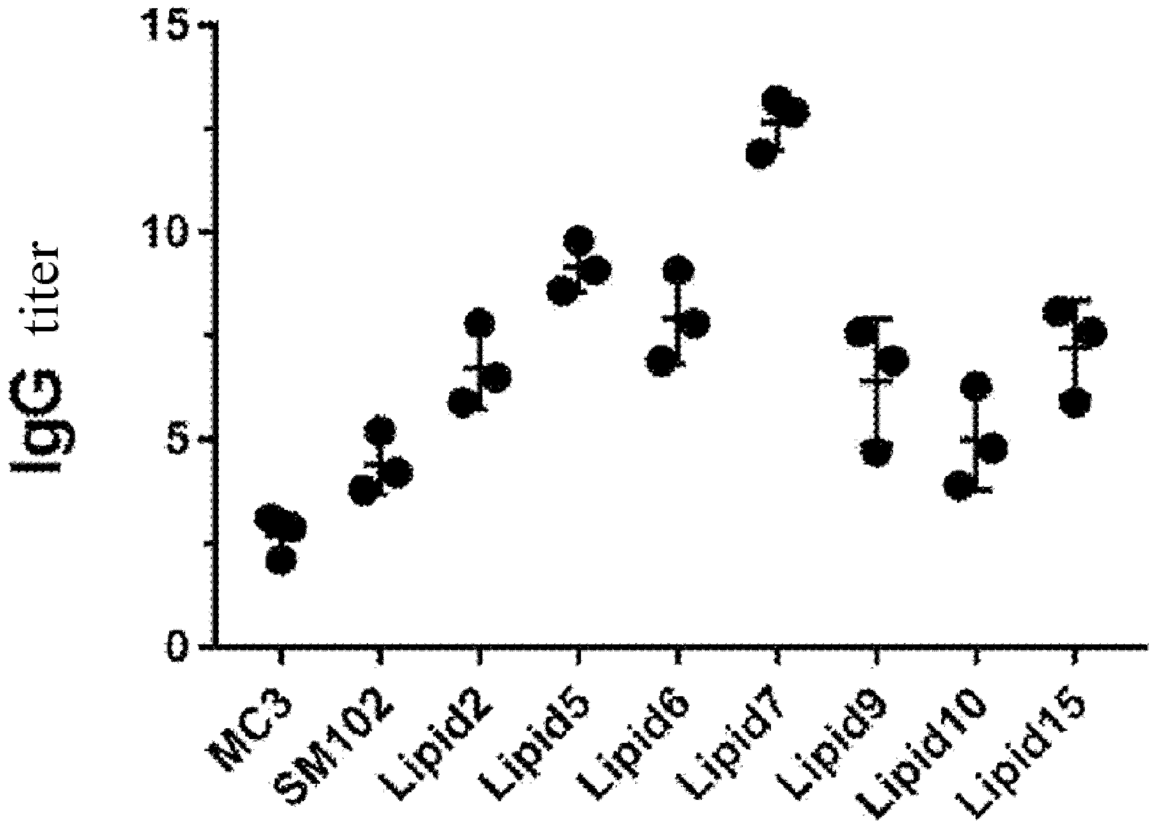


FIG.2

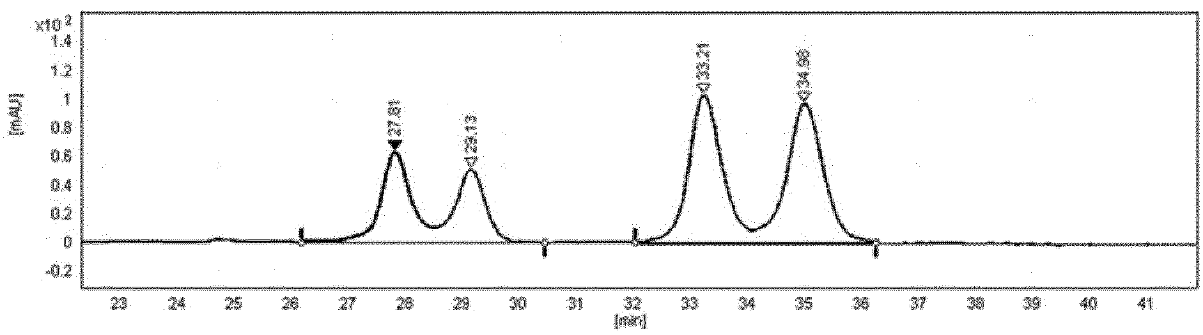


HPLC spectrum of a mixture of compounds (I-6-II), (I-6-III), (I-6-IV) and (I-6-V).

FIG. 3

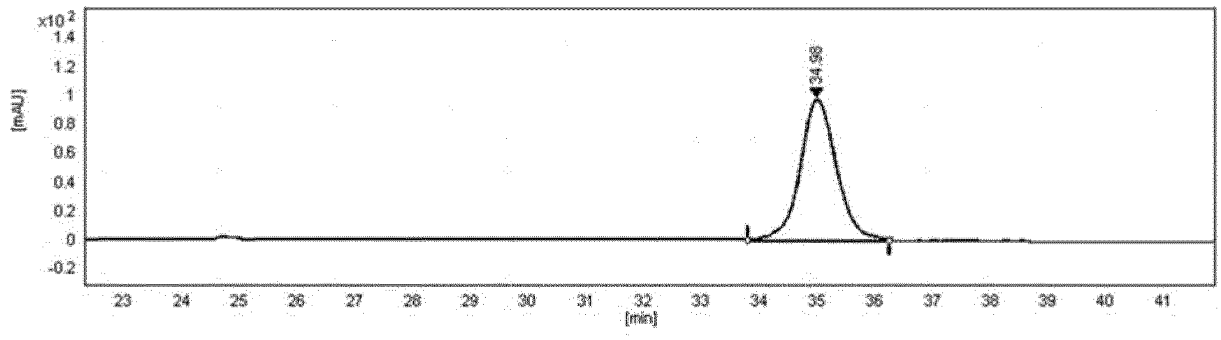


HPLC spectrum of compound (I-6-II).

FIG. 4

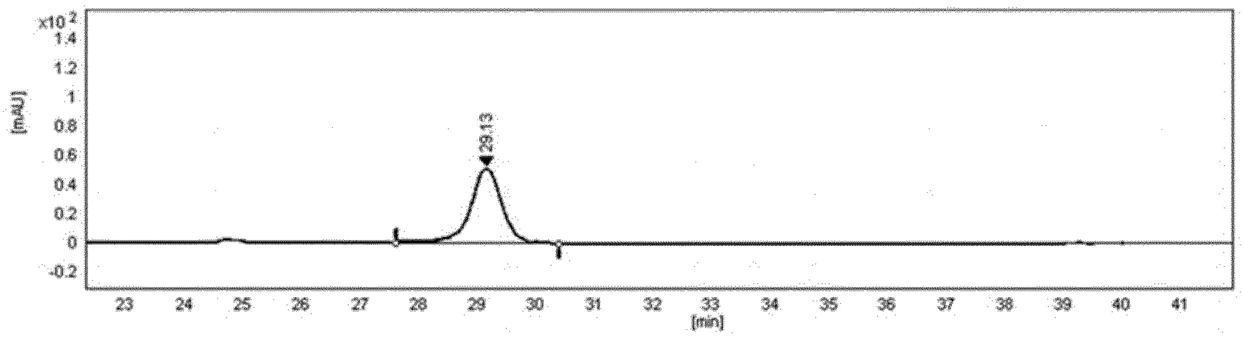


HPLC spectrum of compound (I-6-III).

FIG. 5

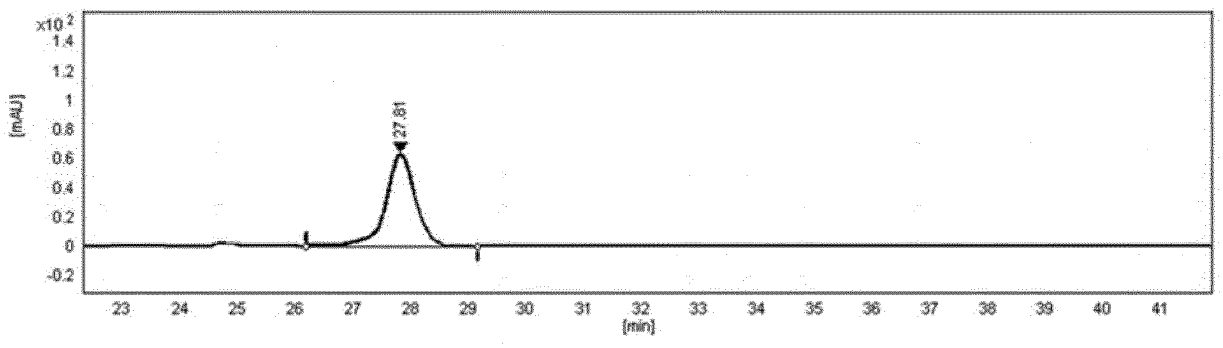


HPLC spectrum of compound (I-6-IV).

FIG. 6

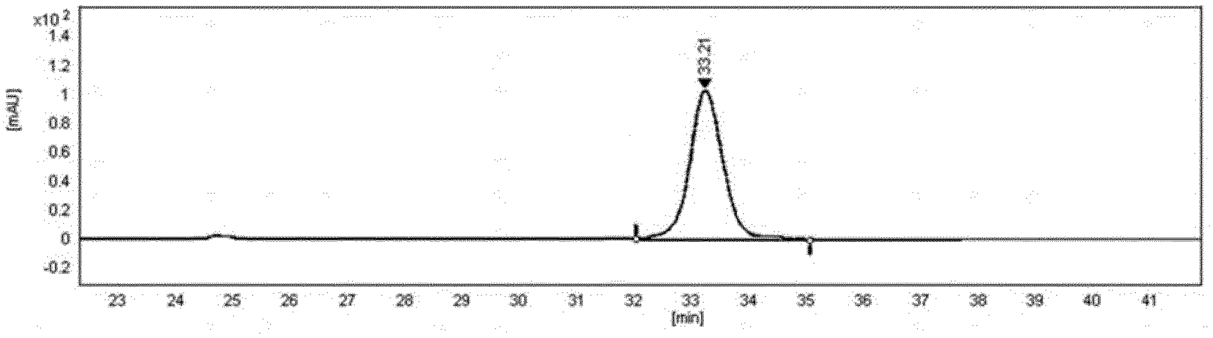


HPLC spectrum of the compound (I-6-V).

FIG. 7

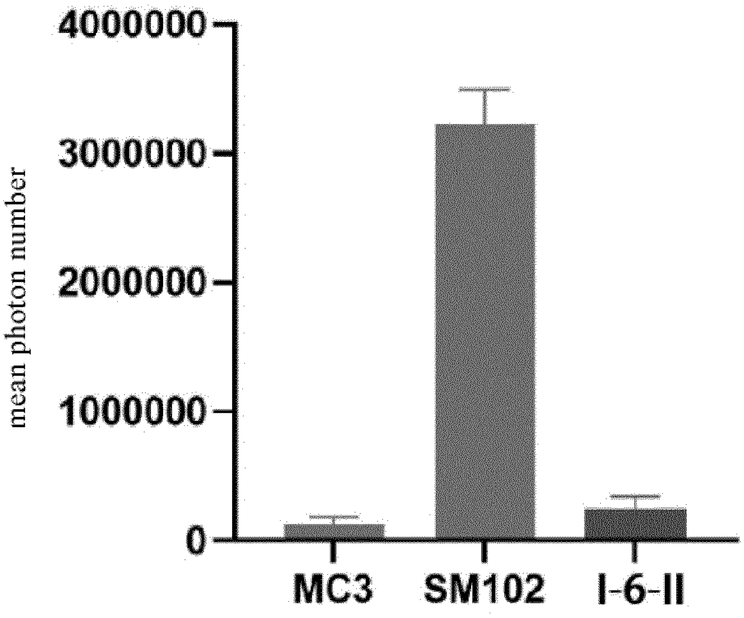


Fluorescence intensities in liver after intramuscular injection in the targeting assay.

FIG. 8

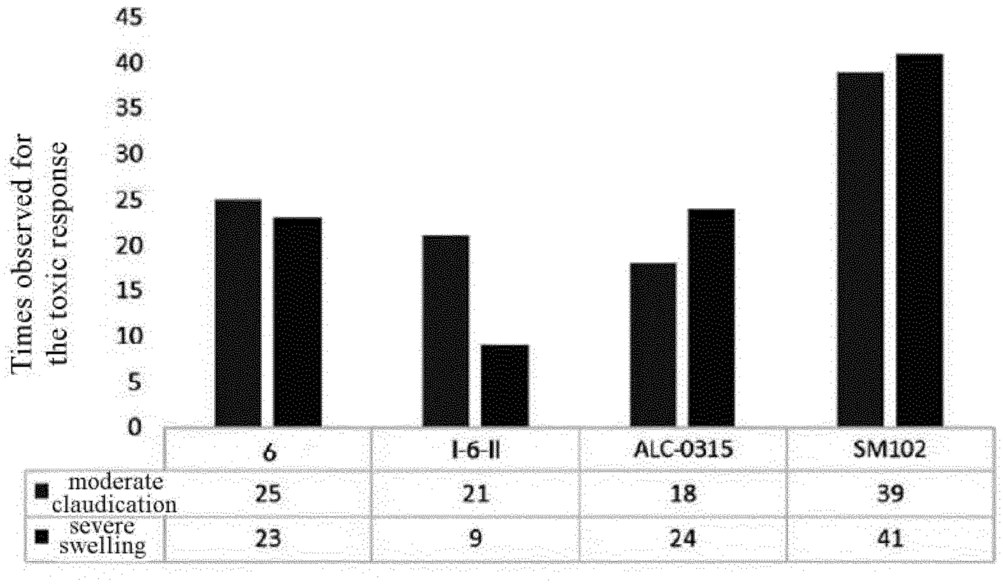


Statistics of severe swelling at the injection site and moderate claudication in the adverse reaction assay by intramuscular injection

FIG. 9

## INTERNATIONAL SEARCH REPORT

International application No.
**PCT/CN2023/107940**

**A. CLASSIFICATION OF SUBJECT MATTER**

C07C219/06(2006.01)i；A61K9/127(2006.01)i；A61K47/18(2017.01)i

According to International Patent Classification (IPC) or to both national classification and IPC

**B. FIELDS SEARCHED**

Minimum documentation searched (classification system followed by classification symbols)

C07C A61K

Documentation searched other than minimum documentation to the extent that such documents are included in the fields searched

Electronic data base consulted during the international search (name of data base and, where practicable, search terms used)

CNTXT, ENTXTC, ENTXT, DWPI, STN (REGISTRY, CAPLUS), CNKI: 高分子, 环丁基, 环己基, 环烷基, 环戊基, 聚, 脂质体, lipid, A61K9/127, C07C219/06, 根据权利要求1进行的结构式检索, structural formula search performed according to claim 1

**C. DOCUMENTS CONSIDERED TO BE RELEVANT**

| Category* | Citation of document, with indication, where appropriate, of the relevant passages | Relevant to claim No. |
|---|---|---|
| X | CN 114073677 A (SHENZHEN SHENXIN BIOTECHNOLOGY CO., LTD.) 22 February 2022 (2022-02-22) description, paragraphs 0011-0018, paragraph 0044, compounds 18-20, paragraphs 0050-0051, paragraphs 0080-0081, and paragraph 0204 | 1-23 |
| E | WO 2023138611 A1 (SUZHOU ABOGEN BIOSCIENCES CO., LTD.) 27 July 2023 (2023-07-27) description, table 1, compounds 39, 43, 54, and 57, and description, paragraph 0241 | 1-23 |
| PX | WO 2022152109 A2 (SUZHOU ABOGEN BIOSCIENCES CO., LTD.) 21 July 2022 (2022-07-21) description, page 55, compounds 67-68 | 1-23 |
| A | WO 2021030701 A1 (ACUITAS THERAPEUTICS INC.) 18 February 2021 (2021-02-18) description, page 148 | 1-23 |
| A | CN 114206827 A (SUZHOU ABOGEN BIOSCIENCES CO., LTD.) 18 March 2022 (2022-03-18) description, table 1, compounds 59-60, 79, and 97 | 1-23 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 November 2023** | **07 November 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/107940**

| Patent document cited in search report | Publication date (day/month/year) | Patent family member(s) | Publication date (day/month/year) |
|---|---|---|---|
| CN 114073677 A | 22 February 2022 | None | |
| WO 2023138611 A1 | 27 July 2023 | None | |
| WO 2022152109 A2 | 21 July 2022 | WO 2022152109 A3 | 25 August 2022 |
| | | TW 202229227 A | 01 August 2022 |
| WO 2021030701 A1 | 18 February 2021 | JP 2022544652 A | 20 October 2022 |
| | | CO 2022002685 A2 | 19 April 2022 |
| | | KR 20220053599 A | 29 April 2022 |
| | | IL 290477 A | 01 April 2022 |
| | | CL 2022000351 A1 | 03 February 2023 |
| | | CR 20220108 A | 27 May 2022 |
| | | US 2023097090 A1 | 30 March 2023 |
| | | BR 112022002708 A2 | 31 May 2022 |
| | | CA 3150458 A1 | 18 February 2021 |
| | | ECSP 22018209 A | 29 July 2022 |
| | | AU 2020328596 A1 | 31 March 2022 |
| | | EP 4013385 A1 | 22 June 2022 |
| | | JOP 20220037 A1 | 30 January 2023 |
| | | MX 2022001720 A | 11 March 2022 |
| | | PE 20220968 A1 | 10 June 2022 |
| | | DE 112020003843 T5 | 19 May 2022 |
| | | DOP 2022000038 A | 31 January 2023 |
| | | GB 2600859 A | 11 May 2022 |
| | | ES 2918001 A2 | 13 July 2022 |
| CN 114206827 A | 18 March 2022 | US 2022153686 A1 | 19 May 2022 |
| | | US 11472766 B2 | 18 October 2022 |
| | | ZA 202210782 B | 26 April 2023 |
| | | IL 297084 A | 01 December 2022 |
| | | JP 2023529522 A | 11 July 2023 |
| | | BR 112022020203 A2 | 22 November 2022 |
| | | EP 4077272 A1 | 26 October 2022 |
| | | CA 3178455 A1 | 14 October 2021 |
| | | AU 2021254312 A1 | 27 October 2022 |
| | | TW 202204309 A | 01 February 2022 |
| | | US 2023286902 A1 | 14 September 2023 |
| | | KR 20220166802 A | 19 December 2022 |
| | | WO 2021204175 A1 | 14 October 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 110520409 A


### Non-patent literature cited in the description


- **XIAOPING ZHANG et al.** Pharmacokinetics of Patisiran, the First Approved RNA Interference Therapy in Patients With Hereditary Transthyretin-Mediated Amyloidosis. *The Journal of Clinical Pharmacology*, 2020, vol. 60 (5), 573-585
- **HOANG THI** ; **THAI THANH et al.** The Importance of Poly(ethylene glycol) Alternatives for Overcoming PEG Immunogenicity in Drug Delivery and Bioconjugation. *Polymers*, vol. 12 (2), 298
- **BERGE et al.** Pharmaceutically acceptable salts. *J. Pharm. Sci.*, 1977, vol. 66, 1-19
- General testing methods 0621. *Pharmacopoeia of the People's Republic of China 2020*, vol. IV